# EUROPEAN PATENT SPECIFICATION

(11) **EP 1 212 438 B1**
(45) Date of publication and mention of the grant of the patent: **17.05.2006**
(21) Application number: 00954241.6
(22) Date of filing: 25.08.2000
(51) Int. Cl.: C12N 15/82, A01H 5/10, A01H 5/00, C12N 15/29

(54) **FLAX SEED SPECIFIC PROMOTERS**
SAMENSPEZIFISCHER PROMOTER AUS FLACHS (LINUM USITATISSIMUM)
PROMOTEURS SPECIFIQUES DES GRAINES DE LIN

(30) Priority: 27.08.1999 US 151044 P; 27.10.1999 US 161722 P; 30.05.2000 CA 2310304
(43) Date of publication of application: 12.06.2002
(73) Proprietor: SemBioSys Genetics Inc., Calgary, Alberta T2N 1N4 (CA); COMMONWEALTH SCIENTIFIC AND INDUSTRIAL RESEARCH ORGANISATION, Canberra, ACT 2601 (AU)
(72) Inventor: CHAUDHARY, Sarita, Belle River, Ontario N0R1A0 (CA); VAN ROOIJEN, Gijs, Calgary, Alberta T2L 1T1 (CA); MOLONEY, Maurice, M., Calgary, Alberta T3A 5N5 (CA); SINGH, Surinder, Downer, ACT 2601 (AU)
(74) Representative: Williams, Aylsa
(86) International application number: PCT/CA2000/000988
(87) International publication number: WO 2001/016340

(56) References cited:
- WO-A-98/18948
- SAMMOUR REDA HELMY: "Proteins of linseed (Linum usitatissimum L.), extraction and characterization by electrophoresis." BOTANICAL BULLETIN OF ACADEMIA SINICA (TAIPEI), vol. 40, no. 2, April 1999 (1999-04), pages 121-126, XP000945031 ISSN: 0006-8063
- BORGMEYER J R ET AL: "ISOLATION AND CHARACTERIZATION OF A 25 KDA ANTIFUNGAL PROTEIN FROM FLAX SEEDS" BIOCHEMICAL AND BIOPHYSICAL RESEARCH COMMUNICATIONS, vol. 187, no. 1, 1992, pages 480-487, XP000941778 ISSN: 0006-291X

## Description

### FIELD OF THE INVENTION

The present invention relates to plant genetic engineering methods useful for the alteration of the constituents of plant seeds. More specifically, the invention relates to promoters that have been obtained from flax and are capable of directing expression of non-native genes in flax seeds as well as the seeds of other plants.

### BACKGROUND OF THE INVENTION

Flax or linseed (Linum usitatissimum) is a commercially important oilseed crop. Flax oil and meal are valuable raw materials derived from flax seed. A further economically significant raw material, flax fiber, is obtainable from the stem of the plant. The flax oil fraction is used for non-edible purposes, for example in the manufacture of varnish and paint, and has more recently become suited for use in the manufacture of a range of edible products, such as margarines and salad oils and dressings, by virtue of newly bred so called Linola cultivars (Green (1986) Can. J. Plant Sci, 66: 499-503). Flax meal is used primarily as a constituent of ruminant feeds while flax fibers are used in the manufacture of linen fabrics. Given its economic importance as a source for raw materials, it is desirable to further improve and diversify the available flax cultivar portfolio both with respect to agronomic performance, for example seed yield, resistance to pathogens and low climatic temperatures, and with respect to yield and quality of the raw materials to suit downstream applications. Although it is possible to obtain improved flax cultivars through conventional plant breeding, as evidenced by the development of the I,inola cultivars, developing an elite agronomic plant line requires large investments in plant breeding due to the long timelines involved. Plant genetic engineering technology allows the isolation of genes directly from unrelated species and the transfer of these genes into elite agronomic backgrounds, thereby significantly reducing the time required to develop new cultivars. In addition plant genetic engineering permits the manufacture of products not naturally obtainable from flax, for example therapeutic agents.

In order to develop novel flax cultivars through plant genetic engineering, control over the expression of the introduced foreign or non-native gene is of critical importance. The desired expression characteristics for the non-native gene, such as the level of expression of the non-native gene, the particular plant tissue or organ in which the non-native gene is expressed, and the particular time in the growth cycle of the plant at which the non-native gene is expressed, will vary depending on the application for which the plant line is developed. For example, the modification of the seed oil composition may require low levels of seed-specific expression of an enzyme involved in fatty acid metabolism at an early stage in seed development (see for example US Patent 5,420,034). On the other hand expression of a pharmaceutical protein could preferably require high levels of leaf-specific expression upon harvesting of the plant leaves (see for example, US Patent 5,929,304).

In order to manipulate the expression characteristics of non-native genes numerous factors can be influenced. One factor is the choice of the transcriptional promoter used. A wide range of plant compatible promoters is currently available and some of the better documented promoters include constitutive promoters such as the 35-S CaMV promoter (Rothstein et al. (1987), Gene 53: 153-161) and the ubiquitin promoter (US Patent 5,614,399), tissue specific promoters such as seed-specific promoters, for example the phaseolin promoter (Sengupta-Gopalan et al., (1985), PNAS USA 82: 3320-3324) and inducible promoters, such as those inducible by heat (Czaxnencka et al., (1989), Mol. Cell. Biol. 9 (8): 3457-3464), UV light, elicitors and wounding (Lois et al., (1989) EMBO J. 8 (6): 1641-1648), or chemicals such as endogenous hormones (Skriver et al. (1991), Proc. Natl. Acad. Sci. USA 88(16): 7266-7270). Other factors that can be manipulated in order to control the expression characteristics of non-native gene in plants include transcriptional modification factors such as introns, polyadenylation sites and transcription termination sites, The expression characteristics of the non-native gene can further be manipulated by factors that affect translation, such as ribosomal binding sites and the codon bias that is exhibited by the host. Furthermore, the non-native gene itself may affect the viability of the transgenic plant, thus limiting particularly the levels of expression that can be attained. In some cases it may be possible to overcome this problem, by expressing the protein in a tissue specific manner, e.g. in the leaves or seed, or by restricting the accumulation of the protein in different subcellular compartments such as for example the cytoplasm, the endoplasmic reticulum or vacuoles, typically by the presence or the absence of specific targeting sequences capable of directing the protein to these compartments. Another factor that will affect the expression characteristics is the location in which the construct inserts itself into the host chromosome. This effect could provide an explanation as to why different plants, transformed with the same recombinant construct, can have fluctuating levels of recombinant protein expression.

To the best of the inventors' knowledge, expression of non-native genes in flax seeds is only documented in PCT Patent Application WO 98/18948. This application discloses two stearoyl-acyl carrier protein desaturase (SAD) genes derived from flax. The associated SAD promoter sequences are useful for the modification of flax and other plants for the expression of endogenous or foreign genes. However the methods taught by WO 98/18948 are limited by the fact that the SAD promoters are not seed-specific in flax and confer expression to leaves, stems, flowers and seeds. Expression of non-native genes thus may result in undesirable side effects in non-seed tissues. In addition the use of the SAD promoters allows limited control over expression level and timing of expression.

There is a need in the art to further improve methods for the expression of non-native genes in flax seeds and other plant seeds.

### SUMMARY OF THE INVENTION

The present invention relates to improved methods for the seed-specific expression of non-native genes in plants. In particular, the invention relates to improved methods for the seed-specific expression of non-native genes in flax.

Accordingly, in one aspect, the invention provides a method for the expression of a nucleic acid sequence of interest in flax seeds comprising:
(a) preparing a chimeric nucleic acid construct comprising in the 5' to 3' direction of transcription as operably linked components
   (1) a seed-specific promoter obtained from flax; and
   (2) the nucleic acid sequence of interest wherein said nucleic acid of interest is non-native to said flax seed-specific promoter,
(b) introducing said chimeric nucleic acid construct into a flax plant cell; and
(c) growing said flax plant cell into a mature flax plant capable of setting seed, wherein said nucleic acid sequence of interest is expressed in the seed under the control of said flax seed-specific promoter, wherein seed-specific means that a gene expressed under the control of the promoter is predominantly expressed in plant seeds with less than 5% of the overall expression level in other plant tissues and wherein said flax seed-specific promoter is an oleosin promoter, a 2S storage protein promoter or a legumin-like seed storage protein promoter.

In a preferred embodiment of the invention, at least one expression characteristic, e.g. timing of expression in the plant's life cycle, conferred by the promoter to the non-native nucleic acid sequence is similar to that expression characteristic when conferred to a native nucleic acid sequence.

In a further aspect, the present invention provides transgenic flax seeds prepared according to a method comprising:
(a) preparing a chimeric nucleic acid construct comprising in the 5' to 3' direction of transcription as operably linked components:
   (1) a seed-specific promoter obtained from flax; and
   (2) a nucleic acid sequence of interest wherein said nucleic acid of interest is non-native to said seed-specific promoter;
(b) introducing said chimeric nucleic acid construct into a flax plant cell; and
(c) growing said flax plant cell into a mature flax plant capable of setting seed, wherein said nucleic acid sequence of interest is expressed in the seed under the control of said seed-specific promoter, wherein seed-specific means that a gene expressed under the control of the promoter is predominantly expressed in plant seeds with less than 5% of the overall expression level in other plant tissues and wherein the flax seed-specific promoter is an oleosin promoter, a 2S storage protein promoter or a legumin-like seed storage protein promoter.

In a further aspect the present invention provides flax plants capable of setting seed prepared by a method comprising:
(a) preparing a chimeric nucleic acid construct comprising in the 5' to 3' direction of transcription as operably linked components:
   (1) a seed-specific promoter obtained from flax; and
   (2) a nucleic acid sequence of interest wherein said nucleic acid of interest is non-native to said seed-specific promoter;
(b) introducing said chimeric nucleic acid construct into a flax plant cell; and
(c) growing said flax plant cell into a mature flax plant capable of setting seed, wherein said nucleic acid sequence of interest is expressed in the seed under the control of said seed-specific promoter, wherein seed-specific means that a gene expressed under the control of the promoter is predominantly expressed in plant seeds with less than 5% of the overall expression level in other plant tissues and wherein the flax seed-specific promoter is an oleosin promoter, a 2S storage protein promoter or a legumin-like seed storage protein promoter.

Novel flax seed specific promoters useful for the expression of non-native genes in flax seeds and the seeds of other plant species, useful for example for modification of the protein or oil composition of the seed are taught herein.

In a preferred embodiment, the seed specific promoter comprises:
(a) a nucleic acid sequence comprising nucleotides 1 to 2023 as shown in Figure 1 (SEQ.ID.NO.:1) wherein T can also be U, nucleotides 21 to 1852 as shown in Figure 2 (SEQ.ID.NO.:4) wherein T can also be U, nucleotides 1 to 400 as shown in Figure 3 (SEQ.ID.NO.:6) wherein T can also be U or nucleotides 1 to 2034 as shown in Figure 4 (SEQ.ID.NO.:8) wherein T can also be U;
(b) a nucleic acid sequence that is complimentary to a nucleic acid sequence of (a);
(c) a nucleic acid sequence that has at least 65% identity to a nucleic acid sequence of (a) or (b);
(d) a nucleic acid sequence that is an analog of a nucleic acid sequence of (a), (b) or (c); or
(e) a nucleic acid sequence that hybridizes to a nucleic acid sequence of (a), (b), (c) or (d) under stringent hybridization conditions.

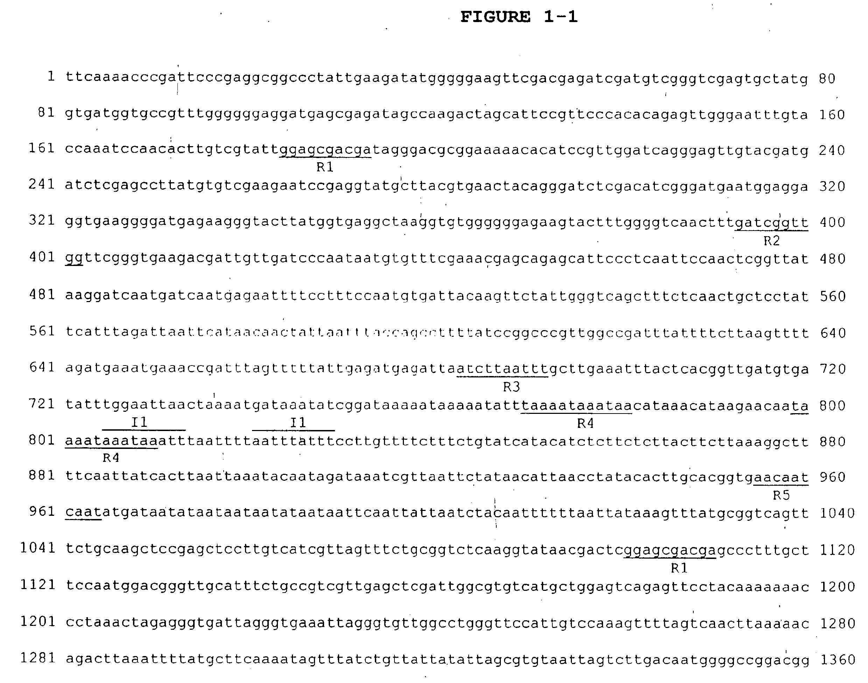

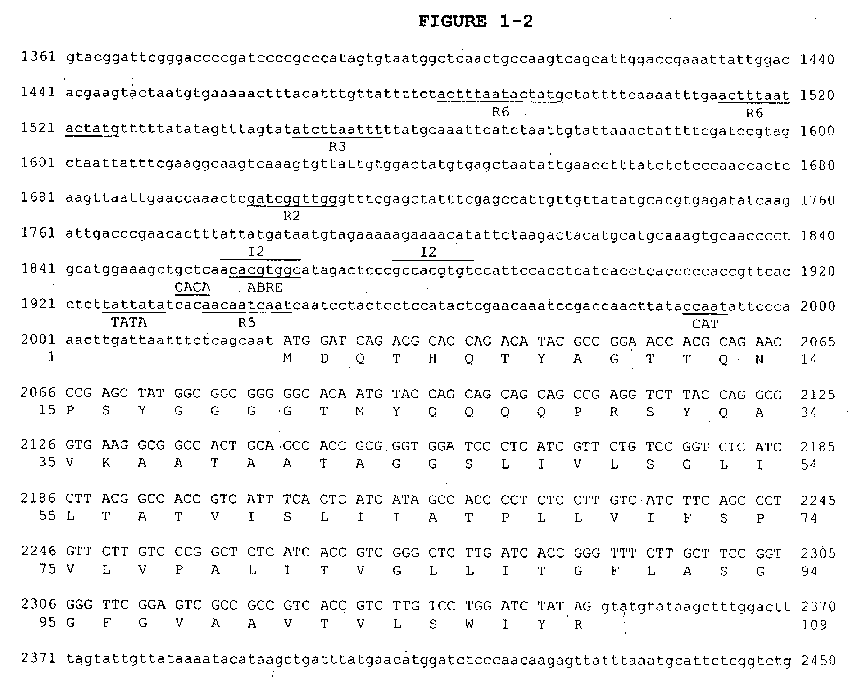

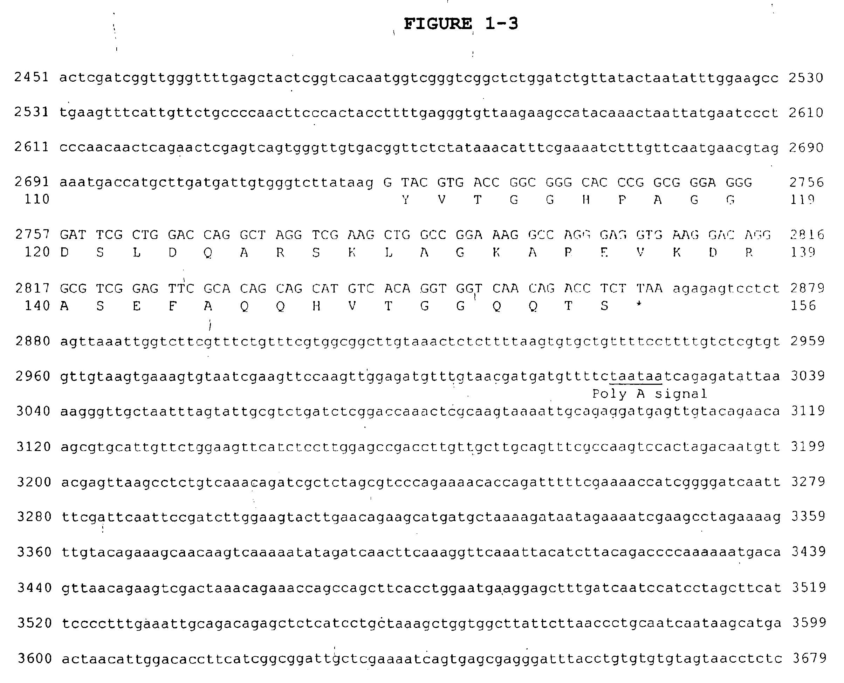

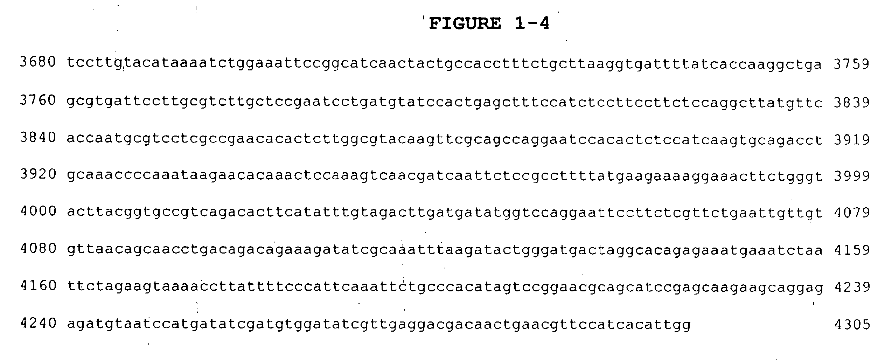

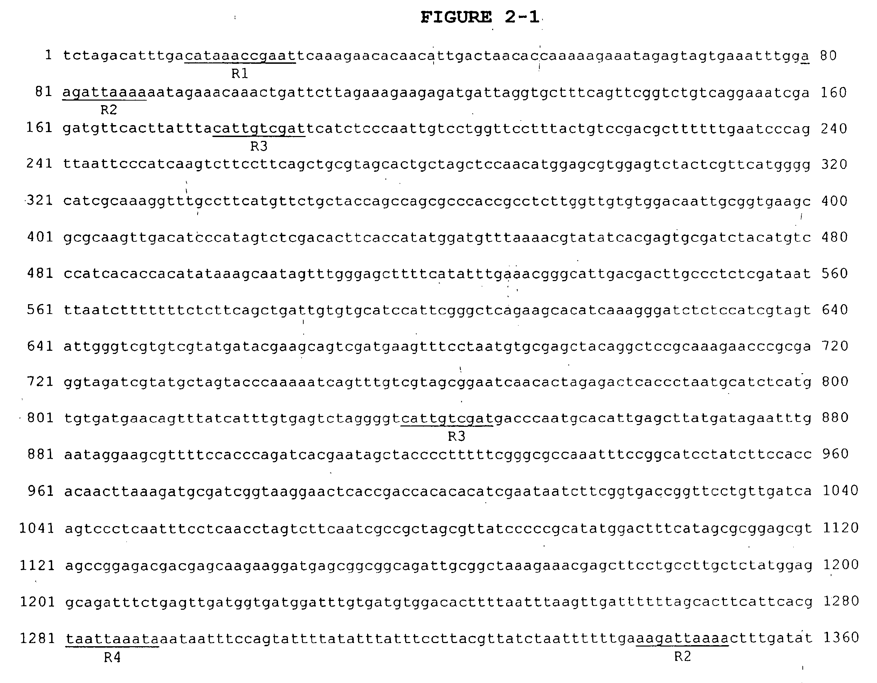

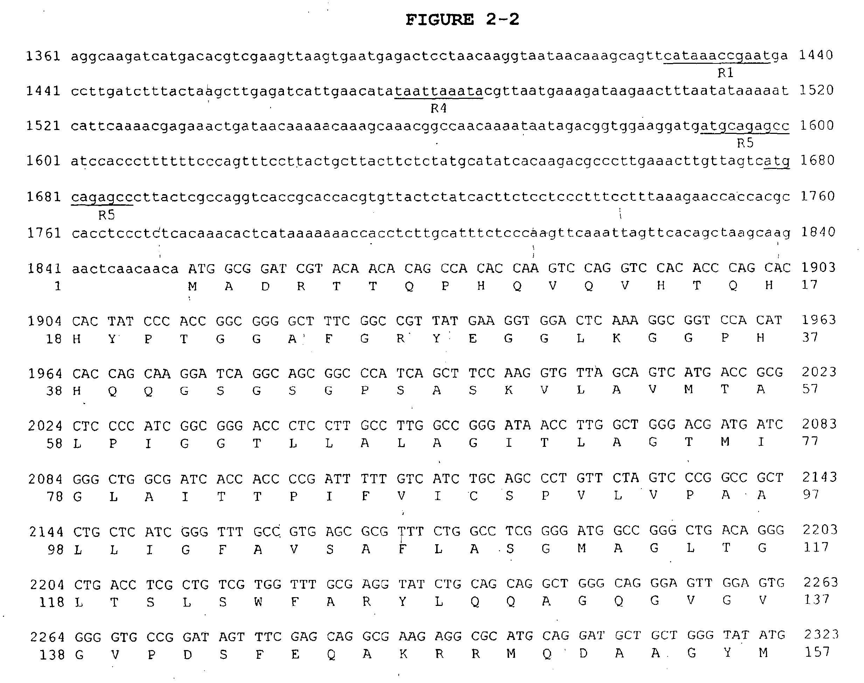

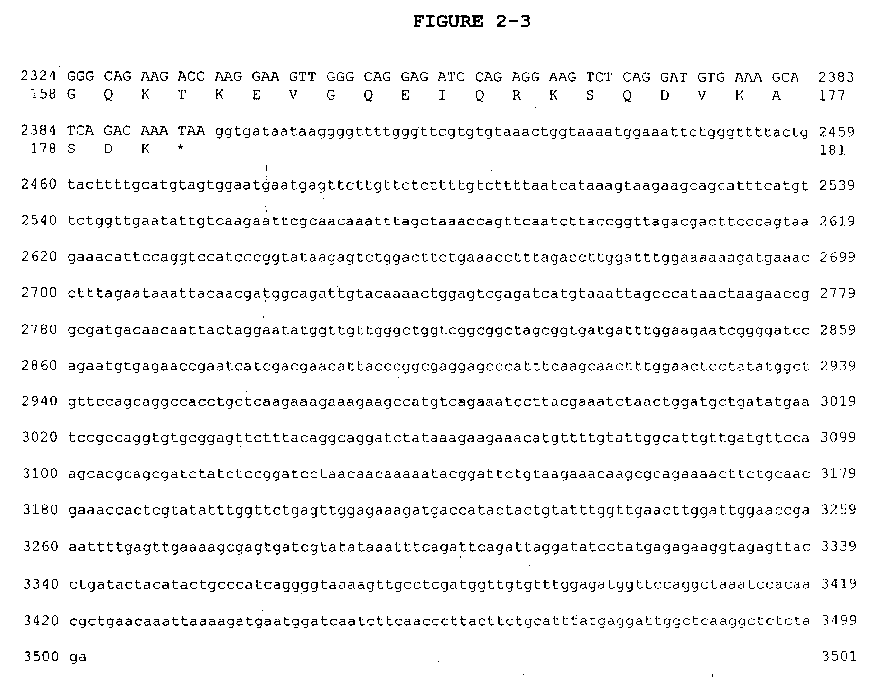

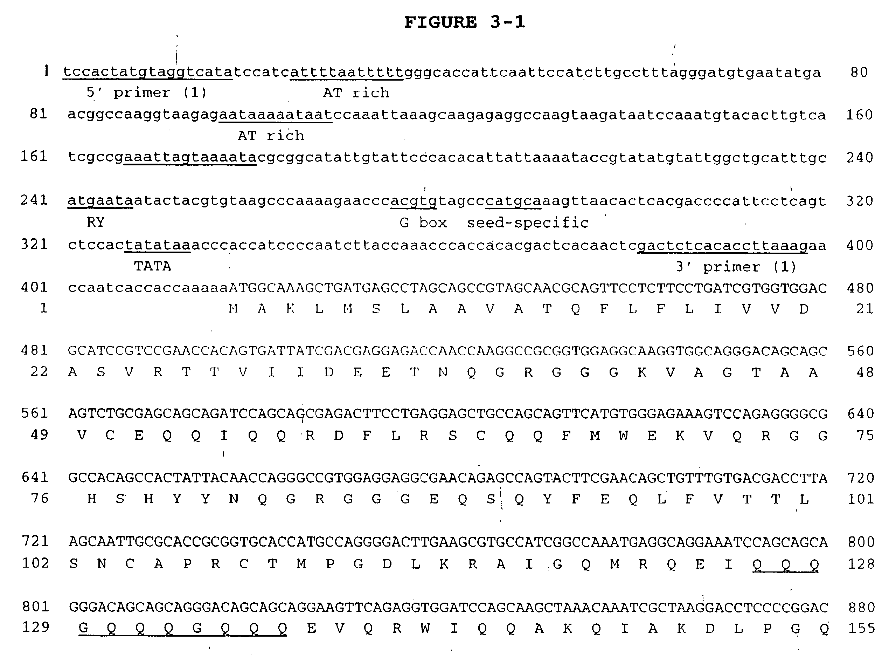

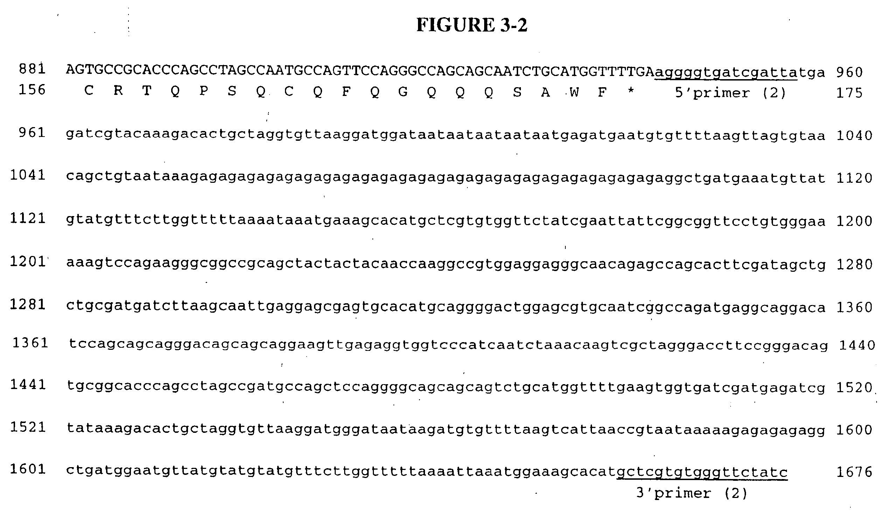

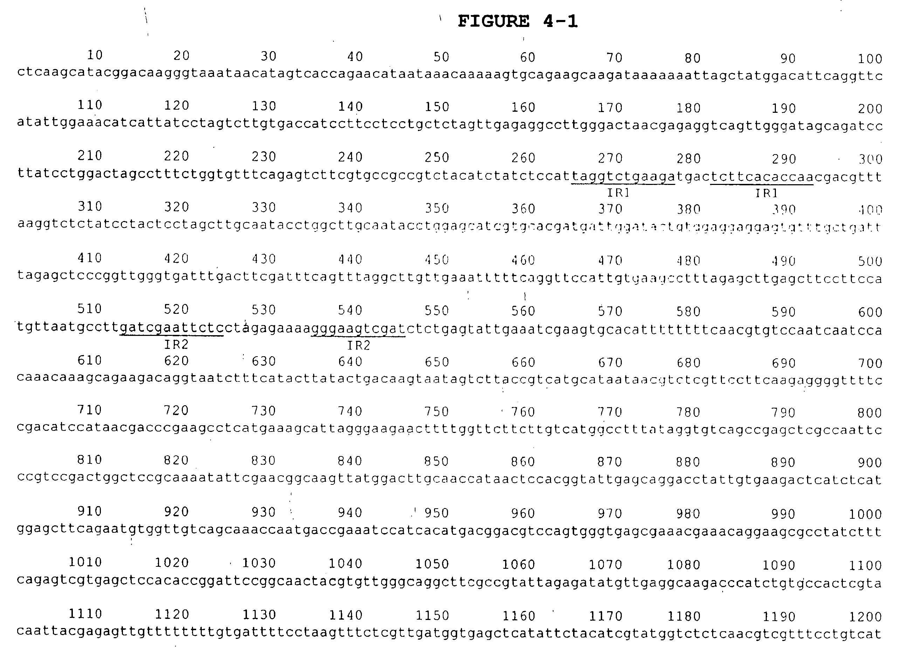

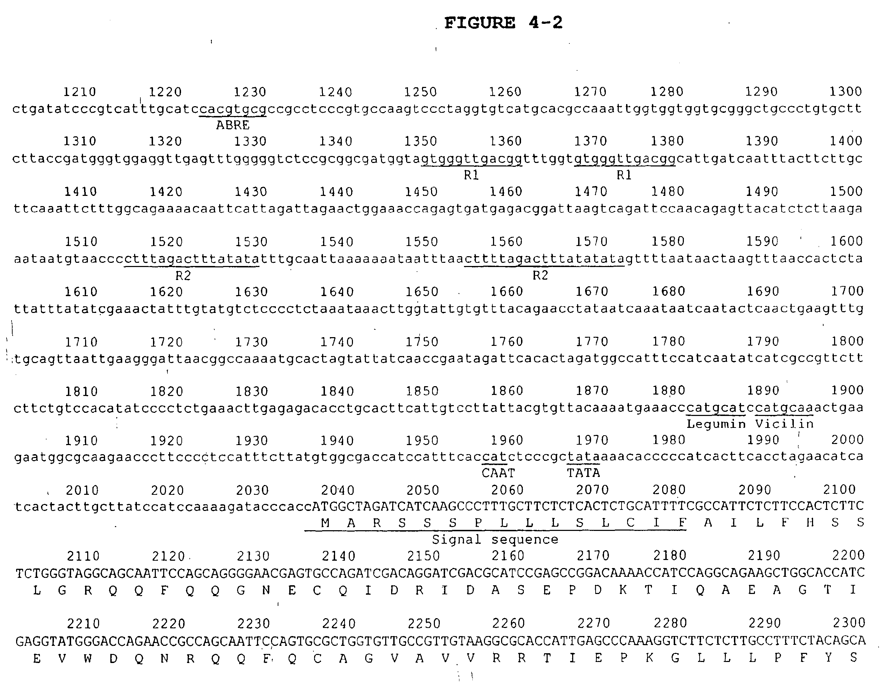

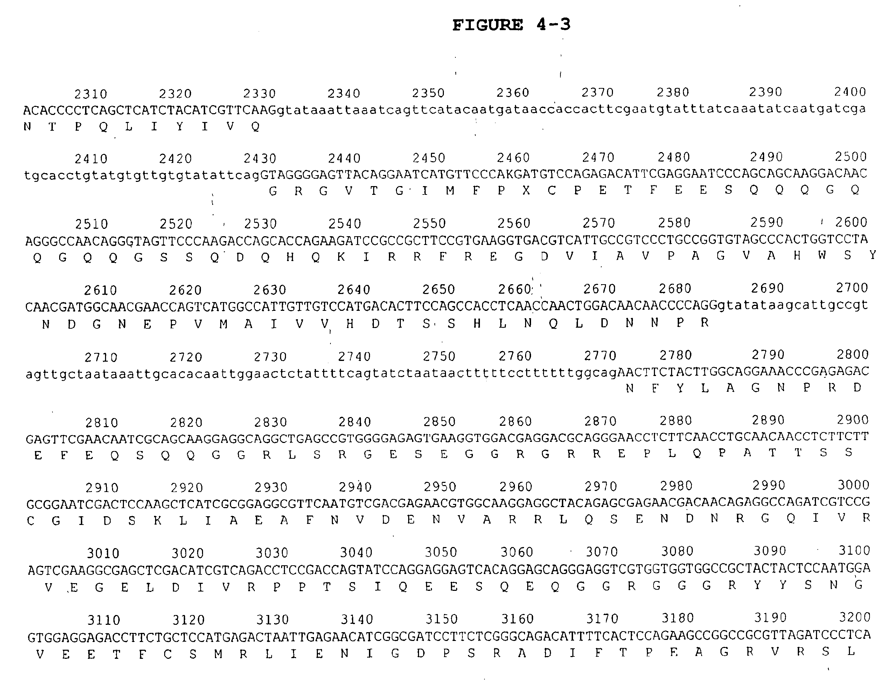

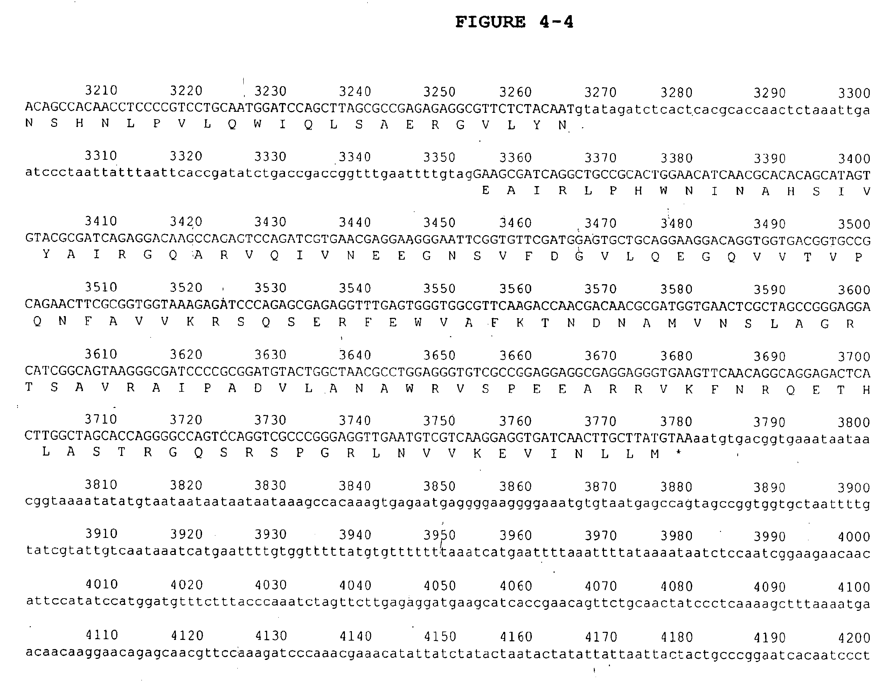

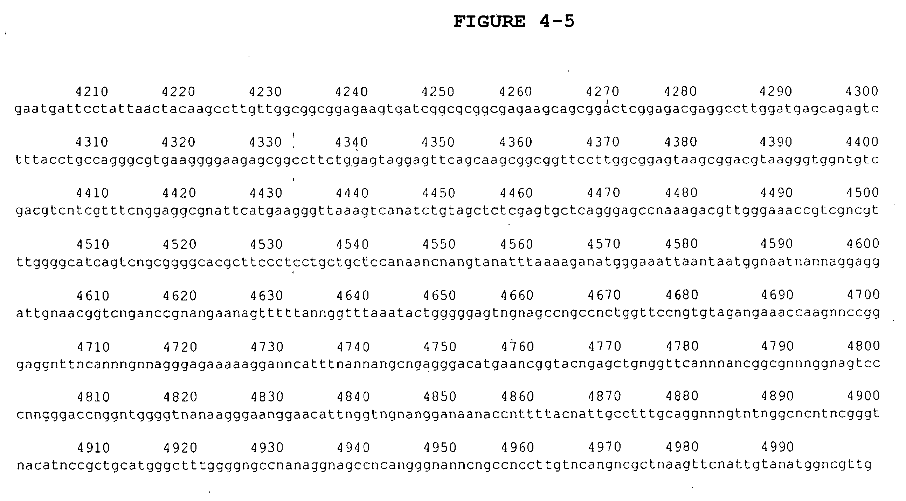

In another aspect, the invention provides an isolated chimeric nucleic acid sequence comprising:
(a) a first nucleic acid sequence comprising a seed-specific promoter obtained from flax which comprises:
   (1) a nucleic acid sequence comprising nucleotides 1-2023 as shown in Figure 1 (SEQ ID NO. 1), wherein T can also be U; a nucleic acid sequence comprising nucleotides 21-1852 as shown in Figure 2 (SEQ ID NO. 4), wherein T can also be U; a nucleic acid sequence comprising nucleotides 1-400 as shown in Figure 3 (SEQ ID NO. 6), wherein T can also be U; or a nucleic acid sequence comprising nucleotides 1-2034 as shown in Figure 4 (SEQ ID NO. 8), wherein T can also be U;
   (2) a nucleic acid sequence that hybridizes to a nucleic acid sequence of (a) under stringent hybridization conditions;
   (3) a nucleic acid sequence that is complimentary to a nucleic acid sequence of (a); or
   (4) a nucleic acid sequence that has at least 65% identity to a nucleic acid sequence of (a); and
(b) a second nucleic acid sequence non-native to said flax seed-specific promoter; wherein seed-specific means that a gene expressed under the control of the promoter is predominantly expressed in plant seeds with less than 5% of the overall expression level in other plant tissues.

Other features and advantages of the present invention will become readily apparent from the following detailed description. It should be understood, however, that the detailed description and the specific examples while indicating preferred embodiments of the invention are given by way of illustration only, since various changes and modifications within the scope of the invention will become apparent to those skilled in the art of this detailed description.

### BRIEF DESCRIPTION OF THE DRAWINGS

The invention will now be described in relation to the drawings in which:
Figure 1 shows the DNA sequence (SEQ.ID.NO.:1) of a flax genomic clone encoding a 16.0 kDa oleosin protein (SEQ,ID.NOS.:2 and 3).
Figure 2 shows the DNA sequence (SEQ.ID.NO.:4) of a flax genomic clone encoding a 18.6 kDa oleosin protein (SEQ.ID.NO.:5).
Figure 3 shows the DNA sequence (SEQ.ID.NO.:6) of a flax genomic clone encoding a 2S storage protein (SEQ.ID.NO.:7).
Figure 4 shows the DNA sequence (SEQ.ID.NO.:8) of a flax genomic clone encoding a 54.5 kDa legumin-like storage protein (SEQ.ID.NOS.:9-12).

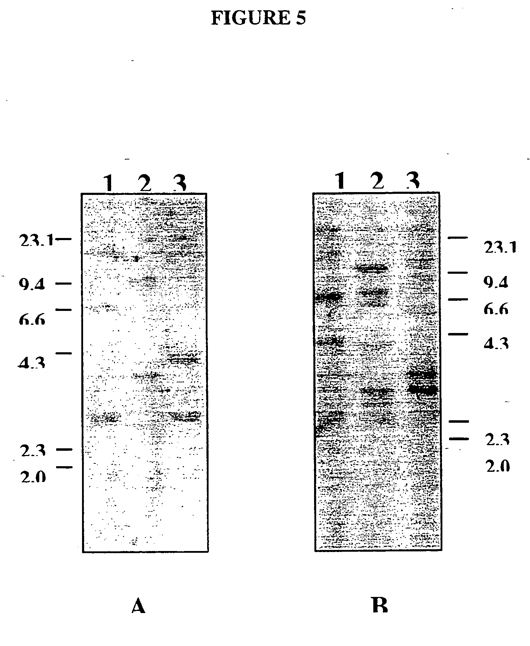

Figure 5 shows Southern blot analysis of flax genomic DNA probed with flax oleosin DNA sequences.

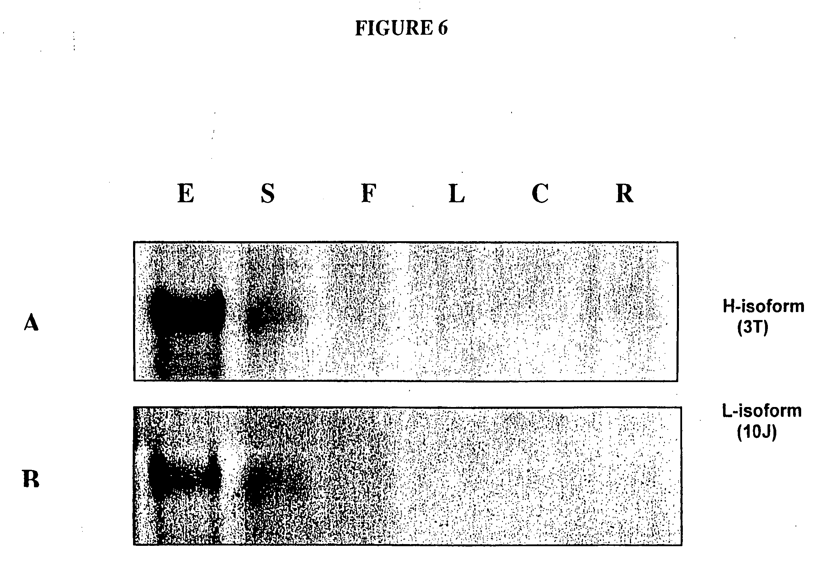

Figure 6 shows a Northern blot analysis of the seed specific expression of flax oleosins.

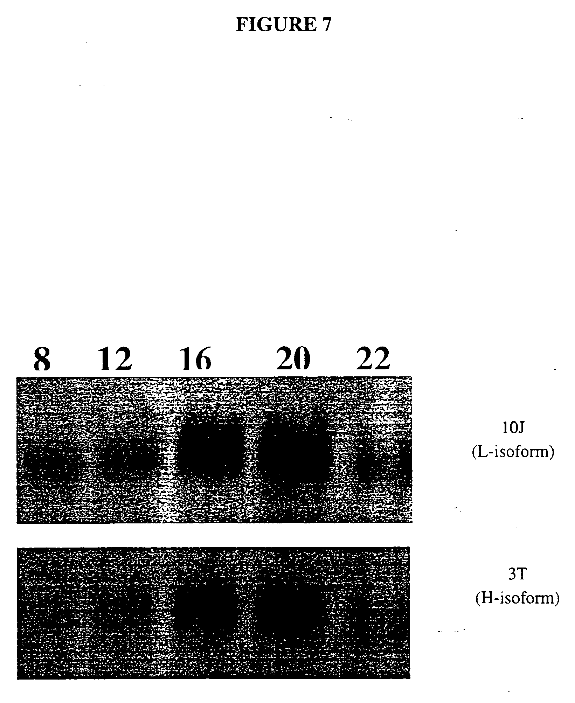

Figure 7 shows a Northern blot analysis of the developmental expression of flax oleosins during seed development.

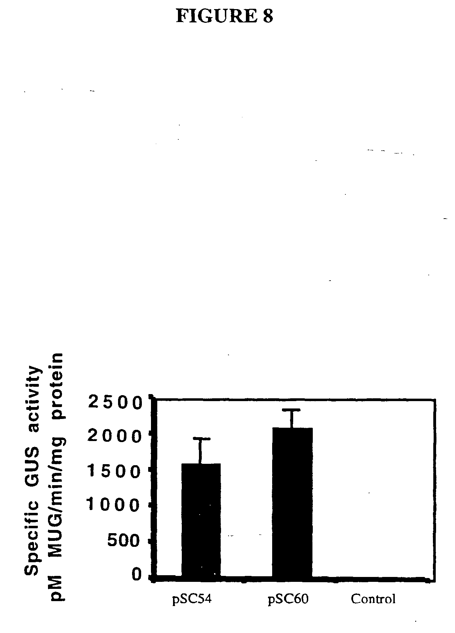

Figure 8 shows the GUS activity of flax embryos bombarded with flax oleosin promoter-GUS-flax terminator gene constructs.

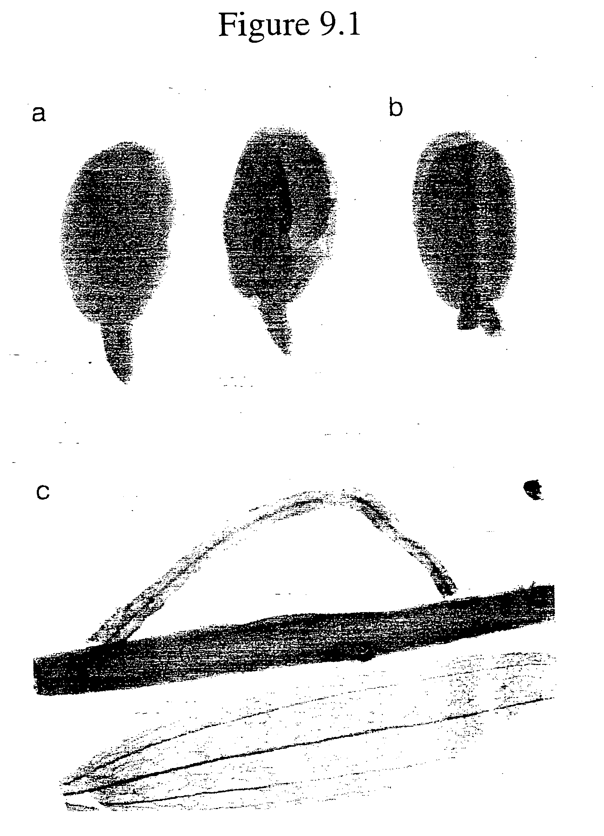

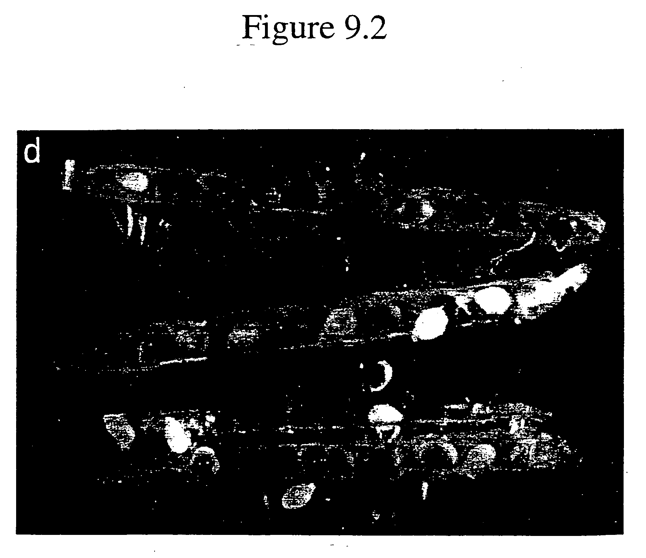

Figure 9 shows GUS expression in developing flax embryos and Arabidopsis seeds of plants transformed with a 2S protein gene promoter GUS fusion.

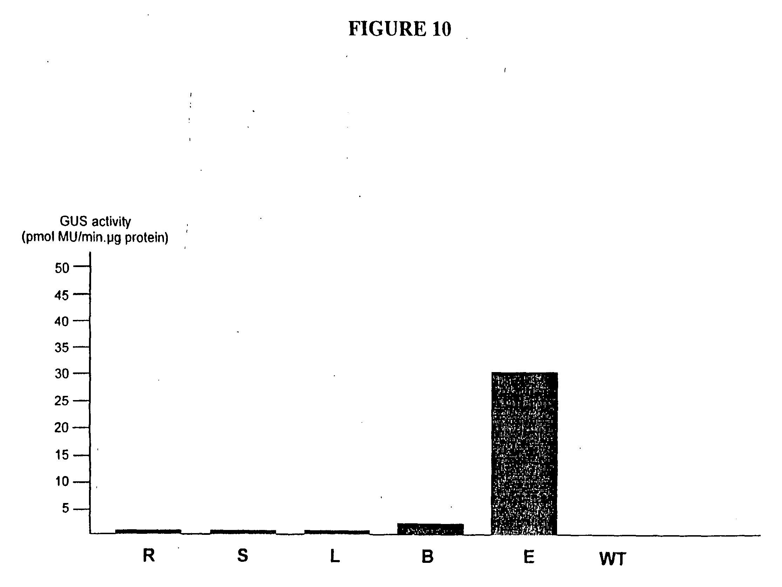

Figure 10 shows the tissue-specific expression of GUS in transgenic flax plants transformed with a linin promoter-GUS-linin terminator gene construct.

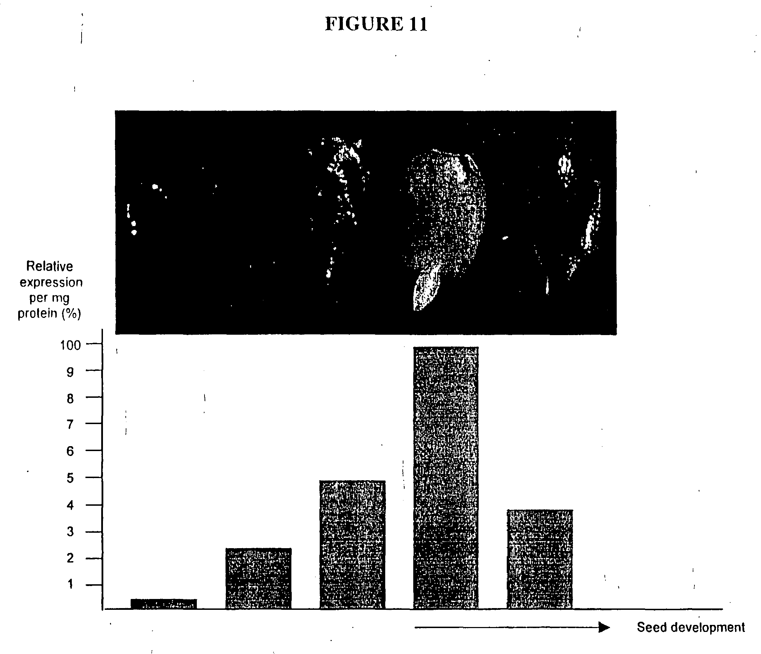

Figure 11 shows the temporal expression of GUS in transgenic flax plants transformed a linin promoter-GUS-linin terminator gene construct.

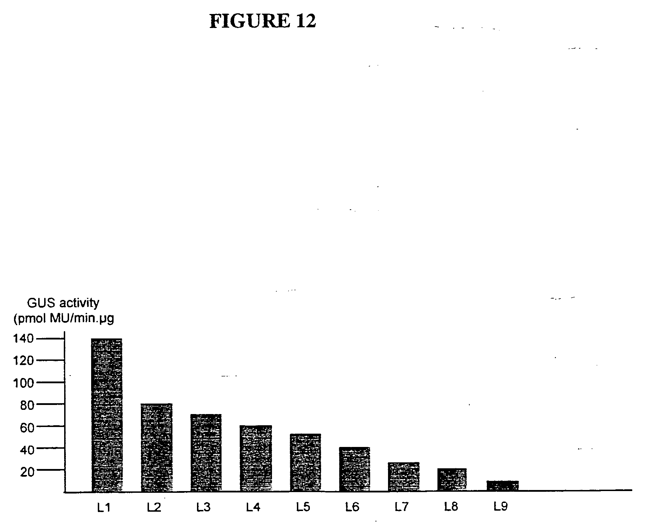

Figure 12 shows the expression of GUS in transgenic Brassica napus plants (L1 to L9) transformed with a linin promoter-GUS-linin terminator gene construct.

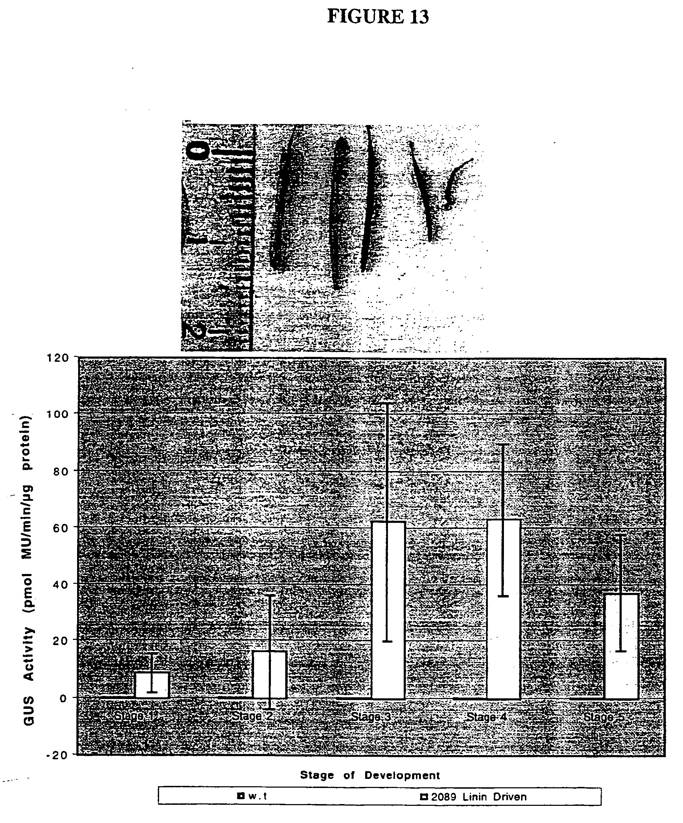

Figure 13 shows the expression of GUS in transgenic Arabidopsis plants transformed with a linin promoter-GUS-linin terminator gene construct at different stages of seed development.

### DETAILED DESCRIPTION OF THE INVENTION

As hereinbefore mentioned, the present invention relates to improved methods for the expression of non-native genes in plants, in particular flax. The invention provides methods allowing the seed-specific expression of non-native genes in flax. The methods of the invention are advantageous in that improved control over the expression of non-native genes in flax seeds is obtained. Expression of the non-native gene is restricted to the seeds, thereby limiting potential undesirable effects resulting from the expression in other plant organs or tissues. In addition, the provided methodology allows improved control over expression characteristics, such as the expression level of the non-native gene and timing of expression of the non-native gene in the developmental cycle of the plant. The methods of the present invention are particularly useful in that in accordance with the present invention the seed composition with respect to valuable raw materials, such as oil, protein and polysaccharides, may be altered both qualitatively and quantitatively.

Accordingly, in one aspect, the invention provides a method for the expression of a nucleic acid sequence of interest in flax seeds comprising:
(a) preparing a chimeric nucleic acid construct comprising in the 5' to 3' direction of transcription as operably linked components;
   (1) a seed-specific promoter obtained from flax; and
   (2) the nucleic acid sequence of interest wherein said nucleic acid of interest is non-native to said flax seed-specific promoter;
(b) introducing said chimeric nucleic acid construct into a flax plant cell; and
(c) growing said flax plant cell into a mature plant capable of setting seed, wherein said nucleic acid sequence of interest is expressed in the seed under the control of said flax seed-specific promoter, wherein seed-specific means that a gene expressed under the control of the promoter is predominantly expressed in plant seeds with less than 5% of the overall expression level in other plant tissues and wherein the flax seed-specific promoter is an oleosin promoter, a 2S storage protein promoter or a legumin-like seed storage protein promoter.

As used herein, the term "non-native" refers to any nucleic acid sequence, including any RNA or DNA sequence, which is not normally associated with the seed-specific promoter. This includes heterologous nucleic acid sequences which are obtained from a different plant species as the promoter as well as homologous nucleic acid sequences which are obtained from the same plant species as the promoter but are not associated with the promoter in the wild-type (non-transgenic) plant.

The non-native nucleic acid sequence when linked to a seed-specific promoter obtained from flax results in a chimeric construct. The chimeric construct is introduced into a flax plant cell to create a transgenic flax plant cell which results in a detectably different phenotype of the flax plant cell or flax plant grown from it when compared with a non-transgenic flax plant cell or flax plant grown from it. A contiguous nucleic acid sequence identical to the nucleic acid sequence of the chimeric construct is not present in the non-transformed flax plant cell or flax plant grown from it. In this respect, chimeric nucleic acid sequences include those sequences which contain a flax promoter linked to a nucleic acid sequence obtained from another plant species or a nucleic acid sequence from flax but normally not associated with that promoter. Chimeric nucleic acid sequences as used herein further include sequences comprising a flax promoter and a nucleic acid sequence that is normally linked to the promoter but additionally containing a non-native nucleic acid sequence. For example, if the promoter is a flax seed-specific oleosin promoter, sequences "non-native" to the flax oleosin promoter also include a sequence comprising a fusion between the flax oleosin gene naturally associated with the oleosin promoter, and a coding sequence of interest that is not naturally associated with the promoter. The term non-native is also meant to include a fusion gene as hereinabove which additionally includes a cleavage sequence separating the nucleic acid sequence that is normally linked to the promoter sequence and the gene encoding the protein of interest.

The term "nucleic acid sequence" refers to a sequence of nucleotide or nucleoside monomers consisting of naturally occurring bases, sugars and intersugar (backbone) linkages. The term also includes modified or substituted sequences comprising non-naturally occurring monomers or portions thereof, which function similarly. The nucleic acid sequences of the present invention may be ribonucleie (RNA) or deoxyribonucleic acids (DNA) and may contain naturally occurring bases including adenine, guanine, cytosine, thymidine and uracil. The sequences may also contain modified bases such as xanthine, hypoxanthine, 2-aminoadenine, 6-methyl, 2-propyl, and other alkyl adenines, 5-halo uracil, 5-halo cytosine, 6-aza uracil, 6-aza cytosine and 6-aza thymine, pseudo uracil, 4-thiouracil, 8-halo adenine, 8-amino adenine, 8-thiol adenine, 8-thio-alkyl adenines, 8-hydroxyl adenine and other 8-substituted adenines, 8-halo guanines, 8-amino guanine, 8-thiol guanine, 8-thioalkyl guanines, 8-hydroxyl guanine and other 8-substituted guanines, other aza and deaza uracils, thymidines, cytosines, adenines, or guanines, 5-trifluoromethyl uracil and 5-trifluoro cytosine.

Novel flax seed specific promoters useful for the expression of non-native genes in flax seeds and the seeds of other plant species are taught herein. The promoters may be used to modify for example the protein, oil or polysaccharide composition of the seeds. In a preferred embodiment, the seed specific promoter comprises:
(a) a nucleic acid sequence comprising nucleotides 1 to 2023 as shown in Figure 1 (SEQ.ID.NO.:1) wherein T can also be U, nucleotides 21 to 1852 as shown in Figure 2 (SEQ.ID.NO.:4) wherein T can also be U, nucleotides 1 to 400 as shown in Figure 3 (SEQ.ID.NO.:6) wherein T can also be U or nucleotides 1 to 2034 Figure 4 (SEQ.ID.NO.:8) wherein T can also be U;
(b) a nucleic acid sequence that is complimentary to a nucleic acid sequence of (a);
(c) a nucleic acid sequence that has at least 65% identity to a nucleic acid sequence of (a) or (b);
(d) a nucleic acid sequence that is an analog of a nucleic acid sequence of (a), (b) or (c); or
(e) a nucleic acid sequence that hybridizes to a nucleic acid sequence of (a), (b), (c) or (d) under stringent hybridization conditions.

The term "sequence that has substantial sequence homology" means those nucleic acid sequences which have slight or inconsequential sequence variations from the sequences in (a) or (b), i.e., the sequences function in substantially the same manner and are capable of driving seed specific expression of non-native nucleic acid sequences. The variations may be attributable to local mutations or structural modifications. Nucleic acid sequences having substantial homology include nucleic acid sequences having at least 65%, more preferably at least 85%, and most preferably 90-95% identity with the nucleic acid sequences comprising nucleotides 1 to 2023 as shown in Figure 1 (SEQ,ID.NO.:1) wherein T can also be U, nucleotides 21 to 1852 as shown in Figure 2 (SBQ.ID.N0.:4) wherein T can also be U, nucleotides 1 to 400 as shown in Figure 3 (SEQ.ID.NO.:6) wherein T can also be U or nucleotides 1 to 2034 as shown in Figure 4 (SEQ.ID.N0.:8) wherein T can also be U.

The term "sequence that hybridizes" means a nucleic acid sequence that can hybridize to a sequence of (a), (b), (c) or (d) under stringent hybridization conditions. Appropriate "stringent hybridization conditions" which promote DNA hybridization are known to those skilled in the art, or may be found in Current Protocols in Molecular Biology, John Wiley & Sons, N.Y. (1989), 6.3.1-6.3.6. For example, the following may be employed: 6.0 x sodium chloride/sodium citrate (SSC) at about 45°C, followed by a wash of 2.0 x SSC at 50°C. The stringency may be selected based on the conditions used in the wash step. For example, the salt concentration in the wash step can be selected from a high stringency of about 0.2 x SSC at 50°C. In addition, the temperature in the wash step can be at high stringency conditions, at about 65°C.

The term "a nucleic acid sequence which is an analog" means a nucleic acid sequence which has been modified as compared to the sequence of (a), (b) or (c) wherein the modification does not alter the utility of the sequence (i.e. as a seed specific promoter) as described herein. The modified sequence or analog may have improved properties over the sequence shown in (a), (b) or (c). One example of a modification to prepare an analog is to replace one of the naturally occurring bases (i.e. adenine, guanine, cytosine or thymidine) of the sequence shown in Figure 1, Figure 2, Figure 3 or Figure 4 with a modified base such as such as xanthine, hypoxanthine, 2-aminoadenine, 6-methyl, 2-propyl and other alkyl adenines, 5-halo uracil, 5-halo cytosine, 6-aza uracil, 6-aza cytosine and 6-aza thymine, pseudo uracil, 4-thiouracil, 8-halo adenine, 8-aminoadenine, 8-thiol adenine, 8-thiolalkyl adenines, 8-hydroxyl adenine and other 8-substituted adenines, 8-halo guanines, 8 amino guanine, 8-thiol guanine, 8-thiolalkyl guanines, 8-hydroxyl guanine and other 8-substituted guanines, other aza and deaza uracils, thymidines, cytosines, adenines, or guanines, 5-trifluoromethyl uracil and 5-trifluoro cytosine.

Another example of a modification is to include modified phosphorous or oxygen heteroatoms in the phosphate backbone, short chain alkyl or cycloalkyl intersugar linkages or short chain heteroatomic or heterocyclic intersugar linkages in the nucleic acid molecule shown in Figure 1, Figure 2, Figure 3 or Figure 4. For example, the nucleic acid sequences may contain phosphorothioates, phosphotriesters, methyl phosphonates, and phosphorodithioates.

A further example of an analog of a nucleic acid molecule of the invention is a peptide nucleic acid (PNA) wherein the deoxyribose (or ribose) phosphate backbone in the DNA (or RNA), is replaced with a polyamide backbone which is similar to that found in peptides (P.E. Nielsen, et al Science 1991, 254,1497). PNA analogs have been shown to be resistant to degradation by enzymes and to have extended lives *in vivo* and *in vitro.* PNAs also bind stronger to a complimentary DNA sequence due to the lack of charge repulsion between the PNA strand and the DNA strand. Other nucleic acid analogs may contain nucleotides containing polymer backbones, cyclic backbones, or acyclic backbones. For example, the nucleotides may have morpholino backbone structures (U.S. Pat. No. 5,034,506). The analogs may also contain groups such as reporter groups, a group for improving the pharmacokinetic or pharmacodynamic properties of nucleic acid sequence.

In another aspect, the invention provides an isolated chimeric nucleic acid sequence comprising
(a) a first nucleic acid sequence comprising a seed-specific promoter obtained from flax which comprises:
   (1) a nucleic acid sequence comprising nucleotides 1-2023 as shown in Figure 1 (SEQ ID NO. 1), wherein T can also be U; a nucleic acid sequence comprising nucleotides 21-1852 as shown in Figure 2 (SEQ ID NO. 4), wherein T can also be U; a nucleic acid sequence comprising nucleotides 1-400 as shown in Figure 3 (SEQ ID NO. 6), wherein T can also be U; or a nucleic acid sequence comprising nucleotides 1-2034 as shown in Figure 4 (SEQ ID NO. 8), wherein T can also be U;
   (2) a nucleic acid sequence that hybridizes to a nucleic acid sequence of (a) under stringent hybridization conditions;
   (3) a nucleic acid sequence that is complimentary to a nucleic acid sequence of (a); or
   (4) a nucleic acid sequence that has at least 65% identity to a nucleic acid sequence of (a); and
(b) a second nucleic acid sequence non-native to said flax seed-specific promoter; wherein seed-specific means that a gene expressed under the control of the promoter is predominantly expressed in plant seeds with less than 5% of the overall expression level in other plant tissues.

In accordance with the present invention, the chimeric nucleic acid sequences can be incorporated in a known manner in a recombinant expression vector which ensures good expression in the seed cell. Accordingly, the present invention includes a recombinant expression vector comprising a chimeric nucleic acid sequence of the present invention suitable for expression in a seed cell.

The term "suitable for expression in a seed cell" means that the recombinant expression vectors contain the chimeric nucleic acids sequence of the invention, a regulatory region and a termination region, selected on the basis of the seed cell to be used for expression, which is operatively linked to the nucleic acid sequence encoding the polypeptide of desirable amino acid composition. Operatively linked is intended to mean that the chimeric nucleic acid sequence encoding the polypeptide is linked to a regulatory sequence and termination region which allows expression in the seed cell. A typical construct consists, in the 5' to 3' direction of a regulatory region complete with a promoter capable of directing expression in a plant, a polypeptide coding region and a transcription termination region functional in plant cells. These constructs may be prepared in accordance with methodology well known to those of skill in the art of molecular biology (see for example: Sambrook et al. (1990), Molecular Cloning, 2nd ed Cold Spring Harbor Press). The preparation of constructs may involve techniques such as restriction digestion, ligation, gel electrophoresis, DNA sequencing and PCR. A wide variety of cloning vectors is available to perform the necessary cloning steps. Especially suitable for this purpose are the cloning vectors with a replication system that is functional in Escherichia coli such as pBR322, the pUC series M13mp series, pACYC184, pBluescript etc. Nucleic acid sequences may be introduced into these vectors and the vectors may be used to transform E. coli which may be grown in an appropriate medium. Plasmids may be recovered from the cells upon harvesting and lysing the cells. Final constructs may be introduced into plant vectors compatible with integration into the plant such as the Ti and Ri plasmids.

In preferred embodiments of the present invention, the flax seed-specific promoter confers to the non-native nucleic acid sequence at least one expression characteristic which is similar or identical to an expression characteristic conferred to the native nucleic acid sequence by the native promoter. The term "expression characteristic" as used herein refers to any measurable property or effect conferred by the flax seed-specific promoter to the nucleic acid sequence operably linked to the flax seed-specific promoter. Thus in preferred embodiments, timing of expression in the plant's life cycle, of the non-native nucleic acid sequence is similar or identical to timing of expression of the native nucleic acid sequence. In further preferred embodiments, the expression level of the heterologous nucleic acid sequence is similar or identical to the expression level of the native nucleic acid sequence. In yet further specific embodiments, the response of the non-native gene to alterations in lighting conditions, changes in wavelength or light intensity for example, changes in temperature, tissue wounding, changes in concentration of chemical agents, such as for example phytohormones and pesticides, is similar to the response of the native nucleic acid sequence to these stimuli. Other desired expression characteristics conferred by a flax seed-specific promoter may be recognized by those skilled in the art and a flax seed-specific promoter may be selected accordingly.

In the present invention the seed specific promoter used is an oleosin promoter, a legumin-like seed storage protein promoter or a 2S storage protein promoter. In particularly preferred embodiments the seed specific promoter has the sequence comprising nucleotides 1 to 2023 shown in Figure 1 wherein T can also be U, nucleotides 21 to 1852 as shown in Figure 2 wherein T can also be U, nucleotides 1 to 400 as shown in Figure 3 wherein T can also be U or nucleotides 1 to 2034 as shown in Figure 4 wherein T can also be U or any nucleic acid sequences obtainable from flax and hybridizing to any one of these four nucleic acid sequences under stringent conditions.

Additional flax seed-specific promoters may be used in accordance with the present invention. These promoters may be obtained in a number of ways. Where a flax seed protein has been isolated, it may be partially sequenced, so that a nucleic acid probe may be designed for identifying RNA specific to the seed. To further enhance the RNA specifically associated with the seed, cDNA may be prepared from seed cells and the cDNA may be subtracted with mRNA or cDNA from non-seed cells. The remaining seed cDNA may then be used to probe a genomic DNA library for complementary sequences. Sequences hybridizing to the cDNA may subsequently be obtained and the associated promoter region may be isolated. It is also possible to screen genomic DNA libraries prepared from flax seed tissues using known seed specific genes from other plant species and subsequently isolate their associated promoters. Due to the relative abundance of seed-storage proteins in seeds it is also be possible to obtain sequence information through random sequencing of flax seed cDNA libraries. Those cDNA sequences matching sequence of known seed-storage proteins could be used to identify the associated promoter. Databases containing sequence information from large scale sequencing from for example Arabidopsis and maize may be searched for known seed-specific proteins and/or promoters and the information may be used to identify promoter sequences in flax that share sequence similarity. Alternative methods to isolate additional flax seed specific promoters may be used and novel flax seed specific promoters may be discovered by those skilled in the art and used in accordance with the present invention.

The nucleic acid sequence of interest linked to the promoter may be any nucleic acid sequence of interest including any RNA or DNA sequence encoding a peptide or protein of interest, for example, an enzyme, or a sequence complementary to a genomic sequence, where the genomic sequence may be at least one of an open reading frame, an intron, a noncoding leader sequence, or any sequence where the complementary sequence will inhibit transcription, messenger RNA processing, for example splicing or translation. The nucleic acid sequence of interest may be synthetic, naturally derived or a combination thereof. As well, the nucleic acid sequence of interest could be a fragment of the natural sequence, for example just include the catalytic domain or a structure of particular importance. Depending upon the nature of the nucleic acid sequence of interest, it may be desirable to synthesize the sequence with plant preferred codons. The plant preferred codons may be determined from the codons of highest frequency in the proteins expressed in the largest amount in particular plant species of interest.

The nucleic acid sequence of interest may encode any of a variety of recombinant proteins. Examples of recombinant proteins which might be expressed by the methods of the present invention include proteins with a favorable catalytic function or a valuable protein that will accumulate to high levels and be extracted if desired. Proteins with a catalytic function, include, but are not limited to, proteins that confer a new biochemical phenotype on the developing seeds. New phenotypes could include such modifications as altered seed-protein or seed oil composition or seed polysaccharide composition, enhanced production of pre-existing desirable products or properties and the reduction or even suppression on an undesirable gene product using antisense, ribozyme or co-supression technologies (Izant and Weintraub (1984) Cell 26: 1007-1015, antisense; Hazelhoff and Gerlach (1988) Nature 334: 585-591, ribozyme; Napoli et al. (1990) Plant Cell 2: 279-289, co-suppression).

It is expected that the desired proteins would be expressed in all embryonic tissues, although varying cellular expression may be detected in the different embryonic tissues such as the embryonic axis and cotyledons. The nucleic acid sequence of interest may be expressed at any stage in seed development. The timing of expression may depend on the particular use of the invention. Expression of enzymes involved in oil modification may be desirable early in seed development, for example before accumulation of seed storage protein.

Besides the promoter region and the nucleic acid sequence of interest, a nucleic acid sequence capable of terminating transcription is typically included in expression vectors. Transcriptional terminators are preferably about 200 to about 1,000 nucleotide base pairs and may comprise any such sequences functional in plants, such as the nopaline synthase termination region (Bevan et al., (1983) Nucl. Acid. Res. 11: 369-385), the phaseolin terminator (van der Geest et al., (1994) Plant J. 6(3): 413-423), the terminator for the octopine synthase gene of Agrobacterium tumefaciens or other similarly functioning elements. These transcription terminator regions can be obtained as described by An (1987), Methods in Enzym. 153: 292 or are already present in plasmids available from commercial sources such as ClonTech, Palo Alto, California. The choice of the appropriate terminator may have an effect of the rate of transcription.

The chimeric construct may further comprise enhancers such as the AMV leader (Jobling and Gehrke (1987), Nature 325: 622-625) or introns. It should be understood that the design of the expression vector may depend on such factors as the choice of the plant species and/or the type of polypeptide to be expressed.

The expression vectors will normally also contain a marker gene. Marker genes comprise all genes that enable distinction of transformed plant cells from non-transformed cells, including selectable and screenable marker genes. Conveniently, a marker may be a resistance marker to a herbicide, for example, glyphosate or phosphinothricin, or to an antibiotic such as kanamycin, G418, bleomycin, hygromycin, chloramphenicol and the like, which confer a trait that can be selected for by chemical means. Screenable markers may be employed to identify transformants through observation. They include but are not limited to the b-glucuronidase or uidA gene, a b-lactamase gene or a green fluorescent protein (Niedz et al. (1995) Plant Cell Rep. 14: 403).

In order to introduce nucleic acid sequences into plant cells in general a variety of techniques are available to the skilled artisan. Agrobacterium-mediated transformation for flax plant cells has been reported and flax transformants may be obtained in accordance with the methods taught by Dong and McHughen (1993) Plant Science 88: 61-77, although a variety of other techniques (see below) may also be used to introduce the chimeric DNA constructs in flax cells if so desired.

Transformed flax plants grown in accordance with conventional agricultural practices known to a person skilled in the art are allowed to set seed. Flax seed may then be obtained from mature flax plants and analyzed for desired altered properties with respect to the wild-type seed.

Two or more generations of plants may be grown and either crossed or selfed to allow identification of plants and strains with desired phenotypic characteristics including production of the recombinant polypeptide. It may be desirable to ensure homozygosity in the plants to assure continued inheritance of the recombinant trait. Methods for selecting homozygous plants are well known to those skilled in the art of plant breeding and include recurrent selfing and selection and anther and microspore culture. Homozygous plants may also be obtained by transformation of haploid cells or tissues followed by regeneration of haploid plantlets subsequently converted to diploid plants by any number of known means (e.g. treatment with colchicine or other microtubule disrupting agents).

The present invention also includes transgenic flax seeds prepared according to a method comprising:
(a) preparing a chimeric nucleic acid construct comprising in the 5' to 3' direction of transcription as operably linked components:
   (1) a seed-specific promoter obtained from flax; and
   (2) a nucleic acid sequence of interest wherein said nucleic acid of interest is non-native to said seed-specific promoter;
(b) introducing said chimeric nucleic acid construct into a flax plant cell; and
(c) growing said flax plant cell into a mature flax plant capable of setting seed wherein said nucleic acid sequence of interest is expressed in the seed under the control of said seed-specific promoter, wherein seed-specific means that a gene expressed under the control of the promoter is predominantly expressed in plant seeds with less than 5% of the overall expression level in other plant tissues and wherein the flax seed-specific promoter is an oleosin promoter, a 2S storage protein promoter or a legumin-like seed storage protein promoter.

Specific promoter sequences comprise nucleotides 1-2023 as shown in Figure 1 (SEQ ID NO. 1) wherein T can also be U, nucleotides 21-1852 as shown in Figure 2 (SEQ.ID.NO.:4) wherein T can also be U, nucleotides 1-400 as shown in Figure 3 (SEQ.ID.NO.:6) wherein T can also be U and nucleotides 1 to 2034 as shown in Figure 4 (SEQ.ID.NO.:8) wherein T can also be U.

The present invention further provides flax plants capable of setting seed prepared by a method comprising:
(a) preparing a chimeric nucleic acid construct comprising in the 5' to 3' direction of transcription as operably linked components:
   (1) a seed-specific promoter obtained from flax; and
   (2) a nucleic acid sequence of interest wherein said nucleic acid of interest is non-native to said seed-specific promoter
(b) introducing said chimeric nucleic acid construct into a flax plant cell; and
(c) growing said flax plant cell into a mature flax plant capable of setting seed wherein said nucleic acid sequence of interest is expressed in the seed under the control of said seed-specific promoter, wherein seed-specific means that a gene expressed under the control of the promoter is predominantly expressed in plant seeds with less than 5% of the overall expression level in other plant tissues and wherein the flax seed-specific promoter is an oleosin promoter, a 2S storage protein promoter or a legumin-like seed storage protein promoter.

The present invention further provides methods of use for the novel promoters comprising nucleotides 1 to 2023 as shown in Figure 1 (SEQ.ID.NO.:1) wherein T can also be U, nucleotides 21 to 1852 as shown in Figure 2 (SEQ.ID.NO.:4) wherein T can also be U, nucleotides 1 to 400 as shown in Figure 3 (SEQ.ID.NO.:6) wherein T can also be U and nucleotides 1 to 2034 as shown in Figure 4 (SEQ.ID.NO.:8) wherein T can also be U, in plant species other than flax. Accordingly, the preparation of chimeric nucleic acid constructs comprising a promoter selected from the group of promoters comprising nucleotides 1 to 2023 as shown in Figure 1 wherein T can also be U, nucleotides 21 to 1852 as shown in Figure 2 wherein T can also be U, nucleotides 1 to 400 as shown in Figure 3 wherein T can also be U, and nucleotides 1 to 2034 as shown in Figure 4 wherein T can also be U; and a nucleic acid sequence of interest, and expression in a seed-specific manner of the nucleic acid sequence of interest in plant species other than flax and under the control of the flax promoter are taught herein.

In another aspect of the present invention there is provided a method for the expression of a nucleic acid sequence of interest in plant seeds comprising:
(a) introducing the chimeric nucleic acid sequence comprising
   (1) a first nucleic acid sequence comprising a seed-specific promoter obtained from flax which comprises:
      (i) a nucleic acid sequence comprising nucleotides 1-2023 as shown in Figure 1 (SEQ ID NO. 1), wherein T can also be U; a nucleic acid sequence comprising nucleotides 21-1852 as shown in Figure 2 (SEQ ID NO. 4), wherein T can also be U; a nucleic acid sequence comprising nucleotides 1-400 as shown in Figure 3 (SEQ ID NO. 6), wherein T can also be U; or a nucleic acid sequence comprising nucleotides 1-2034 as shown in Figure 4 (SEQ ID NO. 8), wherein T can also be U;
      (ii) a nucleic acid sequence that hybridizes to a nucleic acid sequence of (a) under stringent hybridization conditions;
      (iii) a nucleic acid sequence that is complimentary to a nucleic acid sequence of (a); or
      (iv) a nucleic acid sequence that has at least 65% identity to a nucleic acid sequence of (a); and
   (2) a second nucleic acid sequence non-native to said flax seed-specific promoter; wherein seed-specific means that a gene expressed under the control of the promoter is predominantly expressed in plant seeds with less than 5% of the overall expression level in other plant tissues into a plant cell,
(b) growing said plant cell into a mature plant capable of setting seed, wherein said nucleic acid sequence of interest is expressed in the seed under the control of said seed-specific promoter.

A variety of techniques are available for the introduction of nucleic acid sequences, in particular DNA, into plant host cells in general. For example, the chimeric DNA constructs may be introduced into host cells obtained from dicotelydenous plants, such as tobacco, and oleoagenous species, such as *Brassica napus* using standard *Agrobacterium* vectors by a transformation protocol such as described by Moloney *et al.* (1989), Plant Cell Rep. 8: 238-242 or Hinchee *et al.* (1988) Bio/Technol. 6: 915-922; or other techniques known to those skilled in the art. For example, the use of T-DNA for transformation of plant cells has received extensive study and is amply described in EP 0 120 516, Hoekema *et al.,* (1985), Chapter V In: *The Binary Plant Vector System* Offset-drukkerij Kanters BV, Alblasserdam); Knauf *et al.* (1983), *Genetic Analysis of Host Expression by Agrobacterium,* p. 245, In: *Molecular Genetics of Bacteria-Plant Interaction,* Puhler, A. ed. Springer-Verlag, NY); and An *et al.,* (1985), (EMBO J., 4; 277-284). *Agrobacterium* transformation may also be used to transform monocot plant species (US Patent 5,591,616).

Conveniently, explants may be cultivated with *Agrobacterium tumefaciens* or *Agrobacterium rhizogenes* to allow for the transfer of the transcription construct in the plant host cell. Following transformation using *Agrobacterium* the plant cells are dispersed into an appropriate medium for selection, subsequently callus, shoots and eventually plants are recovered. The *Agrobacterium* host will harbour a plasmid comprising the vir genes necessary for transfer of the T-DNA to plant cells. For injection and electroporation (see below) disarmed Ti-plasmids (lacking the tumour genes, particularly the T-DNA region) may be introduced into the plant cell.

The use of non-*Agrobacterium* techniques permits the use of constructs described herein to obtain transformation and expression in a wide variety of monocotyledonous and dicotyledonous plant species. These techniques are especially useful for transformation of plant species that are intractable in an *Agrobacterium* transformation system. Other techniques for gene transfer include particle bombardment (Sanford, (1988), Trends in Biotechn. 6: 299-302), electroporation (Fromm *et al.,* (1985), PNAS USA, 82: 5824-5828; Riggs and Bates, (1986), PNAS USA 83: 5602-5606), PEG mediated DNA uptake (Potrykus *et al.,* (1985), Mol. Gen. Genetics., 199: 169-177), microinjection (Reich *et al.,* Bio/Techn. (1986) 4:1001-1004) and silicone carbide whiskers (Kaeppler *et al.* (1990) Plant Cell Rep. 9: 415-418).

In a further specific applications such as to *B. napus,* the host cells targeted to receive recombinant DNA constructs typically will be derived from cotyledonary petioles as described by Moloney *et al.* (1989) Plant Cell Rep. 8: 238-242. Other examples using commercial oil seeds include cotyledon transformation in soybean explants (Hinchee *et al.,* (1988) Bio/Technol. 6: 915-922) and stem transformation of cotton (Umbeck *et al.,* (1987) Bio/Technol. 5: 263-266).

Following transformation, the cells, for example as leaf discs, are grown in selective medium. Once the shoots begin to emerge, they are excised and placed onto rooting medium. After sufficient roots have formed, the plants are transferred to soil. Putative transformed plants are then tested for presence of a marker. Southern blotting is performed on genomic DNA using an appropriate probe, to show integration into the genome of the host cell.

The methods provided by the present invention can be used in conjunction a broad range of plant species. Particularly preferred plant cells employed in accordance with the present invention include cells from the following plants: soybean (*Glycine max*), rapeseed (*Brassica napus, Brassica campestris*), sunflower (*Helianthus annuus*), cotton (*Gossypium hirsutum*)*,* corn (*Zea mays*), tobacco (*Nicotiana tobacum*), alfalafa (*Medicago sativa*)*,* wheat (*Triticum sp.*), barley (*Hordeum vulgare*), oats (*Avena sativa* L.), sorghum (*Sorghum bicolor*)*, Arabidopsis thaliana,* potato (*Solanum sp.*), flax/linseed (*Linum usitatissimum*)*,* safflower (*Carthamus tinctorius*)*,* oil palm (*Eleais guineeis*), groundnut (*Arachis hypogaea*)*,* Brazil nut (*Bertholletia excelsai* coconut (*Cocus nucifera*), castor (*Ricinus communis*)*,* coriander (*Coriandrum sativum*)*,* squash (*Cucurbita maxima*)*,* jojoba (*Simmondsia chinensis*) and rice (*Oryza sativa*)*.*

The present invention has a variety of uses which include improving the intrinsic value of plant seeds by their accumulation of altered polypeptides or novel recombinant peptides or by the incorporation or elimination or a metabolic step. Use of the invention may result in improved protein quality (for example, increased concentrations or essential or rare amino acids), improved liquid quality by a modification of fatty acid composition, or improved or elevated carbohydrate composition. Examples include the expression of sulfur-rich proteins, such as those found in lupins or brazil nuts in a seed deficient in sulphurous amino acids. Improved protein quality could also be achieved by the expression of a protein or a fragment of a protein that is enriched in essential amino acids including lysine, cysteine, methionine and tryptophan. Alternatively, a fatty acyl coenzyme A, a transferase enzyme capable of modifying fatty acid ratios in triglycerides (storage lipid) could be expressed. In cases where a recombinant protein is allowed to accumulate in the seed, the protein could also be a peptide which has pharmaceutical or industrial value. In this case the peptide could be extracted from the seed and used in crude or purified form as appropriate for the intended use. As well, the polypeptides that are expressed in the seeds can be fragments or derivatives or the native protein. Pharmaceutically useful proteins may include, but are not limited to, anticoagulants, such as hirudin, antibodies, including monoclonal antibodies and antibody fragments, vaccines, cytokines or growth factors such as bovine growth factor, cholinergic differentiation factor (CDF), ciliary neurotrophic factor (CNTF), fibroblast growth factor (FGF), fish growth factor, gonadotropin, granulocyte-colony stimulating factor (G-CSF), granulocyte-macrophage colony-stimulating factor (GM-CSF), human growth hormone, interferon alpha (1FN-a), interferon beta (IFN-b), interferon gamma (IFN-g), interleukin 1-alpha (IL1-a), interleukin 1-beta (IL1-b), interleukin-2 (IL-2), interleukin-3 (IL-3), interleukin-4 (IL-4), interleukin-5 (IL-5), interleukin-6 (IL-6), interleukin-10 (IL-10), leukemia inhibitory factor (LIF), thioredoxin, macrophage colony-stimulating factor (M-CSF), myelornonocytic growth factor, nerve growth factor (NGF), oncostatin M, platelet-derived growth factor (PDGF), prolactin, transforming growth factor alpha (TGF-a), transforming growth factor beta2 (TGF-b2), tumour necrosis factor alpha (TNF-a), and tumour necrosis factor beta (TNF-b). Pharmaceutically useful proteins can also include mammalian proteins, for example, but not limited to a-1-antitrypsin, anti-obesity proteins, blood proteins, collagen, collagenase, elastin, elastase, enteropeptidase, fibrinogen, haemoglobin, human serum albumin, insulin, lactoferrin, myoglobin and pulmonary surfactant proteins.

Industrially useful peptides may include, but are not limited to a-amylase or other amylases, amyloglucosidase, arabinase, catalase, cellobiohydrolase, cellulases, chitinases, chymotrypsin, dehydrogenases, endo-glucanase, chymosin, endo-galactanase, esterases, b-galactosidase, a-galactosidase or other galactosidases, gastric lipases, glucanases, glucose isomerase, hemi-cellulases, hydrolases, isomerase, ligninases, lipases, lyases, lysozymes, oxidases, oxidoreductase, papain, pectinases, pectin lyase, peroxidases, phosphatases, phytase, proteases, pullulanases, reductases, serine proteases, thioredoxin, transferase, trypsin, and xylanase.

The following non-limiting examples are illustrative of the present invention:

### EXAMPLES

### EXAMPLE 1

### Isolation of Seed-Specific Flax Promoters

Seed specific cDNA clones were isolated form a flax seed specific cDNA-library. These cDNA clones were sequenced and the Basic Local Alignment Search Tool (BLAST) was used to compare these sequences against others in public databases such as Genbank. This comparison revealed that the deduced amino acid sequence of several of the isolated cDNAs had a high degree of similarity to both the low and high molecular weight class of oleosins, 2S-albumin and legumin-like storage proteins. Probes were prepared individually from (portions of) cDNAs encoding oleosins, 2S albumin and legumin-like storage proteins and these were used to screen a genomic library prepared from the flax line Forge that is homozygous for four rust resistance genes (Anderson et al. (1997), The Plant Cell 9: 641-651). Several positive lambda clones for each probe were identified after high-stringency screening. The inserts were subcloned into the plasmid vector pBluescript and sequenced. Sequence information revealed that we had isolated the genomic counterparts to the oleosins, 2S albumin and cDNAs *legumin-like* cDNAs. Sequence information of the genomic clones containing sequences encoding a high and low molecular weight oleosin isoforms, 2S albumin and a legumin-like gene are presented in Figures 1 to 4 respectively.

Figure 1 and SEQ.ID.NO.:1 shows the DNA sequence of a flax genomic clone encoding a 16.0 kDa oleosin protein (low molecular weight or L-isoform). Putative regulatory elements are identified and indicated. These include inverted repeats (base pairs 805 to 813 and 821 to 829; base pairs 1858 to 1866 and 1877 to 1885), direct repeats (base pairs 184 to 193 and 1102 and 1111; base pairs 393 to 402 and 1701 to 1710; base pairs 683 to 692 and 1546 to 1555; base pairs 770 to 781 and 799 to 810; base pairs 955 to 964 and 1936 to 1945; base pairs 1483 to 1496 and 1513 to 1526), the abscisic acid responsive element (ABRE) (base pairs 1859 to 1866), CACA box (base pairs 1933 to 1936), TATA box (base pairs 1925 to 1931) and CAT box (base pairs 1989 to 1993). As well, the poly adenylation signal is indicated (base pairs 3020 to 3025). The open reading frame is interrupted by 1 short intron (which are marked) and the 2 exons are translated and indicated in IUPAC single letter amino-acid codes.

Figure 2 and SEQ.ID.NO.:4 shows the DNA sequence of a flax genomic clone encoding a 18.6 kDa oleosin protein (high molecular weight or H-isoform). Putative regulatory elements are identified and indicated. These include direct repeats (base pairs 14 to 25 and 1427 to 1438; base pairs 80 to 89 and 1242 to 1251; base pairs 177 to 186 and 837 to 846; base pairs 1281 to 1290 and 1242 to 1251; base pairs 1591 to 1600 and 1678 to 1287). The open reading frame is not intermpted by introns and is translated and indicated in IUPAC single letter amino-acid codes.

Figure 3 and SEQ.ID.NO.:6 shows the DNA sequence of the flax genomic clone encoding a 2S storage protein. Nucleotide sequencing of this clone revealed it to have an open reading frame of 174 amino acids that showed homology to the plant 2S storage group of proteins. The sequence encodes an open reading frame with 38% overall similarity to a *Brassica oleracea* 2S storage protein, including complete conservation of the glutamine-rich stretch QQQGQQQGQQQ (SEQ.ID.NO.:13). In addition, the 2S storage protein gene promoter contained several putative promoter regulatory elements, These include AT rich repeats (base pairs 25-36, 97-108 and 167-190), RY-like repeat (base pairs 240-247), G-box-like element (base pairs 274-280), seed specific box-like motif (base pairs 285-290) and TATA box (base pairs 327-333).

Figure 4 and SEQ.ID.NO.:8 shows the DNA sequence of a flax genomic clone encoding a 54.4 kDa flax legumin-like seed storage protein. This legumin-like seed storage protein gene will also be referred to as "linin". The deduced amino acid sequence of the linin gene was compared to the *legumin-like* protein from *R. communis,* the legumin precursor from *M. salicifolia, Q.robur* and *G. hirsutum,* the glutelin precursor from *O.sativa* and a 12 S seed storage protein from *A. thaliana.* The linin gene shows a sequence identity/similarity with the corresponding proteins from *R. communis, M. salicifolia, Q.robur, G. hirsutum, O.sativa* and *A. thaliana* of 59/15, 47/16, 50/17, 45/17, 43/18 and 43/18 percent respectively. Putative regulatory elements in the promoter region are identified and indicated. These include inverted repeats (base pairs 265 to 276 and 281 to 292; base pairs 513 to 524 and 535 to 545), repeats (base pairs 1349 to 1360 and 1367 to 1378; base pairs 1513 to 1529 and 1554 to 1572), the abscisic acid responsive element (ABRE) (base pairs 1223 and 1231), legumin box (RY repeats) (between base pairs 1223 and 1231), a possible vicilin box region (base pairs 1887 to 1894), CAAT box (base pairs 1782 to 1785) and TATA box (base pairs 1966 to 1970). As well, the signal peptide for ER membrane targeting is indicated (base pairs 2034-2080). The open reading frame is interrupted by 3 short introns (which are marked) and the 4 exons are translated and indicated in IUPAC single letter amino-acid codes.

Figure 5 shows Southern blot analysis of flax genomic DNA. 60 µg of flax genomic DNA was isolated from leaves, digested with EcoRI (lane I), HindIII (lane 2) and BamHI (lane 3) and was loaded into the respective lanes. A) Hybridizations were performed with random primed 32P-labelled 3T cDNA (high molecular weight flax oleosin isoform). B) Hybridizations were performed with random primed 32P-labelled 7R cDNA (low molecular weight flax oleosin isoform). The results demonstrate that both 3T (high molecular weight oleosin isoform) and 7R (low molecular weight oleosin isoform) oleosin cDNAs hybridize with flax genomic bNA. More specifically the results indicate that 3T is likely to represent a 2-copy gene in flax, as seen by two bands in each lane of digestion. Similarly, 7R is likely to represent a multigene family in flax as multiple bands were detected for each digestion.

### EXAMPLE 2

### Seed specific expression of flax oleosin genes

Figure 6 shows a Northern blot analysis of the seed specific expression of flax oleosins. Northern hybridization of the two oleosin mRNA in different tissues. Ten mg of total RNA was extracted from different tissues, R, root; C, cotyledon; L, leaf; S, seed capsule; E, embryo. The membrane was probed with (A) cDNA encoding high molecular weight (H)-isoform (identical to coding sequence as presented in Figure 2) and (B) cDNA encoding low molecular weight (L) -isoform (identical to coding sequence as presented in Figure 1). Both the transcripts are expressed only in the embryo and seed capsule, which contains embryos.

### EXAMPLE 3

### Developmental expression of flax oleosin genes during seed development

Figure 7 shows a Northern blot analysis of the developmental expression of flax oleosins during seed development. 15 µg per lane of total RNA was loaded in each lane on agarose/formaldehyde gel and blotted onto HybondN+ membrane. 10J: This membrane was probed using the 32P dCTP labeled flax oleosin cDNA clone (low molecular weight isoform). Stages indicated are the number of days past anthesis (DPA). 3T) 15 µg per lane of total RNA was loaded in each lane on agarose/formaldehyde gel and blotted onto HybondN+ membrane. 3T: This membrane was probed using the 32P dCTP labeled flax oleosin cDNA clone (high molecular weight isoform). Both the transcripts were expressed very early in development (6DPA, early cotyledonary stage). Expression is maximum at 16 to 20 DPA (late cotyledonary stage) and declines at 22 DPA (mature embryos).

### EXAMPLE 4

### Transient Seed specific expression of b-glucuronidase (GUS) when under the regulatory control of flax oleosin regulatory sequences

Two constructs were made using standard molecular biology techniques (eg see *Sambrook et al.* (1990), Molecular Cloning, 2nd ed. Cold Spring Harbor Press, including restriction enzyme digestions, ligation and polymerase chain reaction (PCR),
Construct pSC54: The β-glucuxonidase reporter coding sequence from vector GUSN358>S (Clontech Laboratories) was placed between the promoter sequence from nucleotide 21 to 1852 and terminator sequence from 2395 to 3501 (as described in Figure 2). This insert was cloned into pBluescript and the resulting vector is called pSC54
Construct pSC60: The b-glucuronidase reporter coding sequence from vector GUSN358>S (Clontech Laboratories) was placed between the promoter sequence from nucleotide 1 to 2023 and terminator sequence from 2867 to 3925 (as described in Figure 1). This insert was cloned into pBluescript and the resulting vector is called pSC60.

pSC54, pSC60 and a promoter-less GUS construct (Control) were introduced into the flax embryos using particle bombardment using standard protocols (eg see Abenes et al. (1997) Plant Cell reports 17:1-7). Figure 8 shows the GUS activity of flax embryos bombarded with pSC54, pSC60 and a promoterless GUS construct measured 48 hours after particle bombardment. As can be seen the flax oleosin regulatory sequences are sufficient to drive the expression of GUS in flax embryos.

### EXAMPLE 5

### Stable seed specific expression of b-glucuronidase (GUS) in flax and Arabidopsis when under the regulatory control of flax 2S storage protein gene promoter

A GUS reporter gene construct was made by incorporating 5' and 3' regions from the DNA fragment described in Figure 3 into promoterless-GUS pBI101 vector as follows.

A 400bp amplicon from the 5' end of the DNA fragment described in Figure 3 was PCR amplified using the following primers (location shown in Fig 3):
5' primer(1): 5'-TCCACTATGTAGCTCATA-3' (SEQ.ID.NO.:14)
3' primer(1): 5'-CTTTAAGGTGTGACAGTC-3' (SEQ.ID.NO.:15)

The PCR primers also contained restriction sites for HindIII and BamHI which were used to clone the 400bp 5'UTR amplicon into the HindIII/BamHI sites of the pBI101 vector in front of the GUS reporter gene. A 736bp amplicon from the 3' untranslated region (3'UTR) of the DNA fragment described in Figure 3 was PCR amplified using the following primers (location shown in Fig 3):
5' primer (2): 5'-AGGGGTGATCGATTA-3' (SEQ.ID.No: 16)
3' primer (2): 5'-GATAGAACCCACACGAGC-3' (SEQ.ID.NO.: 17)

The PCR primers also contained restriction sites for SacI and EcoRI. The NOS terminator region of the pBI101 vector was cut out with SacI/EcoRI digestion and replaced with the similarly digested 736bp 3'UTR amplicon of the DNA fragment described in Figure 3.

The GUS reporter construct was then electroporated into *Agrobacterium tumifaciens* strain AGLI and transformation of flax (Finnegan et al. (1993) Plant Mol Biol. 22(4): 625-633) and *Arabidopsis* (Valvekens *et al.* Proc. Natl. Acad. Sci. 85; 5536-5540) carried out according to previously described protocols.

Various tissues from flax and *Arabidopsis* plants carrying the GUS reporter construct were assayed histologically for evidence of GUS activity. In the case of flax, leaf tissue, root tissue and mid-maturity embryos dissected out of developing seeds were stained for GUS activity. For *Arabidopsis,* developing seeds were stained for GUS in situ in their siliques.

GUS staining was carried out by immersing the tissues in histochemical buffer containing 0.5 mM X-gluc, 0.5 M potassium phosphate buffer (pH 7.0), 1 mM EDTA, 0.5 M sorbital, 0.5 mM potassium ferricyanide and 0.5 mM potassium ferrocyanide. The staining reaction was carried out for 12-16 hrs at 37°C and the reaction was stopped by adding 95% ethanol. Tissues were subsequently cleared of chlorophyll by repeated washing in 95% ethanol prior to photography. Figure 9 shows clear evidence of strong GUS activity in developing flax embryos and *Arabidopsis* seeds, and no evidence of GUS reporter gene expression in flax roots or leaves, or in *Arabidopsis* silique walls.

### EXAMPLE 6

### Stable seed specific expression of b-glucuronidase (GUS) in flax, Arabidopsis and Brassica napus when under the regulatory control of flax legumin-like storage protein gene regulatory sequences

A construct was made using standard molecular biology techniques, including restriction enzyme digestions, ligation and polymerase chain reaction (PCR). In order to obtain a DNA fragment containing approximately 2 kilobases from the 5' transcriptional initiation region of the flax legumin-like seed storage protein in a configuration suitable for ligation to a GUS coding sequence, a PCR based approach was used. This involved the use of the polymerase chain reaction to amplify the precise sequence desired for the expression analysis. To perform the necessary PCR amplification, two oligonucleotide primers were synthesized (Beckman Oligo 1000M DNA synthesizer) have the following sequences:
5' primer: 5'TATCTAGACTCAAGCATACGGACAAGGGT 3' (SJ-634) (SEQ.ID.NO.:18)

The italicized bases correspond to nucleotide positions 1 to 21 in the sequence reported in Figure 4. The additional nucleotides 5' of this sequence in the primer are not identical to the promoter sequence, but were included in order to place a XbaI site at the 5' end of the amplification product. The XbaI (5'-TCTAGA-3') (SEQ.ID.NO.:19) site is underlined.

A second (3') primer was synthesized which had the following sequence:
3' primer 5'GGTTATCATTGTATGAACTGA3' (SJ-618) (SEQ.ID.NO.:20)

This primer contains the precise complement (shown in italics) to the sequence reported in Figure 4 from bases 2343 to 2363. This primer was not designed with an additional restriction enzyme site due to the fact that a natural NcoI site (5'-CCATGG-3') (SEQ.ID.NO.:21) straddles the start codon between base pairs 2034 and 2039, thereby allowing for insertion of the storage protein promoter into the appropriate cloning vector.

These two primers were used in a PCR amplification reaction to produce a DNA fragment containing the sequence between nucleotides 1 and 2342 of the flax seed storage protein gene with a XbaI site at the 5' end and a NcoI site 302 base pairs from the 3' end. PCR amplification was performed using the enzyme Pfu (Strategene) using conditions recommended by the enzyme manufacturer and a temperature program of 94°C (denaturation) for 1 minute, 55°C (annealing) for 1 minute, and 72°C (elongation) for 3.5 minutes. The template was the legumin seed storage protein genomic clone shown in Figure 4.

The resulting amplification product was subsequently digested with XbaI and NcoI to remove the desired 2 kb promoter region. This promoter figment was cloned into the XbaI and NcoI sites of a XbaI and NcoI digested plasmid designated pGUS1318 (Plasmid pGUSN358S (Clontech Laboratories) was cut with NcoI and EcoRI and the GUS insert was cloned into pBluescriptKS+ (Stratagene) which was adapted to contain an NcoI site in the multiple cloning site.) The resulting plasmid containing the promoter-GUS fusion was called pPGUS1318. The terminator of the legumin seed storage protein from flax was also amplified from the above mentioned genomic clone. To perform the necessary PCR amplification, oligonucleotide primers were synthesized having the following sequences:
5' primer: 5' GCAAGCTTAATGTGACGGTGAAATAATAACGG 3' (8J620) (SEQ.ID.NO.:22)

The italicized bases correspond to nucleotide positions 3780 to 3803 in the sequence reported in Figure 4. The additional nucleotides 5' of this sequence in the primer are not identical to the promoter sequence, but were included in order to place a HindIII site at the 5' end of the amplification product. The HindIII site (5'-AAGCTT-3') (SEQ.ID.NO.:23) is underlined.

A second (3') primer was synthesized which had the following sequence:
3' primer 5'TAGGTACCTGGCAGGTAAAGACTCTGCTC3' (SJ-618) (SEQ.ID.NO.:24)

This primer contains the precise complement (shown in italics) to the sequence reported in Figure 4 from bases 4311 to 4290. The additional nucleotides 5' of this sequence in the primer are not identical to the promoter sequence, but were included in order to place a KpnI site at the 5' end of the amplification product. The KpnI site (5'-GGTACC-3') (SEQ.ID.NO.:25) is underlined.

These two primers were used in a PCR amplification reaction to produce a DNA fragment containing the sequence between nucleotides 3779 and 4311 of the flax seed storage protein gene terminator with a HindIII site at the 5' end and a KpnI site at 3' end. Amplification using PCT was as described above. The above pPGUS1318 vector that contains the amplified promoter was digested with XhoI and treated with Klenow to create a blunt end. The vector was subsequently digested with KpnI and the above amplified terminator sequence was inserted so that it was located 3' of the GUS coding sequence. The resulting vector containing the flax seed storage protein promoter, GUS and the flax seed storage protein terminator is referred to as pPGUST.

The XbaI-KpnI insert of pPGUST which contains the linin promoter-GUS coding sequence-linin terminator sequence was ligated into the XbaI-KpnI sites of pSBS3000 (This vector is a derivative from the *Agrobacterium* binary plasmid pPZP221 (Hajdukiewicz *et al.,* 1994, Plant Molec. Biol. 25: 989-994). In pSBS3000 the plant gentamycin resistance gene of pPZP221 was replaced with parsley ubiquitin promoter-phosphinothricin acetyl transferase gene-parsley ubiquitin termination sequence to confer resistance to the herbicide glufosinate ammonium). The resulting vector is called pSBS2089. In addition the XbaI-KpnI insert of pPGUST which contains the linin promoter-GUS coding sequence-linin terminator sequence was ligated into the XbaI-KpnI sites of the *Agrobacterium* binary plasmid pCGN1559 (MacBride and Summerfield, 1990, Plant Molec. Biol. 14 269-276, confers resistance to the antibiotic kanamycin)). The resulting vector was called pSBS2083. Plasmids pSBS2089 and pSBS2083 were electroporated into *Agrobacterium* strain EHA101. *Agrobacterium* strain EHA101 (pSBS2089) was used to transform flax and Arabidopsis, *Agrobacterium* strain EHA101 (pSBS2083) was used to transform *Brassica napus.* Flax transformation was performed essentially as described in Jordan and McHughen (1988) Plant cell reports 7: 281-284, except transgenic shoots were selected on 10 µM L-phosphinothricine instead of kanamycin. Arabidopsis transformation was done essentially as described in "Arabidopsis Protocols; Methods in Molecular Biology" Vol 82. Edited by Martinez-Zapater JM and Salinas J. ISBN 0-89603-391-0 pg 259-266 (1998) except the putative transgenic plants were selected on agarose plates containing 80µM L-phosphinothricine. *Brassica napus* transformation was done essentially as described in Moloney *et al.* (1989). Plant Cell Reports. 8: 238-242.

Figure 10 shows the tissue-specific expression of GUS in transgenic flax plants transformed with a linin-GUS gene construct (pSBS2089). GUS expression was measured in roots (R), stems (S), leaves (L), Buds (B) and embryo (E). Some expression was seen in buds, and maximal expression was achieved in embryo tissues. No detectable expression was seen in any of the untransformed (WT) tissues.

Figure 11 shows the temporal expression of GUS in transgenic flax plants transformed with a linin-GUS gene construct (pSBS2089). As can be seen, maximum expression is achieved in mature (pre-dessicated) flax embryos.

Figure 12 shows the absolute expression of GUS in transgenic *Brassica napus* plants (L1 to L9) transformed with a linin-GUS gene construct (pSBS2083). As can be seen high level expression can be achieved in Brassica napus plants. When comparing individual transgenic plants, a typical variation in expression due to position effect can also be seen.

Figure 13 shows expression of GUS in transgenic Arabidopsis siliques (transformed with a linin-GUS gene construct (pSBS2089)) during seed development. As can be seen high level expression can also be achieved in Arabidopsis seed tissues. Maximum expression is achieved at stage 4 (mature but not fully dessicated) of seed development. No detectable expression is observed in non-seed tissues such as leaves, stems, roots and silique walls (results not shown).

While the present invention has been described with reference to what are presently considered to be the preferred examples, it is to be understood that the invention is not limited to the disclosed examples. To the contraty, the invention is intended to cover various modifications and equivalent arrangements included within the scope of the appended claims.

## Claims

1. A method for the expression of a nucleic add sequence of interest in flax seeds comprising:
(a) preparing a chimeric nucleic acid construct comprising in the 5' to 3' direction of transcription as operably linked components:
(1) a seed-specific promoter obtained from flax; and
(2) said nucleic add science of interest wherein said nucleic acid of interest is non-native to said seed-specific promoter;
(b) introducing said chimeric nucleic add construct into a flax plant cell; and
(c) growing said flax plant cell into a mature flax plant capable of setting seed
wherein said nucleic add sequence of interest is expressed in the seed under the control of said seed-specific promoter, wherein seed-specific means that a gene expressed under the control of the promoter is predominantly expressed in plant seeds with less than 5% of the overall expression level in other plant tissues and wherein said flax seed-specific promoter is selected from the group of promoters consisting of oleosin promoters, 2S storage protein promoters and legumin-like seed-storage protein promoters.

2. The method according to claim 1 wherein at least one expression characteristic conferred by said promoter to its native nucleic acid sequence is conferred to said non-native nucleic acid sequence.

3. The method according to claim 2 wherein said expression characteristic is timing of expression, level of expression, response to a change in lighting conditions, response to a change in temperature, response to a change in concentration of a chemical agent.

4. The method according to claim 1 wherein said seed-specific flax promoter comprises: 1
(a) a nucleic acid sequence compring nucleotides 1 to 2023 as shown in Figure 1 (SEQID.NO.:1), wherein T can also be U;
(b) a nucleic add sequence that is complimentary to a nucleic acid sequence of
(c) a nucleic add sequence that has at least 65% identity to a nucleic acid sequence of (a) or (b);
(d) a nucleic acid sequence that is an analog of a nucleic acid sequence of (a), (b) or (c); or
(e) a nucleic acid sequence that hybridizes to a nudeic acid sequence of (a), (b), (c) or (d) under stringent hybridization conditions.

5. The method according to claim 1 wherein said seed-specific flax promoter comprises:
(a) a nucleic add sequence comprising nucleotides 21 to 1852 as shown in Figure 2 (SEQ.ID.NO.:4), wherein T can also be U;
(b) a nucleic acid sequence that is complimentary to a nucleic add sequence of (a);
(c) a nucleic add sequence that has at least 65% identity to a nucleic acid sequence of (a) or (b);
(d) a nucleic acid sequence that is an analog of a nucleic add sequence of (a), (b) or (c); or
(e) a nucleic acid sequence that hybridizes to a nucleic add sequence of (a), (b), (c) or (d) under stringent hybridization conditions.

6. The method according to claim 1 wherein said seed-specific flax promoter comprises:
(a) a nucleic acid sequence comprising nucleotides 1 to 400 as shown in Figure 3 (SEQ.ID.NO.:6), wherein T can also be U;
(b) a nucleic add sequence that is complimentary to a nucleic add sequence of (a);
(c) a nucleic acid sequence that has at least 65% identity to a nucleic acid sequence of (a) or (b);
(d) a nucleic acid sequence that is an analog of a nucleic acid sequence of (a), (b) or (c); or
(e) a nucleic acid sequence that hybridizes to a nucleic acid sequence of (a), (b), (c) or (d) under stringent hybridization conditions.

7. The method according to claim 1 wherein said seed-specific flax promoter comprises:
(a) a nucleic acid sequence comprising nucleotides 1 to 2034 as shown in Figure 4 (SEQ.ID.NO.:8), wherein T can also be U;
(b) a nucleic acid sequence that is complimentary to a nucleic acid sequence of (a);
(c) a nucleic acid sequence that has at least 65% identity to a nucleic acid sequence of (a) or (b);
(d) a nucleic acid sequence that is an analog of a nucleic acid sequence of (a), (b) or (c); or
(e) a nucleic acid sequence that hybridizes to a nucleic acid sequence of (a), (b), (c) or (d) under stringent hybridization conditions.

8. The method according to claim 1 wherein expression of said nucleic add sequence of interest results in an alteration in protein or fatty add composition in said seed,

9. Transgenic flax seed prepared according to a method comprising:
(a) preparing a chimeric nucleic acid construct comprising in the 5' to 3' direction of transcription as operably linked components:
(1) a seed-specific promoter obtained from flax; and
(2) a nucleic acid sequence of interest wherein said nucleic acid of interest is non-native to said seed-specific promoter;
(b) introducing said chimeric nucleic acid construct into a flax plant cell; and
(c) growing said flax plant cell into a mature flax plant capable of setting seed
wherein said nucleic acid sequence of interest is expressed in the seed under the control of said seed-specific promoter wherein seed-specific means that a gene expressed under the control of the promoter is predominantly expressed in plant seeds with less than 5% of the overall expression level in other plant tissues and wherein said promoter is a seed storage protein promoter, an oleosin promoter, a 2S storage protein promoter or a legumin-like seed-storage protein promoter.

10. Transgenic flax seed according to claim 9 wherein at least one expression characteristic conferred by said seed-specific promoter to its native nucleic acid sequence is conferred to said non-native nucleic acid sequence.

11. The transgenic flax seed according to claim 10 wherein said expression characteristic is timing of expression or level of expression.

12. Transgenic flax seed according to claim 10 wherein said seed-specific promoter comprises:
(a) a nucleic acid sequence comprising nucleotides 1 to 2023 as shown in Figure 1 (SEQ.ID.NO.:1), wherein T can also be U;
(b) a nucleic acid sequence that is complimentary to a nucleic acid sequence of (a);
(c) a nucleic acid sequence that has at least 65% identity to a nucleic acid sequence of (a) or (b);
(d) a nucleic acid sequence that is an analog of a nucleic acid sequence of (a), (b) or (c); or
(e) a nucleic acid sequence that hybridizes to a nucleic add sequence of (a), (b), (c) or (d) under stringent hybridization conditions.

13. Transgenic flax seed according to claim 10 wherein said seed-specific promoter comprises:
(a) a nucleic acid sequence comprising nucleotides 21 to 1852 as shown in Figure 2 (SEQ.ID.NO.:4), wherein T can also be U;
(b) a nucleic acid sequence that is complimentary to a nucleic acid sequence of (a);
(c) a nucleic acid sequence that has at least 65% identity to a nucleic acid sequence of (a) or (b);
(d) a nucleic acid sequence that is an analog of a nucleic acid sequence of (a), (b) or (c); or
(e) a nucleic acid sequence that hybridizes to a nucleic add sequence of (a), (b), (c) or (d) under stringent hybridization conditions.
(f)

14. Transgenic flax seed according to claim 10 wherein said seed-specific promoter comprises:
(a) a nucleic acid sequence comprising nucleotides 1 to 400 as shown in Figure 3 (SEQ.ID.NO.:6), wherein T can also be U;
(b) a nucleic acid sequence that is complimentary to a nucleic add sequence of (a);
(c) a nucleic acid sequence that has at least 65% identity to a nucleic acid sequence of (a) or (b);
(d) a nucleic acid sequence that is an analog of a nucleic acid sequence of (a), (b) or (c); or
(e) a nucleic acid sequence that hybridizes to a nucleic acid sequence of (a), (b), (c) or (d) under stringent hybridization conditions.

15. Transgenic flax seed according to claim 10 wherein said seed-specific promoter comprises:
(a) a nucleic acid sequence comprising nucleotides 1 to 2034 as shown in Figure 4 (SEQ.ID.NO.:8), wherein T can also be U;
(b) a nucleic acid sequence that is complimentary to a nucleic.acid sequence of (a);
(c) a nucleic add sequence that has at least 65% identity to a nucleic acid sequence of (a) or (b);
(d) a nucleic acid sequence that is an analog of a nucleic acid sequence of (a), (b) or (c); or
(e) a nucleic acid sequence that hybridizes to a nucleic acid sequence of (a), (b), (c) or (d) under stringent hybridization conditions.

16. Transgenic flax seed according to claim 10 wherein expression of said non-native gene of interest results in an alteration in the seed protein or fatty acid composition.

17. Transgenic flax plants capable of setting seed prepared by a method comprising:
(a) preparing a chimeric nucleic acid construct comprising in the 5' to 3' direction of transcription as operably linked components:
(1) a seed-specific promoter obtained from flax, and
(2) a nucleic acid sequence of interest wherein said nucleic add of interest is non-native to said seed-specific promoter;
(b) introducing said chimeric nucleic acid construct into a flax plant cell; and
(c) growing said flax plant cell into a mature flax plant capable of setting seed
wherein said nucleic acid sequence of interest is expressed in the seed under the control of said seed-specific promoter, wherein seed-specific means that a gene expressed under the control of the promoter is predominantly expressed in plant seeds with less than 5% of the overall expression level in other plant tissues and wherein said promoter is a seed storage protein promoter, an oleosin promoter, a 2S storage protein promoter or a legumin-like seed-storage protein promoter.

18. An isolated nucleic acid sequence capable of directing seed-specific expression in a plant comprising:
(a) a nucleic acid sequence comprising nucleotides 1 to 2023 as shown in Figure 1 (SEQ.ID.NO.:1), wherein T can also be U;
(b) a nucleic acid sequence that is complimentary to a nucleic acid sequence of (a);
(c) a nucleic add sequence that has at least 65% identity to a nudeic acid sequence of (a) or (b); or
(d) a nucleic add sequence that is an analog of a nucleic acid sequence of (a), (b) or (c); or
(e) a nucleic acid sequence that hybridizes to a nucleic acid sequence of (a), (b), (c) or (d) under stringent hybridization conditions,
wherein seed-specific expression means that a gene expressed under the control of said isolated nucleic acid is predominantly expressed in plant seeds with less than 5% of the overall expression level in other plant tissues.

19. An isolated nucleic acid sequence capable of directing seed-specific expression in a plant comprising:
(a) a nucleic acid sequence comprising nucleotides 21 to 1852 as shown in Figure 2 (SEQ.ID.NO.-4), wherein T can alsobe U;
(b) a nucleic acid sequence that is complimentary to a nucleic acid sequence of (a);
(c) a nucleic acid sequence mat has at least 65% identity to a nucleic acid sequence of (a) or (b); or
(d) a nucleic acid sequence that is an analog of a nucleic acid sequence of (a), (b) or (c); or
(e) a nucleic acid sequence that hybridizes to a nucleic acid sequence of (a), (b), (c) or (d) under stringent hybridization conditions;
wherein seed-specific expression means that a gene expressed under the control of said isolated nucleic acid is predominantly expressed in plant seeds with less than 5% of the overall expression level in other plant tissues.

20. An isolated nucleic acid sequence capable of directing seed-specific expression in a plant comprising:
(a) a nucleic add sequence comprising nucleotides 1 to 400 as shown in Figure 3 (SEQ.ID.NO.:6), wherein T can also be U;
(b) a nucleic acid sequence that is complimentary to a nucleic acid sequence of (a);
(c) a nucleic acid sequence that has at least 65% identity to a nucleic acid sequence of (a) or (b); or
(d) a nucleic acid sequence that is an analog of a nucleic acid sequence of (a), (b) or (c); or
(e) a nucleic acid sequence that hybridizes to a nucleic acid sequence of (a), (b), (c) or (d) under stringent hybridization conditions;
wherein seed-specific expression means that a gene expressed under the control of said isolated nucleic acid is predominantly expressed in plant seeds with less man 5% of the overall expression level in other plant tissues.

21. An isolated nucleic acid sequence capable of directing seed-spedfic expression in a plant comprising:
(a) a nucleic acid sequence comprising nucleotides 1 to 2034 as shown in Figure 4 (SEQ.ID.NO.:8), wherein T can also be U;
(b) a nucleic acid sequence that is complimentary to a nudeic acid sequence of (a);
(c) a nucleic acid sequence that has at least 65% identity to a nucleic acid sequence of (a) or (b); or
(d) a nucleic acid sequence that is an analog of a nucleic acid sequence of (a), (b) or (c); or
(e) a nucleic acid sequence that hybridizes to a nudeic acid sequence of (a), (b), (c) or (d) under stringent hybridization conditions;
wherein seed-specific expression means that a gene expressed under the control of said isolated nucleic acid is predominantly expressed in plant seeds with less than 5% of the overall expression level in other plant tissues.

22. An isolated chimeric nucleic acid sequence comprising:
(a) a first nucleic acid sequence comprising a seed-specific promoter obtained from flax which comprises:
(1) a nucleic acid sequence comprising nucleotides 1-2023 as shown in Figure 1 (SEQ.ID.NO.:1), wherein T can also be U;
(2) a nucleic acid sequence that hybridizes to a nucleic add sequence of (a) under stringent hybridization conditions;
(3) a nucleic acid sequence that is complimentary to a nucleic acid sequence of (a); or
(4) a nucleic acid sequence that has at least 65% identity to a nucleic acid sequence of (a); and
(b) a second nucleic acid sequence non-native to said flax seed-specific promoter;
wherein seed-specific means that a gene expressed under the control of the promoter is predominantly expressed in plant seeds with less than 5% of the overall expression level in other plant tissues.

23. An isolated chimeric nucleic acid sequence comprising:
(a) a first nucleic add sequence comprising a seed-specific promoter obtained from flax which comprises:
(1) a nucleic acid sequence comprisimg nucleotides 21-1852 as shown in Figure 2 (SEQ.ID.NO:4), wherein T can also be U;
(2) a nucleic acid sequence that hybridizes to a nucleic acid sequence of (a) under stringent hybridization conditions;
(3) a nucleic acid sequence that is complianentary to a nucleic add sequence of (a); or
(4) a nucleic acid sequence that has at least 65% identity to a nucleic acid sequence of (a); and
(b) a second nucleic acid sequence non-native to said flax seed-specific promoter
wherein seed-specific means that a gene expressed under the control of the promoter is predominantly expressed in plant seeds with less than 5% of the overall expression level in other plant tissues.

24. An isolated chimeric nucleic acid sequence comprising:
(a) a first nucleic acid sequence comprising a seed-specific promoter obtained from flax which comprises:
(1) a nucleic acid sequence comprising nucleotides 1-400 as shown in Figure 3 (SEQ.ID.NO.:6), wherein T can also be U;
(2) a nucleic acid sequence that hybridizes to a nucleic acid sequence of (a) under stringent hybridization conditions;
(3) a nucleic acid sequence that is complimentary to a nucleic add sequence of (a); or
(4) a nucleic add sequence that has at least 65% identity to a nucleic acid sequence of (a); and
(b) a second nucleic acid sequence non-native to said flax seed-specific promoter
wherein seed-specific means that a gene expressed under the control of the promoter is predominantly expressed in plant seeds with less than 5% of the overall expression level in other plant tissues.

25. An isolated chimeric nucleic acid sequence comprising:
(a) a first nucleic acid sequence comprising a seed-specific promoter obtained from flax which comprises;
(1) a nucleic acid sequence comprising nucleotides 1-2034 as shown in Figure 4 (SEQ.ID.NO.:8), wherein T can also be U;
(2) a nucleic acid sequence that hybridizes to a nucleic acid sequence of (a) under stringent hybridization conditions;
(3) a nucleic acid sequence that is complimentary to a nucleic acid sequence of (a); or
(4) a nucleic acid sequence that has at least 65% identity to a nucleic acid sequence of (a); and
(b) a second nucleic acid sequence non-native to said flax seed-specific promoter
wherein seed-specific means that a gene expressed under the control of the promoter is predominantly expressed in plant seeds with less than 5% of the overall expression level in other plant tissues.

26. A method for the expression of a nucleic acid sequence of interest in a plant seed comprising:
(a) introducing the chimeric nucleic acid sequence according to any one of claims 22 to 25 into a plant cell, and
(b) growing said plant cell into a mature plant capable of setting seed,
wherein the second nucleic acid sequence is expressed in the seed under the control of the seed specific promoter.

27. A method according to claim 26 wherein said plant cell is selected from the group of plants consisting of soybean (*Glycine max*), rapeseed (*Brassica* napus, *Brassica campestris*), sunflower (*Helianthus annuus*), cotton (*Gossypium hirsutum*), com (*Zea mays*), tobacco (*Nicotiana tobacum*), alfalafa (*Medicago sativa*), wheat (*Triticum* sp.), barley (*Hordeum vulgare*), oats (*Avena sativa L.*), sorghum (*Sorghum bicolor*), *Arabidopsis thaliana,* potato (*Solanum* sp.), flax/linseed (*Linum usitatissimum*), safflower (*Carthamus tinctorius*), oil palm (*Eleais guineeis*), groundnut (*Arachis hypogaea*), Brazil nut (*Bertholletia excelsa*) coconut (*Cocus nucifera*), castor (*Ricinus communis*), coriander (*Coriandrum sativum*), squash (*Cucurbita maxima*), jojoba (*Simmondsia chinensis*) and rice (*Oryza sativa*).

28. A plant prepared according to the method of claim 26.

29. A plant cell comprising the chimeric nucleic acid sequence according to any one of claims 22 to 25.

30. Plant seed comprisiag the chimeric nucleic acid sequence according to claim 22 to 25.

31. Plant seed obtained from a plant prepared according to the method of claim 26.

32. A recombinant expression vector comprising a nucleic acid sequence accenting to claim 18 to 21.

33. A recombinant expression vector comprisimg a nucleic acid sequence according to claim 22 to 25.

## Patentansprüche

1. Verfahren zur Expression einer interessierenden Nukleinsäuresequenz in Leinsamen, welches folgendes umfaßt:
(a) Herstellen eines chimären Nukleinsäurekonstrukts, welches in der 5'-nach-3'-Transkriptionsrichtung als funktionsfähig verknüpfte Komponenten die folgenden umfaßt:
(1) einen samenspezifischen, aus Lein erhaltenen Promotor und
(2) die interessierende Nukleinsäuresequenz, wobei die interessierende Nukleinsäuresequenz in dem samenspezifischen Promotor nicht nativ ist,
(b) Einbringen des chimären Nukleinsäurekonstrukts in eine Leinpflanzenzelle und
(c) Züchten der Leinpflanzenzelle zu einer reifen Leinpflanze, die in der Lage ist, Samen zu bilden, wobei die interessierende Nukleinsäuresequenz unter der Steuerung des samenspezifischen Promotors in dem Samen exprimiert wird, wobei samenspezifisch bedeutet, daß ein unter der Steuerung des Promotors exprimiertes Gen vorherrschend in Pflanzensamen mit weniger als 5% der Gesamtexpressionsmenge in anderen Pflanzengeweben exprimiert wird, und wobei der leinsamenspezifische Promotor aus der Gruppe von Promotoren ausgewählt ist, bestehend aus Oleosinpromotoren, 2S-Speicherproteinpromotoren und Legumin-artigen Samenspeicherproteinpromotoren.

2. Verfahren nach Anspruch 1, wobei wenigstens eine Expressionscharakteristik, die der Promotor seiner nativen Nukleinsäuresequenz verleiht, der nicht-nativen Nukleinsäuresequenz verliehen wird.

3. Verfahren nach Anspruch 2, wobei die Expressionscharakteristik die zeitliche Steuerung der Expression, die Expressionsmenge, die Reaktion auf eine Veränderung der Lichtbedingungen, die Reaktion auf eine Veränderung der Temperatur und die Reaktion auf eine Veränderung der Konzentration eines chemischen Mittels ist.

4. Verfahren nach Anspruch 1, wobei der leinsamenspezifische Promotor folgendes umfaßt:
(a) eine Nukleinsäuresequenz, die die Nukleotide 1 bis 2023 umfaßt, wie sie in Figur 1 gezeigt sind (SEQ ID NO:1), wobei T auch U sein kann,
(b) eine Nukleinsäuresequenz, die zu einer Nukleinsäuresequenz von (a) komplementär ist,
(c) eine Nukleinsäuresequenz, die wenigstens 65% Identität zu einer Nukleinsäuresequenz von (a) oder (b) besitzt,
(d) eine Nukleinsäuresequenz, die ein Analoges einer Nukleinsäuresequenz von (a), (b) oder (c) ist, oder
(e) eine Nukleinsäuresequenz, die unter stringenten Hybridisierungsbedingungen an eine Nukleinsäuresequenz von (a), (b), (c) oder (d) hybridisiert.

5. Verfahren nach Anspruch 1, wobei der leinsamenspezifische Promotor folgendes umfaßt:
(a) eine Nukleinsäuresequenz, die die Nukleotide 21 bis 1852 umfaßt, wie sie in Figur 2 gezeigt sind (SEQ ID NO:4), wobei T auch U sein kann,
(b) eine Nukleinsäuresequenz, die zu einer Nukleinsäuresequenz von (a) komplementär ist,
(c) eine Nukleinsäuresequenz, die wenigstens 65% Identität zu einer Nukleinsäuresequenz von (a) oder (b) besitzt,
(d) eine Nukleinsäuresequenz, die ein Analoges einer Nukleinsäuresequenz von (a), (b) oder (c) ist, oder
(e) eine Nukleinsäuresequenz, die unter stringenten Hybridisierungsbedingungen an eine Nukleinsäuresequenz von (a), (b), (c) oder (d) hybridisiert.

6. Verfahren nach Anspruch 1, wobei der leinsamenspezifische Promotor folgendes umfaßt:
(a) eine Nukleinsäuresequenz, die die Nukleotide 1 bis 400 umfaßt, wie sie in Figur 3 gezeigt sind (SEQ ID NO:6), wobei T auch U sein kann,
(b) eine Nukleinsäuresequenz, die zu einer Nukleinsäuresequenz von (a) komplementär ist,
(c) eine Nukleinsäuresequenz, die wenigstens 65% Identität zu einer Nukleinsäuresequenz von (a) oder (b) besitzt,
(d) eine Nukleinsäuresequenz, die ein Analoges einer Nukleinsäuresequenz von (a), (b) oder (c) ist, oder
(e) eine Nukleinsäuresequenz, die unter stringenten Hybridisierungsbedingungen an eine Nukleinsäuresequenz von (a), (b), (c) oder (d) hybridisiert.

7. Verfahren nach Anspruch 1, wobei der leinsamenspezifische Promotor folgendes umfaßt:
(a) eine Nukleinsäuresequenz, die die Nukleotide 1 bis 2034 umfaßt, wie sie in Figur 4 gezeigt sind (SEQ ID NO:8), wobei T auch U sein kann,
(b) eine Nukleinsäuresequenz, die zu einer Nukleinsäuresequenz von (a) komplementär ist,
(c) eine Nukleinsäuresequenz, die wenigstens 65% Identität zu einer Nukleinsäuresequenz von (a) oder (b) besitzt,
(d) eine Nukleinsäuresequenz, die ein Analoges einer Nukleinsäuresequenz von (a), (b) oder (c) ist, oder
(e) eine Nukleinsäuresequenz, die unter stringenten Hybridisierungsbedingungen an eine Nukleinsäuresequenz von (a), (b), (c) oder (d) hybridisiert.

8. Verfahren nach Anspruch 1, wobei die Expression der interessierenden Nukleinsäuresequenz zu einer Veränderung der Protein- oder Fettsäurezusammensetzung in dem Samen führt.

9. Transgener Leinsamen, hergestellt nach einem Verfahren, welches folgendes umfaßt:
(a) Herstellen eines chimären Nukleinsäurekonstrukts, welches in der 5'-nach-3'-Transkriptionsrichtung als funktionsfähig verknüpfte Komponenten die folgenden umfaßt:
(1) einen samenspezifischen, aus Lein erhaltenen Promotor und
(2) die interessierende Nukleinsäuresequenz, wobei die interessierende Nukleinsäuresequenz in dem samenspezifischen Promotor nicht nativ ist,
(b) Einbringen des chimären Nukleinsäurekonstrukts in eine Leinpflanzenzelle und
(c) Züchten der Leinpflanzenzelle zu einer reifen Leinpflanze, die in der Lage ist, Samen zu bilden, wobei die interessierende Nukleinsäuresequenz unter der Steuerung des samenspezifischen Promotors in dem Samen exprimiert wird, wobei samenspezifisch bedeutet, daß ein unter der Steuerung des Promotors exprimiertes Gen vorherrschend in Pflanzensamen mit weniger als 5% der Gesamtexpressionsmenge in anderen Pflanzengeweben exprimiert wird, und wobei der Promotor ein Samenspeicherproteinpromotor, ein Oleosinpromotor, ein 2S-Speicherproteinpromotor oder ein Legumin-artiger Samenspeicherproteinpromotor ist.

10. Transgener Leinsamen nach Anspruch 9, wobei wenigstens eine Expressionscharakteristik, die der samenspezifische Promotor seiner nativen Nukleinsäuresequenz verleiht, der nicht-nativen Nukleinsäuresequenz verliehen wird.

11. Transgener Leinsamen nach Anspruch 10, wobei die Expressionscharakteristik die zeitliche Steuerung der Expression oder die Expressionsmenge ist.

12. Transgener Leinsamen nach Anspruch 10, wobei der samenspezifische Promotor folgendes umfaßt:
(a) eine Nukleinsäuresequenz, die die Nukleotide 1 bis 2023 umfaßt, wie sie in Figur 1 gezeigt sind (SEQ ID NO:1), wobei T auch U sein kann,
(b) eine Nukleinsäuresequenz, die zu einer Nukleinsäuresequenz von (a) komplementär ist,
(c) eine Nukleinsäuresequenz, die wenigstens 65% Identität zu einer Nukleinsäuresequenz von (a) oder (b) besitzt,
(d) eine Nukleinsäuresequenz, die ein Analoges einer Nukleinsäuresequenz von (a), (b) oder (c) ist, oder
(e) eine Nukleinsäuresequenz, die unter stringenten Hybridisierungsbedingungen an eine Nukleinsäuresequenz von (a), (b), (c) oder (d) hybridisiert.

13. Transgener Leinsamen nach Anspruch 10, wobei der samenspezifische Promotor folgendes umfaßt:
(a) eine Nukleinsäuresequenz, die die Nukleotide 21 bis 1852 umfaßt, wie sie in Figur 2 gezeigt sind (SEQ ID NO:4), wobei T auch U sein kann,
(b) eine Nukleinsäuresequenz, die zu einer Nukleinsäuresequenz von (a) komplementär ist,
(c) eine Nukleinsäuresequenz, die wenigstens 65% Identität zu einer Nukleinsäuresequenz von (a) oder (b) besitzt,
(d) eine Nukleinsäuresequenz, die ein Analoges einer Nukleinsäuresequenz von (a), (b) oder (c) ist, oder
(e) eine Nukleinsäuresequenz, die unter stringenten Hybridisierungsbedingungen an eine Nukleinsäuresequenz von (a), (b), (c) oder (d) hybridisiert.

14. Transgener Leinsamen nach Anspruch 10, wobei der samenspezifische Promotor folgendes umfaßt:
(a) eine Nukleinsäuresequenz, die die Nukleotide 1 bis 400 umfaßt, wie sie in Figur 3 gezeigt sind (SEQ ID NO:6), wobei T auch U sein kann,
(b) eine Nukleinsäuresequenz, die zu einer Nukleinsäuresequenz von (a) komplementär ist,
(c) eine Nukleinsäuresequenz, die wenigstens 65% Identität zu einer Nukleinsäuresequenz von (a) oder (b) besitzt,
(d) eine Nukleinsäuresequenz, die ein Analoges einer Nukleinsäuresequenz von (a), (b) oder (c) ist, oder
(e) eine Nukleinsäuresequenz, die unter stringenten Hybridisierungsbedingungen an eine Nukleinsäuresequenz von (a), (b), (c) oder (d) hybridisiert.

15. Transgener Leinsamen nach Anspruch 10, wobei der samenspezifische Promotor folgendes umfaßt:
(a) eine Nukleinsäuresequenz, die die Nukleotide 1 bis 2034 umfaßt, wie sie in Figur 4 gezeigt sind (SEQ ID NO:8), wobei T auch U sein kann,
(b) eine Nukleinsäuresequenz, die zu einer Nukleinsäuresequenz von (a) komplementär ist,
(c) eine Nukleinsäuresequenz, die wenigstens 65% Identität zu einer Nukleinsäuresequenz von (a) oder (b) besitzt,
(d) eine Nukleinsäuresequenz, die ein Analoges einer Nukleinsäuresequenz von (a), (b) oder (c) ist, oder
(e) eine Nukleinsäuresequenz, die unter stringenten Hybridisierungsbedingungen an eine Nukleinsäuresequenz von (a), (b), (c) oder (d) hybridisiert.

16. Transgener Leinsamen nach Anspruch 10, wobei die Expression des interessierenden, nicht-nativen Gens zu einer Veränderung der Protein- oder Fettsäurezusammensetzung in dem Samen führt.

17. Transgene Leinpflanzen, die in der Lage sind, Samen zu bilden, hergestellt durch ein Verfahren, welches folgendes umfaßt:
(a) Herstellen eines chimären Nukleinsäurekonstrukts, welches in der 5'-nach-3'-Transkriptionsrichtung als funktionsfähig verknüpfte Komponenten die folgenden umfaßt:
(1) einen samenspezifischen, aus Lein erhaltenen Promotor und
(2) die interessierende Nukleinsäuresequenz, wobei die interessierende Nukleinsäuresequenz in dem samenspezifischen Promotor nicht nativ ist,
(b) Einbringen des chimären Nukleinsäurekonstrukts in eine Leinpflanzenzelle und
(c) Züchten der Leinpflanzenzelle zu einer reifen Leinpflanze, die in der Lage ist, Samen zu bilden, wobei die interessierende Nukleinsäuresequenz unter der Steuerung des samenspezifischen Promotors in dem Samen exprimiert wird, wobei samenspezifisch bedeutet, daß ein unter der Steuerung des Promotors exprimiertes Gen vorherrschend in Pflanzensamen mit weniger als 5% der Gesamtexpressionsmenge in anderen Pflanzengeweben exprimiert wird, und wobei der Promotor ein Samenspeicherproteinpromotor, ein Oleosinpromotor, ein 2S-Speicherproteinpromotor oder ein Legumin-artiger Samenspeicherproteinpromotor ist.

18. Isolierte Nukleinsäuresequenz, die in der Lage ist, die samenspezifische Expression in einer Pflanze zu steuern, und die folgendes umfaßt:
(a) eine Nukleinsäuresequenz, die die Nukleotide 1 bis 2023 umfaßt, wie sie in Figur 1 gezeigt sind (SEQ ID NO:1), wobei T auch U sein kann,
(b) eine Nukleinsäuresequenz, die zu einer Nukleinsäuresequenz von (a) komplementär ist,
(c) eine Nukleinsäuresequenz, die wenigstens 65% Identität zu einer Nukleinsäuresequenz von (a) oder (b) besitzt,
(d) eine Nukleinsäuresequenz, die ein Analoges einer Nukleinsäuresequenz von (a), (b) oder (c) ist, oder
(e) eine Nukleinsäuresequenz, die unter stringenten Hybridisierungsbedingungen an eine Nukleinsäuresequenz von (a), (b), (c) oder (d) hybridisiert,
wobei samenspezifische Expression bedeutet, daß ein unter der Steuerung der isolierten Nukleinsäure exprimiertes Gen vorherrschend in Pflanzensamen mit weniger als 5% der Gesamtexpressionsmenge in anderen Pflanzengeweben exprimiert wird.

19. Isolierte Nukleinsäuresequenz, die in der Lage ist, die samenspezifische Expression in einer Pflanze zu steuern, und die folgendes umfaßt:
(a) eine Nukleinsäuresequenz, die die Nukleotide 21 bis 1852 umfaßt, wie sie in Figur 2 gezeigt sind (SEQ ID NO:4), wobei T auch U sein kann,
(b) eine Nukleinsäuresequenz, die zu einer Nukleinsäuresequenz von (a) komplementär ist,
(c) eine Nukleinsäuresequenz, die wenigstens 65% Identität zu einer Nukleinsäuresequenz von (a) oder (b) besitzt,
(d) eine Nukleinsäuresequenz, die ein Analoges einer Nukleinsäuresequenz von (a), (b) oder (c) ist, oder
(e) eine Nukleinsäuresequenz, die unter stringenten Hybridisierungsbedingungen an eine Nukleinsäuresequenz von (a), (b), (c) oder (d) hybridisiert,
wobei samenspezifische Expression bedeutet, daß ein unter der Steuerung der isolierten Nukleinsäure exprimiertes Gen vorherrschend in Pflanzensamen mit weniger als 5% der Gesamtexpressionsmenge in anderen Pflanzengeweben exprimiert wird.

20. Isolierte Nukleinsäuresequenz, die in der Lage ist, die samenspezifische Expression in einer Pflanze zu steuern, und die folgendes umfaßt:
(a) eine Nukleinsäuresequenz, die die Nukleotide 1 bis 400 umfaßt, wie sie in Figur 3 gezeigt sind (SEQ ID NO:6), wobei T auch U sein kann,
(b) eine Nukleinsäuresequenz, die zu einer Nukleinsäuresequenz von (a) komplementär ist,
(c) eine Nukleinsäuresequenz, die wenigstens 65% Identität zu einer Nukleinsäuresequenz von (a) oder (b) besitzt,
(d) eine Nukleinsäuresequenz, die ein Analoges einer Nukleinsäuresequenz von (a), (b) oder (c) ist, oder
(e) eine Nukleinsäuresequenz, die unter stringenten Hybridisierungsbedingungen an eine Nukleinsäuresequenz von (a), (b), (c) oder (d) hybridisiert,
wobei samenspezifische Expression bedeutet, daß ein unter der Steuerung der isolierten Nukleinsäure exprimiertes Gen vorherrschend in Pflanzensamen mit weniger als 5% der Gesamtexpressionsmenge in anderen Pflanzengeweben exprimiert wird.

21. Isolierte Nukleinsäuresequenz, die in der Lage ist, die samenspezifische Expression in einer Pflanze zu steuern, und die folgendes umfaßt:
(a) eine Nukleinsäuresequenz, die die Nukleotide 1 bis 2034 umfaßt, wie sie in Figur 4 gezeigt sind (SEQ ID NO:8), wobei T auch U sein kann,
(b) eine Nukleinsäuresequenz, die zu einer Nukleinsäuresequenz von (a) komplementär ist,
(c) eine Nukleinsäuresequenz, die wenigstens 65% Identität zu einer Nukleinsäuresequenz von (a) oder (b) besitzt,
(d) eine Nukleinsäuresequenz, die ein Analoges einer Nukleinsäuresequenz von (a), (b) oder (c) ist, oder
(e) eine Nukleinsäuresequenz, die unter stringenten Hybridisierungsbedingungen an eine Nukleinsäuresequenz von (a), (b), (c) oder (d) hybridisiert,
wobei samenspezifische Expression bedeutet, daß ein unter der Steuerung der isolierten Nukleinsäure exprimiertes Gen vorherrschend in Pflanzensamen mit weniger als 5% der Gesamtexpressionsmenge in anderen Pflanzengeweben exprimiert wird.

22. Isolierte chimäre Nukleinsäuresequenz, welche folgendes umfaßt:
(a) eine erste Nukleinsäuresequenz, die einen samenspezifischen, aus Lein erhaltenen Promotor umfaßt, welcher folgendes umfaßt:
(1) eine Nukleinsäuresequenz, die die Nukleotide 1 bis 2023 umfaßt, wie sie in Figur 1 gezeigt sind (SEQ ID N0:1), wobei T auch U sein kann,
(2) eine Nukleinsäuresequenz, die unter stringenten Hybridisierungsbedingungen an eine Nukleinsäuresequenz von (a) hybridisiert,
(3) eine Nukleinsäuresequenz, die zu einer Nukleinsäuresequenz von (a) komplementär ist, oder
(4) eine Nukleinsäuresequenz, die wenigstens 65% Identität zu einer Nukleinsäuresequenz von (a) besitzt, und
(b) eine zweite Nukleinsäuresequenz, die in dem leinsamenspezifischen Promotor nicht nativ ist, wobei samenspezifisch bedeutet, daß ein unter der Steuerung des Promotors exprimiertes Gen vorherrschend in Pflanzensamen mit weniger als 5% der Gesamtexpressionsmenge in anderen Pflanzengeweben exprimiert wird.

23. Isolierte chimäre Nukleinsäuresequenz, welche folgendes umfaßt:
(a) eine erste Nukleinsäuresequenz, die einen samenspezifischen, aus Lein erhaltenen Promotor umfaßt, welcher folgendes umfaßt:
(1) eine Nukleinsäuresequenz, die die Nukleotide 21 bis 1852 umfaßt, wie sie in Figur 2 gezeigt sind (SEQ ID NO:4), wobei T auch U sein kann,
(2) eine Nukleinsäuresequenz, die unter stringenten Hybridisierungsbedingungen an eine Nukleinsäuresequenz von (a) hybridisiert,
(3) eine Nukleinsäuresequenz, die zu einer Nukleinsäuresequenz von (a) komplementär ist, oder
(4) eine Nukleinsäuresequenz, die wenigstens 65% Identität zu einer Nukleinsäuresequenz von (a) besitzt, und
(b) eine zweite Nukleinsäuresequenz, die in dem leinsamenspezifischen Promotor nicht nativ ist, wobei samenspezifisch bedeutet, daß ein unter der Steuerung des Promotors exprimiertes Gen vorherrschend in Pflanzensamen mit weniger als 5% der Gesamtexpressionsmenge in anderen Pflanzengeweben exprimiert wird.

24. Isolierte chimäre Nukleinsäuresequenz, welche folgendes umfaßt:
(a) eine erste Nukleinsäuresequenz, die einen samenspezifischen, aus Lein erhaltenen Promotor umfaßt, welcher folgendes umfaßt:
(1) eine Nukleinsäuresequenz, die die Nukleotide 1 bis 400 umfaßt, wie sie in Figur 3 (SEQ ID NO:6) gezeigt sind, wobei T auch U sein kann,
(2) eine Nukleinsäuresequenz, die unter stringenten Hybridisierungsbedingungen an eine Nukleinsäuresequenz von (a) hybridisiert,
(3) eine Nukleinsäuresequenz, die zu einer Nukleinsäuresequenz von (a) komplementär ist, oder
(4) eine Nukleinsäuresequenz, die wenigstens 65% Identität zu einer Nukleinsäuresequenz von (a) besitzt, und
(b) eine zweite Nukleinsäuresequenz, die in dem leinsamenspezifischen Promotor nicht nativ ist, wobei samenspezifisch bedeutet, daß ein unter der Steuerung des Promotors exprimiertes Gen vorherrschend in Pflanzensamen mit weniger als 5% der Gesamtexpressionsmenge in anderen Pflanzengeweben exprimiert wird.

25. Isolierte chimäre Nukleinsäuresequenz, welche folgendes umfaßt:
(a) eine erste Nukleinsäuresequenz, die einen samenspezifischen, aus Lein erhaltenen Promotor umfaßt, welcher folgendes umfaßt:
(1) eine Nukleinsäuresequenz, die die Nukleotide 1 bis 2034 umfaßt, wie sie in Figur 4 gezeigt sind (SEQ ID NO:8), wobei T auch U sein kann,
(2) eine Nukieinsäuresequenz, die unter stringenten Hybridisierungsbedingungen an eine Nukleinsäuresequenz von (a) hybridisiert,
(3) eine Nukleinsäuresequenz, die zu einer Nukleinsäuresequenz von (a) komplementär ist, oder
(4) eine Nukleinsäuresequenz, die wenigstens 65% Identität zu einer Nukleinsäuresequenz von (a) besitzt, und
(b) eine zweite Nukleinsäuresequenz, die in dem leinsamenspezifischen Promotor nicht nativ ist, wobei samenspezifisch bedeutet, daß ein unter der Steuerung des Promotors exprimiertes Gen vorherrschend in Pflanzensamen mit weniger als 5% der Gesamtexpressionsmenge in anderen Pflanzengeweben exprimiert wird.

26. Verfahren zur Expression einer interessierenden Nukleinsäuresequenz in einem Pflanzensamen, welches folgendes umfaßt:
(a) Einbringen der chimären Nukleinsäuresequenz gemäß einem der Ansprüche 22 bis 25 in eine Pflanzenzelle und
(b) Züchten der Pflanzenzelle zu einer reifen Pflanze, die in der Lage ist, Samen zu bilden,
wobei die zweite Nukleinsäuresequenz unter der Steuerung des samenspezifischen Promotors in dem Samen exprimiert wird.

27. Verfahren nach Anspruch 26, wobei die Pflanzenzelle aus der Gruppe von Pflanzen ausgewählt ist, bestehend aus Sojabohne (*Glycine max*), Raps (*Brassica napus, Brassica campestris*), Sonnenblume (*Helianthus annuus*), Baumwolle (*Gossypium hirsurum*), Mais (Zea *mays*), Tabak (*Nicotiana tobacum*), Alfalfa (*Medicago sativa*), Weizen (*Triticum sp.*), Gerste (*Hordeum vulgare*), Hafer (*Avena sativa L.*), Hirse (*Sorghum bicolor*), *Arabidopsis thaliana,* Kartoffel (*Solanum* sp.), Flachs/Leinsamen (*Linum usitatissimum*)*,* Distel (*Carthamus tinctorius*), Ölpalme (*Eleais guineeis*), Erdnuß (*Arachis hypogaea*)*,* Paranuß (*Bertholletia excelsa*)*,* Kokosnuß (*Cocus nucifera*)*,* Rizinus (*Ricinus communis*)*,* Koriander (*Coriandrum sativum*)*,* Kürbis (*Cucurbita maxima*)*,* Jojoba (*Simmondsia chinensis*) und Reis (*Oryza sativa*)*.*

28. Pflanze, hergestellt nach dem Verfahren von Anspruch 26.

29. Pflanzenzelle, welche die chimäre Nukleinsäuresequenz nach einem der Ansprüche 22 bis 25 umfaßt.

30. Pflanzensamen, welcher die chimäre Nukleinsäuresequenz nach einem der Ansprüche 22 bis 25 umfaßt.

31. Pflanzensamen, erhalten von einer Pflanze, hergestellt nach dem Verfahren von Anspruch 26.

32. Rekombinanter Expressionsvektor, welcher eine Nukleinsäuresequenz nach einem der Ansprüche 18 bis 21 umfaßt.

33. Rekombinanter Expressionsvektor, welcher eine Nukleinsäuresequenz nach einem der Ansprüche 22 bis 25 umfaßt.

## Revendications

1. Procédé pour l'expression d'une séquence d'acide nucléique d'intérêt dans les graines de lin comprenant :
(a) la préparation d'une construction d'acide nucléique chimère comprenant comme composants liés fonctionnellement dans le sens de transcription 5' à 3' :
(1) un promoteur spécifique de graine obtenu du lin ; et
(2) ladite séquence nucléique d'intérêt pour laquelle ledit acide nucléique d'intérêt est non natif dudit promoteur spécifique de graine ;
(b) l'introduction de ladite construction d'acide nucléique chimère dans une cellule végétale de lin ; et
(c) la culture de ladite cellule végétale de lin dans une plante mature de lin capable de former des graines dans laquelle la séquence nucléique d'intérêt est exprimée dans la graine sous le contrôle dudit promoteur spécifique de graine, dans lequel spécifique à la graine signifie qu'un gène exprimé sous le contrôle du promoteur est exprimé de façon prédominante dans des graines de plante avec moins de 5% du niveau d'expression global dans d'autres tissus de la plante et dans lequel ledit promoteur spécifique de graine de lin est choisi parmi le groupe de promoteurs consistant en les promoteurs oléosine, les promoteurs de protéine de stockage 2S et les promoteurs de protéine de stockage de graine du genre légumineuse.

2. Procédé selon la revendication 1 dans lequel au moins une caractéristique de l'expression conférée par ledit promoteur à sa séquence d'acide nucléique native est conférée à ladite séquence d'acide nucléique non native.

3. Procédé selon la revendication 2 dans lequel ladite caractéristique de l'expression est le déroulement de l'expression, le niveau d'expression, la réponse à un changement de condition de lumière, la réponse à un changement de température, la réponse à un changement de concentration d'un agent chimique.

4. Procédé selon la revendication 1, dans lequel ledit promoteur spécifique de graine de lin comprend :
(a) une séquence d'acide nucléique comprenant les nucléotides 1 à 2023 comme montré dans la Figure 1 (SEQ.ID NO1) dans laquelle T peut égalemertt être U ;
(b) une séquence d'acide nucléique qui est complémentaire d'une séquence d'acide nucléique de (a) ;
(c) une séquence d'acide nucléique qui possède au moins 65% d'identité avec une séquence de (a) ou (b) ;
(d) une séquence d'acide nucléique qui est un analogue d'une séquence d'acide nucléique de (a), (b) ou (c); ou
(e) une séquence d'acide nucléique qui s'hybride avec une séquence d'acide nucléique de (a),(b), (c) ou (d) dans des conditions d'hybridation drastiques.

5. Procédé selon la revendication 1, dans lequel ledit promoteur spécifique de graine de lin comprend :
(a) une séquence d'acide nucléique comprenant les nucléotides 21 à 1852 comme montré dans la Figure 2 (SEQ.ID N° 4) dans laquelle T peut également être U ;
(b) une séquence d'acide nucléique qui est complémentaire d'une séquence d'acide nucléique de (a) ;
(c) une séquence d'acide nucléique qui possède au moins 65% d'identité avec une séquence d'acide nucléique de (a) ou (b) ;
(d) une séquence d'acide nucléique qui est un analogue d'une séquence d'acide nucléique de (a), (b) ou (c); ou
(e) une séquence d'acide nucléique qui s'hybride avec une séquence d'acide nucléique de (a),(b) (c) ou (d) dans des conditions d'hybridation drastiques ;

6. procédé selon la revendication 1, dans lequel ledit promoteur spécifique de graine de lin comprend :
(a) une séquence d'acide nucléique comprenant les nucléotides 1 à 400 comme montré dans la Figure 3 (SEQ.ID N° 6) dans laquelle T peut également être U ;
(b) une séquence d'acide nucléique qui est complémentaire d'une séquence d'acide nucléique de (a) ;
(c) une séquence d'acide nucléique qui possède au moins 65% d'identité avec une séquence d'acide nucléique de (a) ou (b) ;
(d) une séquence d'acide nucléique qui est un analogue d'une séquence d'acide nucléique de (a),(b) ou (c); ou
(e) une séquence d'acide nucléique qui s'hybride avec une séquence d'acide nucléique de (a),(b),(c) ou (d) dans des conditions d'hybridation drastiques.

7. Procédé selon la revendication 1, dans lequel ledit promoteur spécifique de graine de lin comprend :
(a) une séquence d'acide nucléique comprenant les nucléotides 1 à 2034 comme montré dans la figure 4 (SEQ.ID N° 8) dans laquelle T peut également être U;
(b) une séquence d'acide nucléique qui est complémentaire d'une séquence d'acide nucléique de (a) ;
(c) une séquence d'acide nucléique qui possède au moins 65% d'identité avec une séquence d'acide nucléique de (a) ou (b) ;
(d) une séquence, d'acide nucléique qui est un analogue d'une séquence d'acide nucléique de (a),(b) ou (c); ou
(e) une séquence d'acide nucléique qui s'hybride avec une séquence d'acide nucléique de (a),(b) (c) ou (d) dans des conditions d'hybridation drastiques.

8. Procédé selon la revendication 1, dans lequel l'expression de ladite séquence d'acide nucléique d'intérêt résulte en une altération de la composition protéique ou d'acide gras de ladite graine.

9. Graine transgénique de lin préparée selon un procédé comprenant :
(a) la préparation d'une construction d'acide nucléique chimère comprenant dans le sens de transcription 5' à 3' comme composants liés fonctionnellement:
(1) un promoteur spécifique provenant de graine obtenu du lin ; et
(2) une séquence nucléique d'intérêt pour laquelle ledit acide nucléique d'intérêt est non natif dudit promoteur spécifique de graine ;
(b) l'introduction de ladite construction d'acide nucléique chimère dans une cellule végétale de lin ; et
(c) la culture de ladite cellule végétale de lin dans une plante de lin mature capable de former des graines, dans laquelle la séquence nucléique d'intérêt est exprimée dans la graine sous le contrôle dudit promoteur spécifique de graine, dans laquelle spécifique à la graine signifie qu'un gène exprimé sous le contrôle du promoteur est exprimé de façon prédominante dans des graines de plante avec moins de 5% du niveau d'expression global dans d'autres tissus de la plante et dans laquelle ledit promoteur est un promoteur de protéine de stockage de graine, un promoteur oléosine, un promoteur de protéine de stockage 2S ou un promoteur de protéine de stockage de graine de genre légumineuse.

10. Graine transgénique de lin selon la revendication 9, dans laquelle au moins une caractéristique de l'expression conférée par ledit promoteur spécifique de la graine à sa séquence nucléique native est conférée à ladite séquence d'acide nucléique non native.

11. Graine transgénique de lin selon la revendication 10, dans laquelle ladite caractéristique de l'expression est le déroulement de l'expression ou le niveau d'expression.

12. Graine transgénique de lin selon la revendication 10, dans laquelle ledit promoteur spécifique de la graine comprend:
(a) une séquence d'acide nucléique comprenant les nucléotides 1 à 2023 comme montré dans la Figure 1 (SEQ.ID N° 1) dans laquelle T peut également être U ;
(b) une séquence d'acide nucléique qui est complémentaire d'une séquence d'acide nucléique de (a) ;
(c) une séquence d'acide nucléique qui possède au moins 65% d'identité avec une séquence d'acide nucléique de (a) ou (b) ;
(d) une séquence d'acide nucléique qui est un analogue d'une séquence d'acide nucléique de (a), (b) ou (c); ou
(e) une séquence d'acide nucléique qui s'hybride avec une séquence d'acide nucléique de (a), (b), (c) ou (d) dans des conditions d'hybridation drastiques.

13. Graine transgénique de lin selon la revendication 10 dans laquelle ledit promoteur spécifique de la graine comprend :
(a) une séquence d'acide nucléique comprenant les nucléotides 21 à 1852 comme montré dans la Figure 2 (SEQ.ID N°4) dans laquelle T peut également être U ;
(b) une séquence d'acide nucléique qui est complémentaire d'une séquence d'acide nucléique de (a) ;
(c) une séquence d'acide nucléique qui possède au moins 65% d'identité avec une séquence d'acide nucléique de (a) ou (b) ;
(d) une séquence d'acide nucléique qui est un analogue d'une séquence d'acide nucléique de (a),(b) ou (c); ou
(e) une séquence d'acide nucléique qui s'hybride avec une séquence d'acide nucléique de (a),(b),(c) ou (d) dans des conditions d'hybridation drastiques.

14. Graine transgénique de lin selon la revendication 10, dans laquelle ledit promoteur spécifique de la graine comprend
(a) une séquence d'acide nucléique comprenant les nucléotides 1 à 400 comme montré dans la Figure 3 (SEQ.ID N°6) dans laquelle T peut également être U ;
(b) une séquence d'acide nucléique qui est complémentaire d'une séquence d'acide nucléique de (a) ;
(c) une séquence d'acide nucléique qui possède au moins 65% d'identité avec une séquence d'acide nucléique de (a) ou (b) ;
(d) une séquence d'acide nucléique qui est un analogue d'une séquence d'acide nucléique de (a), (b) ou (c); ou
(e) une séquence d'acide nucléique qui s'hybride avec une séquence d'acide nucléique de (a), (b) (c) ou (d) dans des conditions d'hybridation drastiques.

15. Graine transgénique de lin selon la revendication 10, dans laquelle ledit promoteur spécifique de la graine comprend ;
(a) une séquence d'acide nucléique comprenant les nucléotides 1 à 2034 comme montré dans la Figure 4 (SEQ. ID N°8) dans laquelle T peut également être U ;
(b) une séquence d'acide nucléique qui est complémentaire d'une séquence d'acide nucléique de (a) ;
(c) une séquence d'acide nucléique qui possède au moins 65% d'identité avec une séquence d'acide nucléique de (a) ou (b)
(d) une séquence d'acide nucléique qui est un analogue d'une séquence d'acide nucléique de (a),(b) ou (c); ou
(e) une séquence d'acide nucléique qui s'hybride avec une séquence d'acide nucléique de (a),(b), (c) ou (d) dans des conditions d'hybridation drastiques.

16. Graine transgénique de lin selon la revendication 10, dans laquelle l'expression du gène non natif d'intérêt résulte en une altération de la composition protéique ou d'acide gras de ladite graine.

17. Plantes transgéniques de lin capables de former des graines préparées par un procédé comprenant :
(a) la préparation d'une construction d'acide nucléique chimère comprenant dans le sens de transcription 51 à 3' comme composants liée fonctionnellement;
(1) un promoteur spécifique de graine obtenu du lin ; et
(2) une séquence d'acide nucléique d'intérêt pour laquelle ledit acide nucléique d'intérêt est non natif dudit promoteur spécifique de graine ;
(b) l'introduction de ladite construction d'acide nucléique chimère dans une cellule végétale de lin ; et
(c) la culture de ladite cellule végétale de lin dans une plante de lin mature capable de former des graines dans laquelle la séquence nucléique d'intérêt est exprimée dans la graine sous le contrôle dudit promoteur spécifique de graine, dans lesquelles spécifique à la graine signifie qu'un gène exprimé sous le contrôle du promoteur est exprimé prédominament dans des graines de plante avec moins de 5% du niveau d'expression global dans d'autres tissus de la plante et dans lesquelles ledit promoteur est un promoteur de protéine de stockage de graine, un promoteur oléosine, un promoteur de protéine de stockage 2S et un promoteur de protéine de stockage de graine du genre légumineuse.

18. Séquence d'acide nucléique isolée capable de diriger l'expression spécifique de la graine dans une plante comprenant :
(a) une séquence d'acide nucléique comprenant les nucléotides 1 à 2023 comme montré dans la Figure 1 (SEQ.ID N°1) dans laquelle T peut également être U ;
(b) une séquence d'acide nucléique qui est complémentaire d'une séquence d'acide nucléique de (a) ;
(c) une séquence d'acide nucléique qui possède au moins 65% d'identité avec une séquence d'acide nucléique de (a) ou (b) ;
(d) une séquence d'acide nucléique qui est un analogue d'une séquence d'acide nucléique de (a),(b) ou (c); ou
(e) une séquence d'acide nucléique qui s'hybride avec une séquence d'acide nucléique de (a), (b), (c) ou (d) dans des conditions d'hybridation drastiques, dans laquelle spécifique à la graine signifie qu'un gêne exprimé sous le contrôle du promoteur est exprimé de façon prédominante dans des graines de plante avec moins de 5% du niveau d'expression global dans d'autres tissus de la plante.

19. Séquence d'acide nucléique isolée capable de diriger l'expression spécifique de la graine dans une plante comprenant :
(a) une séquence d'acide nucléique comprenant les nucléotides 21 à 1852 somme montré dans la Figure 2 (SEQ.ID N°4) dans laquelle T peut également être U ;
(b) une séquence d'acide nucléique qui est complémentaire d'une séquence d'acide nucléique de (a) ;
(c) une séquence d'acide nucléique qui possède au moins 65% d'identité avec une séquence d'acide nucléique de (a) ou (b) ;
(d) une séquence d'acide nucléique qui est un analogue d'une séquence d'acide nucléique de (a),(b) ou (c); ou
(e) une séquence d'acide nucléique qui s'hybride avec une séquence d'acide nucléique de (a), (b), (c) ou (d) dans des conditions d'hybridation drastiques, dans laquelle spécifique à la graine signifie qu'un gène exprimé sous le contrôle du promoteur est exprimé de façon prédominante dans des graines de plante avec moins de 5% du niveau d'expression global dans d'autres tissus de la plante.

20. Séquence d'acide nucléique isolée capable de diriger l'expression spécifique de la graine dans une plante comprenant :
(a) une séquence d'acide nucléique comprenant les nucléotides 1 à 400 comme montré dans la Figure 3 (SEQ.ID N°6) dans laquelle T peut également être U ;
(b) une séquence d'acide nucléique qui est complémentaire d'une séquence d'acide nucléique de (a) ;
(c) une séquence d'acide nucléique qui possède au moins 65% d'identité avec une séquence d'acide nucléique de (a) ou (b) ;
(d) une séquence d'acide nucléique qui est un analogue d'une séquence d'acide nucléique de (a), (b) ou (c); ou
(e) une séquence d'acide nucléique qui s'hybride avec une séquence d'acide nucléique de (a), (b), (c) ou (d) dans des conditions d'hybridation drastiques, dans laquelle spécifique à la graine signifie qu'un gène exprimé sous le contrôle du promoteur est exprimé de façon prédominante dans des graines de plante avec moins de 5% du niveau d'expression global dans d'autres tissus de la plante.

21. Séquence d'acide nucléique isolée chimère capable de diriger l'expression spécifique de la graine dans une plante comprenant ;
(a) une séquence d'acide nucléique comprenant les nucléotides 1 à 2034 comme montré dans la Figure 4 (SEQ.ID NO8) dans laquelle T peu également être U ;
(b) une séquence d'acide nucléique qui est complémentaire à une séquence d'acide nucléique de (a) ;
(c) une séquence d'acide nucléique qui possède au moins 65% d'identité à la séquence de (a) ou (b) ;
(d) une séquence d'acide nucléique qui est un analogue d'une séquence d'acide nucléique de (a),(b) ou (c); ou
(e) une séquence d'acide nucléique qui s'hybride avec une séquence d'acide nucléique de (a), (b), (c) ou (d) dans des conditions d'hybridation drastiques, dans laquelle spécifique à la graine signifie qu'un gène exprimé sous le contrôle du promoteur est exprimé de façon prédominante dans des graines de plante avec moins de 5% du niveau d'expression global dans d'autres tissus de la plante.

22. Séquence d'acide nucléique isolée chimère comprenant :
(a) une première séquence d'acide nucléique comprenant un promoteur spécifique de la graine obtenue du lin qui comprend :
(1) une séquence d'acide nucléique comprenant les nucléotides 1 à 2023 comme montré dans la Figure 1 (SEQ.ID N°1) dans laquelle T peut également être U ;
(2) une séquence d'acide nucléique qui s'hybride avec une séquence d'acide nucléique de (a) dans des conditions d'hybridation drastiques;
(3) une séquence d'acide nucléique qui est complémentaire d'une séquence d'acide nucléique de (a) ; ou
(4) une séquence d'acide nucléique qui possède au moins 65% d'identité avec une séquence d'acide nucléique de (a) ; et
(b) une seconde séquence d'acide nucléique non native dudit promoteur spécifique de la graine, dans laquelle spécifique à la graine signifie qu'un gène exprimé sous le contrôle du promoteur est exprimé de façon prédominante dans la graine de plante avec moins de 5% du niveau d'expression global dans d'autres tissus de la plante.

23. Séquence d'acide nucléique chimère isolée comprenant:
(a) une première séquence d'acide nucléique comprenant un promoteur spécifique de la graine obtenu du lin qui comprend :
(1) une séquence d'acide nucléique comprenant les nucléotides 21 à 1852 comme montré dans la Figure 2 (SEQ.ID N°4) dans laquelle T peut également être U ;
(2) une séquence d'acide nucléique qui s'hybride avec une séquence d'acide nucléique de (a) dans des conditions d'hybridation drastiques ;
(3) une séquence d'acide nucléique qui est complémentaire d'une séquence d'acide nucléique de (a) ; ou
(4) une séquence d'acide nucléique qui possède au moins 65% d'identité avec une séquence d'acide nucléique de (a) ; et
(b) une seconde séquence d'acide nucléique non native dudit promoteur spécifique de la graine de lin, dans laquelle spécifique à la graine signifie qu'un gène exprimé sous le contrôle du promoteur est exprimé de façon prédominante dans la graine de plante avec moins de 5% du niveau d'expression global dans d'autres tissus de la plante.

24. Séquence d'acide nucléique isolée chimère comprenant;
(a) une première séquence d'acide nucléique comprenant un promoteur spécifique de la graine obtenu du lin qui comprend :
(1) une séquence d'acide nucléique comprenant les nucléotides 1 à 400 comme montré dans la Figure 3 (SEQ.ID N°6) dans laquelle T peut également être U ;
(2) une séquence d'acide nucléique qui s'hybride avec une séquence d'acide nucléique de (a) dans des conditions d'hybridation drastiques;
(3) une séquence d'acide nucléique qui est complémentaire d'une séquence d'acide nucléique de (a) ou
(4) une séquence d'acide nucléique qui possède au moins 65% d'identité avec une séquence d'acide nucléique de (a) ; et
(b) une seconde séquence d'acide nucléique non native dudit promoteur spécifique de la graine de lin, dans laquelle spécifique à la graine signifie qu'un gène exprimé sous le contrôle du promoteur est exprimé de façon prédominante dans la graine de plante avec moins de 5% du niveau d'expression global dans d'autres tissus de la plante.

25. Séquence d'acide nucléique chimère isolée comprenant:
(a) une première séquence d'acide nucléique comprenant un promoteur spécifique de la graine obtenu du lin qui comprend :
(1) une séquence d'acide nucléique comprenant les nucléotides 1 à 2034 comme montré dans la Figure 4 (SEQ.ID N°8) dans laquelle T peut également être U ;
(2) une séquence d'acide nucléique qui s'hybride avec une séquence d'acide nucléique de (a) dans des conditions d'hybridation drastiques ;
(3) une séquence d'acide nucléique qui est complémentaire d'une séquence d'acide nucléique de (a) ; ou
(4) une séquence d'acide nucléique qui possède au moins 65% d'identité avec une séquence d'acide nucléique de (a) ; et
(b) une seconde séquence d'acide nucléique non native dudit promoteur spécifique de la graine de lin, dans laquelle spécifique à la graine signifie qu'un gène exprimé sous le contrôle du promoteur est exprimé de façon prédominante dans la graine de plante avec moins de 5% du niveau d'expression global dans d'autres tissus de la plante.

26. Procédé pour l'expression d'une séquence d'acide nucléique d'intérêt dans les graines de lin comprenant
(a) l'introduction de la séquence d'acide nucléique chimère selon l'une des revendications 22 à 25 dans une cellule de plante ; et
(b) la culture de ladite cellule de plante dans une plante mature capable de former des graines, dans laquelle la seconde séquence nucléique est exprimée dans la graine sous le contrôle du promoteur spécifique de graine.

27. Procédé selon la revendication 26 dans lequel la cellule de plante est sélectionnée dans le groupe de plantes constitué par le soja (Glycine max), la graine de colza (Brassica Napus, Brassica campestris), le tournesol (Hélianthus annus), le coton (Gossypium hirsutum), le maïs (Zea mays), le tabac (Nicotiana tabacum), l'alfalfa (Medicago sativa L.), le blé (Triticum sp.), l' orge (Hordeum vulgare), l'avoine (Avena sativa L.), le sorgho (Sorghum bicolor), Arabidopsis thaliana, la pomme de terre (Solanum sp.), le lin (linum usitatissimum), le carthame (Carthamus tinctorius), le palmier à huile (Eleais guineeis), la graine d'arachide (Arachis hypogaea), la noix du Brésil (Bertholletia excelsa), la noix de coco (Cocus nucifera), le ricin (Ricinus communis), la coriandre (Coriandrum sativum), la courge (Cucurbita maxima), le jojoba (Simmondsia chinensis), et le riz (Oryza saliva).

28. Plante préparée selon le procédé de la revendication 26.

29. Cellule de plante comprenant la séquence d'acide nucléique chimère selon l'une des revendications 22 à 25.

30. Graine de plante comprenant la séquence d'acide nucléique chimère selon l'une des revendications 22 à 25.

31. Graine de plante obtenue d'une plante préparée selon le procédé de la revendication 26,

32. Vecteur d'expression recombinant comprenant une séquence d'acide nucléique selon les revendications 22 à 25.

33. Vecteur d'expression recombinant comprenant une séquence d'acide nucléique selon les revendications 22 à 25.


### SEQUENCE LISTING

<110> Chaudhary, Sarita
   van Rooijen, Gijs
   Moloney, Maurice
   Singh, Surinder
   SemBioSys Genetics Inc.
   Commonwealth Scientific and Industrial Research Organisation
<120> Flax Seed Specific Promoters
<130> 9369-147
<140>
   <141>
<150> 60/151044
   <151> 1999-08-27
<150> 60/161,722
   <151> 1999-10-27
<160> 25
<170> PatentIn Ver. 2.0
<210> 1
   <211> 4305
   <212> DNA
   <213> Linum usitatissimum
<400> 1
   ttcaaaaccc gattcccgag gcggccctat tgaagatatg ggggaagttc gacgagatcg 60
   atgtcgggtc gagtgctatg gtgatggtgc cgtttggggg gaggatgagc gagatagcca 120
   agactagcat tccgttccca cacagagttg ggaatttgta ccaaatccaa cacttgtcgt 180
   attggagcga cgatagggac gcggaaaaac acatccgttg gatcagggag ttgtacgatg 240
   atctcgagcc ttatgtgtcg aagaatccga ggtatgctta cgtgaactac agggatctcg 300
   acatcgggat gaatggagga ggtgaagggg atgagaaggg tacttatggt gaggctaagg 360
   tgtgggggga gaagtacttt ggggtcaact ttgatcggtt ggttcgggtg aagacgattg 420
   ttgatcccaa taatgtgttt cgaaacgagc agagcattcc ctcaattcca actcggttat 480
   aaggatcaat gatcaatgag aattttcctt tccaatgtga ttacaagttc tattgggtca 540
   gctttctcaa ctgctcctat tcatttagat taattcataa caactattaa tttaccagcc 600
   ttttatccgg cccgttggcc gatttatttt cttaagtttt agatgaaatg aaaccgattt 660
   agtttttatt gagatgagat taatcttaat ttgcttgaaa tttactcacg gttgatgtga 720
   tatttggaat taactaaaat gataaatatc ggataaaaat aaaaatattt aaaataaata 780
   acataaacat aagaacaata aaataaataa atttaatttt aatttatttc cttgttttct 840
   ttctgtatca tacatctctt ctcttacttc ttaaaggctt ttcaattatc acttaattaa 900
   atacaataga taaatcgtta attctataac attaacctat acacttgcac ggtgaacaat 960
   caatatgata atataataat aatataataa ttcaattatt aatctacaat tttttaatta 1020
   taaagtttat gcggtcagtt tctgcaagct ccgagctcct tgtcatcgtt agtttctgcg 1080
   gtctcaaggt ataacgactc ggagcgacga gccctttgct tccaatggac gggttgcatt 1140
   tctgccgtcg ttgagctcga ttggcgtgtc atgctggagt cagagttcct acaaaaaaac 1200
   cctaaactag agggtgatta gggtgaaatt agggtgttgg cctgggttcc attgtccaaa 1260
   gttttagtca acttaaaaac agacttaaat tttatgcttc aaaatagttt atctgttatt 1320
   atattagcgt gtaattagtc ttgacaatgg ggccggacgg gtacggattc gggaccccga 1380
   tccccgccca tagtgtaatg gctcaactgc caagtcagca ttggaccgaa attattggac 1440
   acgaagtact aatgtgaaaa actttacatt tgttattttc tactttaata ctatgctatt 1500
   ttcaaaattt gaactttaat actatgtttt tatatagttt agtatatctt aatttttatg 1560
   caaattcatc taattgtatt aaactatttt cgatccgtag ctaattattt cgaaggcaag 1620
   tcaaagtgtt attgtggact atgtgagcta atattgaacc tttatctctc ccaaccactc 1680
   aagttaattg aaccaaactc gatcggttgg gtttcgagct atttcgagcc attgttgtta 1740
   tatgcacgtg agatatcaag attgacccga acactttatt atgataatgt agaaaaagaa 1800
   aacatattct aagactacat gcatgcaaag tgcaacccct gcatggaaag ctgctcaaca 1860
   cgtggcatag actcccgcca cgtgtccatt ccacctcatc acctcacccc caccgttcac 1920
   ctcttattat atcacaacaa tcaatcaatc ctactcctcc atactcgaac aaatccgacc 1980
   aacttatacc aatattccca aacttgatta atttctcagc aatatggatc agacgcacca 2040
   gacatacgcc ggaaccacgc agaacccgag ctatggcggc gggggcacaa tgtaccagca 2100
   gcagcagccg aggtcttacc aggcggtgaa ggcggccact gcagccaccg cgggtggatc 2160
   cctcatcgtt ctgtccggtc tcatccttac ggccaccgtc atttcactca tcatagccac 2220
   ccctctcctt gtcatcttca gccctgttct tgtcccggct ctcatcaccg tcgggctctt 2280
   gatcaccggg tttcttgctt ccggtgggtt cggagtcgcc gccgtcaccg tcttgtcctg 2340
   gatctatagg tatgtataag ctttggactt tagtattgtt ataaaataca taagctgatt 2400
   tatgaacatg gatctcccaa caagagttat ttaaatgcat tctcggtctg actcgatcgg 2460
   ttgggttttg agctactcgg tcacaatggt cgggtcggct ctggatctgt tatactaata 2520
   tttggaagcc tgaagtttca ttgttctgcc ccaacttccc actacctttt gagggtgtta 2580
   agaagccata caaactaatt atgaatccct cccaacaact cagaactcga gtcagtgggt 2640
   tgtgacggtt ctctataaac atttcgaaaa tctttgttca atgaacgtag aaatgaccat 2700
   gcttgatgat tgtgggtctt ataaggtacg tgaccggcgg gcacccggcg ggaggggatt 2760
   cgctggacca ggctaggtcg aagctggccg gaaaggccag ggaggtgaag gacagggcgt 2820
   cggagttcgc acagcagcat gtcacaggtg gtcaacagac ctcttaaaga gagtcctcta 2880
   gttaaattgg tcttcgtttc tgtttcgtgg cggcttgtaa actctctttt aagtgtgctg 2940
   ttttcctttt gtctcgtgtg ttgtaagtga aagtgtaatc gaagttccaa gttggagatg 3000
   tttgtaacga tgatgttttc taataatcag agatattaaa agggttgcta atttagtatt 3060
   gcgtctgatc tcggaccaaa ctcgcaagta aaattgcaga ggatgagttg tacagaacaa 3120
   gcgtgcattg ttctggaagt tcatctcctt ggagccgacc ttgttgcttg cagtttcgcc 3180
   aagtccacta gacaatgtta cgagttaagc ctctgtcaaa cagatcgctc tagcgtccca 3240
   gaaaacacca gatttttcga aaaccatcgg ggatcaattt tcgattcaat tccgatcttg 3300
   gaagtacttg aacagaagca tgatgctaaa agataataga aaatcgaagc ctagaaaagt 3360
   tgtacagaaa gcaacaagtc aaaaatatag atcaacttca aaggttcaaa ttacatctta 3420
   cagaccccaa aaaatgacag ttaacagaag tcgactaaac agaaaccagc cagcttcacc 3480
   tggaatgaag gagctttgat caatccatcc tagcttcatt cccctttgaa attgcagaca 3540
   gagctctcat cctgctaaag ctggtggctt attcttaacc ctgcaatcaa taagcatgaa 3600
   ctaacattgg acaccttcat cggcggattg ctcgaaaatc agtgagcgag ggatttacct 3660
   gtgtgtgtag taacctctct ccttgtacat aaaatctgga aattccggca tcaactactg 3720
   ccacctttct gcttaaggtg attttatcac caaggctgag cgtgattcct tgcgtcttgc 3780
   tccgaatcct gatgtatcca ctgagctttc catctccttc cttctccagg cttatgttca 3840
   ccaatgcgtc ctcgccgaac acactcttgg cgtacaagtt cgcagccagg aatccacact 3900
   ctccatcaag tgcagacctg caaaccccaa ataagaacac aaactccaaa gtcaacgatc 3960
   aattctccgc cttttatgaa gaaaaggaaa cttctgggta cttacggtgc cgtcagacac 4020
   ttcatatttg tagacttgat gatatggtcc aggaattcct tctcgttctg aattgttgtg 4080
   ttaacagcaa cctgacagac agaaagatat cgcaaattta agatactggg atgactaggc 4140
   acagagaaat gaaatctaat tctagaagta aaaccttatt ttcccattca aattctgccc 4200
   acatagtccg gaacgcagca tccgagcaag aagcaggaga gatgtaatcc atgatatcga 4260
   tgtggatatc gttgaggacg acaactgaac gttccatcac attgg                 4305
<210> 2
   <211> 109
   <212> PRT
   <213> Linum usitatissimum
<400> 2
   Met Asp Gln Thr His Gln Thr Tyr Ala Gly Thr Thr Gln Asn Pro Ser
    1               5                  10                  15
   Tyr Gly Gly Gly Gly Thr Met Tyr Gln Gln Gln Gln Pro Arg Ser Tyr
               20                  25                  30
   Gln Ala Val Lys Ala Ala Thr Ala Ala Thr Ala Gly Gly Ser Leu Ile
           35                  40                  45
   Val Leu Ser Gly Leu Ile Leu Thr Ala Thr Val Ile Ser Leu Ile Ile
       50                  55                  60
   Ala Thr Pro Leu Leu Val Ile Phe Ser Pro Val Leu Val Pro Ala Leu
   65                  70                  75                  80
   Ile Thr Val Gly Leu Leu Ile Thr Gly Phe Leu Ala Ser Gly Gly Phe
                   85                  90                  95
   Gly Val Ala Ala Val Thr Val Leu Ser Trp Ile Tyr Arg
               100                 105
<210> 3
   <211> 46
   <212> PRT
   <213> Linum usitatissimum
<400> 3
   Tyr Val Thr Gly Gly His Pro Ala Gly Gly Asp Ser Leu Asp Gln Ala
    1               5                  10                  15
   Arg Ser Lys Leu Ala Gly Lys Ala Arg Glu Val Lys Asp Arg Ala Ser
               20                  25                  30
   Glu Phe Ala Gln Gln His Val Thr Gly Gly Gln Gln Thr Ser
           35                  40                  45
<210> 4
   <211> 3501
   <212> DNA
   <213> Linum usitatissimum
<400> 4
   tctagacatt tgacataaac cgaattcaaa gaacacaaca ttgactaaca ccaaaaagaa 60
   atagagtagt gaaatttgga agattaaaaa atagaaacaa actgattctt agaaagaaga 120
   gatgattagg tgctttcagt tcggtctgtc aggaaatcga gatgttcact tatttacatt 180
   gtcgattcat ctcccaattg tcctggttcc tttactgtcc gacgcttttt tgaatcccag 240
   ttaattccca tcaagtcttc cttcagctgc gtagcactgc tagctccaac atggagcgtg 300
   gagtctactc gttcatgggg catcgcaaag gtttgccttc atgttctgct accagccagc 360
   gcccaccgcc tcttggttgt gtggacaatt gcggtgaagc gcgcaagttg acatcccata 420
   gtctcgacac ttcaccatat ggatgtttaa aacgtatatc acgagtgcga tctacatgtc 480
   ccatcacacc acatataaag caatagtttg ggagcttttc atatttgaaa cgggcattga 540
   cgacttgccc tctcgataat ttaatctttt tttctcttca gctgattgtg tgcatccatt 600
   cgggctcaga agcacatcaa agggatctct ccatcgtagt attgggtcgt gtcgtatgat 660
   acgaagcagt cgatgaagtt tcctaatgtg cgagctacag gctccgcaaa gaacccgcga 720
   ggtagatcgt atgctagtac ccaaaaatca gtttgtcgta gcggaatcaa cactagagac 780
   tcaccctaat gcatctcatg tgtgatgaac agtttatcat ttgtgagtct aggggtcatt 840
   gtcgatgacc caatgcacat tgagcttatg atagaatttg aataggaagc gttttccacc 900
   cagatcacga atagctaccc ctttttcggg cgccaaattt ccggcatcct atcttccacc 960
   acaacttaaa gatgcgatcg gtaaggaact caccgaccac acacatcgaa taatcttcgg 1020
   tgaccggttc ctgttgatca agtccctcaa tttcctcaac ctagtcttca atcgccgcta 1080
   gcgttatccc ccgcatatgg actttcatag cgcggagcgt agccggagac gacgagcaag 1140
   aaggatgagc ggcggcagat tgcggctaaa gaaacgagct tcctgccttg ctctatggag 1200
   gcagatttct gagttgatgg tgatggattt gtgatgtgga cacttttaat ttaagttgat 1260
   tttttagcac ttcattcacg taattaaata aataatttcc agtattttat atttatttcc 1320
   ttacgttatc taattttttg aaagattaaa actttgatat aggcaagatc atgacacgtc 1380
   gaagttaagt gaatgagact cctaacaagg taataacaaa gcagttcata aaccgaatga 1440
   ccttgatctt tactaagctt gagatcattg aacatataat taaatacgtt aatgaaagat 1500
   aagaacttta atataaaaat cattcaaaac gagaaactga taacaaaaac aaagcaaacg 1560
   gccaacaaaa taatagacgg tggaaggatg atgcagagcc atccaccctt ttttcccagt 1620
   ttccttactg cttacttctc tatgcatatc acaagacgcc cttgaaactt gttagtcatg 1680
   cagagccctt actcgccagg tcaccgcacc acgtgttact ctatcacttc tcctcccttt 1740
   cctttaaaga accaccacgc cacctccctc tcacaaacac tcataaaaaa accacctctt 1800
   gcatttctcc caagttcaaa ttagttcaca gctaagcaag aactcaacaa caatggcgga 1860
   tcgtacaaca cagccacacc aagtccaggt ccacacccag caccactatc ccaccggcgg 1920
   ggctttcggc cgttatgaag gtggactcaa aggcggtcca catcaccagc aaggatcagg 1980
   cagcggccca tcagcttcca aggtgttagc agtcatgacc gcgctcccca tcggcgggac 2040
   cctccttgcc ttggccggga taaccttggc tgggacgatg atcgggctgg cgatcaccac 2100
   cccgattttt gtcatctgca gccctgttct agtcccggcc gctctgctca tcgggtttgc 2160
   cgtgagcgcg tttctggcct cggggatggc cgggctgaca gggctgacct cgctgtcgtg 2220
   gtttgcgagg tatctgcagc aggctgggca gggagttgga gtgggggtgc cggatagttt 2280
   cgagcaggcg aagaggcgca tgcaggatgc tgctgggtat atggggcaga agaccaagga 2340
   agttgggcag gagatccaga ggaagtctca ggatgtgaaa gcatcagaca aataaggtga 2400
   taataagggg ttttgggttc gtgtgtaaac tggtaaaatg gaaattctgg gttttactgt 2460
   acttttgcat gtagtggaat gaatgagttc ttgttctctt ttgtctttta atcataaagt 2520
   aagaagcagc atttcatgtt ctggttgaat attgtcaaga attcgcaaca aatttagcta 2580
   aaccagttca atcttaccgg ttagacgact tcccagtaag aaacattcca ggtccatccc 2640
   ggtataagag tctggacttc tgaaaccttt agaccttgga tttggaaaaa agatgaaacc 2700
   tttagaataa attacaacga tggcagattg tacaaaactg gagtcgagat catgtaaatt 2760
   agcccataac taagaaccgg cgatgacaac aattactagg aatatggttg ttgggctggt 2820
   cggcggctag cggtgatgat ttggaagaat cggggatcca gaatgtgaga accgaatcat 2880
   cgacgaacat tacccggcga ggagcccatt tcaagcaact ttggaactcc tatatggctg 2940
   ttccagcagg ccacctgctc aagaaagaaa gaagccatgt cagaaatcct tacgaaatct 3000
   aactggatgc tgatatgaat ccgccaggtg tgcggagttc tttacaggca ggatctataa 3060
   agaagaaaca tgttttgtat tggcattgtt gatgttccaa gcacgcagcg atctatctcc 3120
   ggatcctaac aacaaaaata cggattctgt aagaaacaag cgcagaaaac ttctgcaacg 3180
   aaaccactcg tatatttggt tctgagttgg agaaagatga ccatactact gtatttggtt 3240
   gaacttggat tggaaccgaa attttgagtt gaaaagcgag tgatcgtata taaatttcag 3300
   attcagatta ggatatccta tgagagaagg tagagttacc tgatactaca tactgcccat 3360
   caggggtaaa agttgcctcg atggttgtgt ttggagatgg ttccaggcta aatccacaac 3420
   gctgaacaaa ttaaaagatg aatggatcaa tcttcaaccc ttacttctgc atttatgagg 3480
   attggctcaa ggctctctag a                                          3501
<210> 5
   <211> 180
   <212> PRT
   <213> Linum usitatissimum
<400> 5
   Met Ala Asp Arg Thr Thr Gln Pro His Gln Val Gln Val His Thr Gln
    1               5                  10                  15
   His His Tyr Pro Thr Gly Gly Ala Phe Gly Arg Tyr Glu Gly Gly Leu
                20                  25                  30
   Lys Gly Gly Pro His His Gln Gln Gly Ser Gly Ser Gly Pro Ser Ala
            35                  40                  45
   Ser Lys Val Leu Ala Val Met Thr Ala Leu Pro Ile Gly Gly Thr Leu
       50                  55                  60
   Leu Ala Leu Ala Gly Ile Thr Leu Ala Gly Thr Met Ile Gly Leu Ala
   65                  70                  75                  80
   Ile Thr Thr Pro Ile Phe Val Ile Cys Ser Pro Val Leu Val Pro Ala
                   85                  90                  95
   Ala Leu Leu Ile Gly Phe Ala Val Ser Ala Phe Leu Ala Ser Gly Met
               100                 105                 110
   Ala Gly Leu Thr Gly Leu Thr Ser Leu Ser Trp Phe Ala Arg Tyr Leu
           115                 120                 125
   Gln Gln Ala Gly Gln Gly Val Gly Val Gly Val Pro Asp Ser Phe Glu
       130                 135                 140
   Gln Ala Lys Arg Arg Met Gln Asp Ala Ala Gly Tyr Met Gly Gln Lys
   145                 150                 155                 160
   Thr Lys Glu Val Gly Gln Glu Ile Gln Arg Lys Ser Gln Asp Val Lys
                   165                 170                 175
   Ala Ser Asp Lys
               180
<210> 6
   <211> 1676
   <212> DNA
   <213> Linum usitatissimum
<400> 6
   tccactatgt aggtcatatc catcatttta atttttgggc accattcaat tccatcttgc 60
   ctttagggat gtgaatatga acggccaagg taagagaata aaaataatcc aaattaaagc 120
   aagagaggcc aagtaagata atccaaatgt acacttgtca tcgccgaaat tagtaaaata 180
   cgcggcatat tgtattccca cacattatta aaataccgta tatgtattgg ctgcatttgc 240
   atgaataata ctacgtgtaa gcccaaaaga acccacgtgt agcccatgca aagttaacac 300
   tcacgacccc attcctcagt ctccactata taaacccacc atccccaatc ttaccaaacc 360
   caccacacga ctcacaactc gactctcaca ccttaaagaa ccaatcacca ccaaaaaatg 420
   gcaaagctga tgagcctagc agccgtagca acgcagttcc tcttcctgat cgtggtggac 480
   gcatccgtcc gaaccacagt gattatcgac gaggagacca accaaggccg cggtggaggc 540
   aaggtggcag ggacagcagc agtctgcgag cagcagatcc agcagcgaga cttcctgagg 600
   agctgccagc agttcatgtg ggagaaagtc cagaggggcg gccacagcca ctattacaac 660
   cagggccgtg gaggaggcga acagagccag tacttcgaac agctgtttgt gacgacctta 720
   agcaattgcg caccgcggtg caccatgcca ggggacttga agcgtgccat cggccaaatg 780
   aggcaggaaa tccagcagca gggacagcag cagggacagc agcaggaagt tcagaggtgg 840
   atccagcaag ctaaacaaat cgctaaggac ctccccggac agtgccgcac ccagcctagc 900
   caatgccagt tccagggcca gcagcaatct gcatggtttt gaaggggtga tcgattatga 960
   gatcgtacaa agacactgct aggtgttaag gatggataat aataataata atgagatgaa 1020
   tgtgttttaa gttagtgtaa cagctgtaat aaagagagag agagagagag agagagagag 1080
   agagagagag agagagagag agaggctgat gaaatgttat gtatgtttct tggtttttaa 1140
   aataaatgaa agcacatgct cgtgtggttc tatcgaatta ttcggcggtt cctgtgggaa 1200
   aaagtccaga agggcggccg cagctactac tacaaccaag gccgtggagg agggcaacag 1260
   agccagcact tcgatagctg ctgcgatgat cttaagcaat tgaggagcga gtgcacatgc 1320
   aggggactgg agcgtgcaat cggccagatg aggcaggaca tccagcagca gggacagcag 1380
   caggaagttg agaggtggtc ccatcaatct aaacaagtcg ctagggacct tccgggacag 1440
   tgcggcaccc agcctagccg atgccagctc caggggcagc agcagtctgc atggttttga 1500
   agtggtgatc gatgagatcg tataaagaca ctgctaggtg ttaaggatgg gataataaga 1560
   tgtgttttaa gtcattaacc gtaataaaaa gagagagagg ctgatggaat gttatgtatg 1620
   tatgtttctt ggtttttaaa attaaatgga aagcacatgc tcgtgtgggt tctatc     1676
<210> 7
   <211> 174
   <212> PRT
   <213> Linum usitatissimum
<400> 7
   Met Ala Lys Leu Met Ser Leu Ala Ala Val Ala Thr Gln Phe Leu Phe
   1               5                   10                  15
   Leu Ile Val Val Asp Ala Ser Val Arg Thr Thr Val Ile Ile Asp Glu
               20                  25                  30
   Glu Thr Asn Gln Gly Arg Gly Gly Gly Lys Val Ala Gly Thr Ala Ala
           35                  40                  45
   Val Cys Glu Gln Gln Ile Gln Gln Arg Asp Phe Leu Arg Ser Cys Gln
       50                  55                  60
   Gln Phe Met Trp Glu Lys Val Gln Arg Gly Gly His Ser His Tyr Tyr
   65                  70                  75                  80
   Asn Gln Gly Arg Gly Gly Gly Glu Gln Ser Gln Tyr Phe Glu Gln Leu
                   85                  90                  95
   Phe Val Thr Thr Leu Ser Asn Cys Ala Pro Arg Cys Thr Met Pro Gly
               100                 105                 110
   Asp Leu Lys Arg Ala Ile Gly Gln Met Arg Gln Glu Ile Gln Gln Gln
           115                 120                 125
   Gly Gln Gln Gln Gly Gln Gln Gln Glu Val Gln Arg Trp Ile Gln Gln
   130 135 140
   Ala Lys Gln Ile Ala Lys Asp Leu Pro Gly Gln Cys Arg Thr Gln Pro
   145 150 155 160
   Ser Gln Cys Gln Phe Gln Gly Gln Gln Gln Ser Ala Trp Phe
   165 170
<210> 8
   <211> 4999
   <212> DNA
   <213> Linum usitatissimum
<400> 8
   ctcaagcata cggacaaggg taaataacat agtcaccaga acataataaa caaaaagtgc 60
   agaagcaaga taaaaaaatt agctatggac attcaggttc atattggaaa catcattatc 120
   ctagtcttgt gaccatcctt cctcctgctc tagttgagag gccttgggac taacgagagg 180
   tcagttggga tagcagatcc ttatcctgga ctagcctttc tggtgtttca gagtcttcgt 240
   gccgccgtct acatctatct ccattaggtc tgaagatgac tcttcacacc aacgacgttt 300
   aaggtctcta tcctactcct agcttgcaat acctggcttg caatacctgg agcatcgtgc 360
   acgatgattg gatactgtgg aggaggagtg tttgctgatt tagagctccc ggttgggtga 420
   tttgacttcg atttcagttt aggcttgttg aaatttttca ggttccattg tgaagccttt 480
   agagcttgag cttccttcca tgttaatgcc ttgatcgaat tctcctagag aaaagggaag 540
   tcgatctctg agtattgaaa tcgaagtgca catttttttt caacgtgtcc aatcaatcca 600
   caaacaaagc agaagacagg taatctttca tacttatact gacaagtaat agtcttaccg 660
   tcatgcataa taacgtctcg ttccttcaag aggggttttc cgacatccat aacgacccga 720
   agcctcatga aagcattagg gaagaacttt tggttcttct tgtcatggcc tttataggtg 780
   tcagccgagc tcgccaattc ccgtccgact ggctccgcaa aatattcgaa cggcaagtta 840
   tggacttgca accataactc cacggtattg agcaggacct attgtgaaga ctcatctcat 900
   ggagcttcag aatgtggttg tcagcaaacc aatgaccgaa atccatcaca tgacggacgt 960
   ccagtgggtg agcgaaacga aacaggaagc gcctatcttt cagagtcgtg agctccacac 1020
   cggattccgg caactacgtg ttgggcaggc ttcgccgtat tagagatatg ttgaggcaag 1080
   acccatctgt gccactcgta caattacgag agttgttttt tttgtgattt tcctaagttt 1140
   ctcgttgatg gtgagctcat attctacatc gtatggtctc tcaacgtcgt ttcctgtcat 1200
   ctgatatccc gtcatttgca tccacgtgcg ccgcctcccg tgccaagtcc ctaggtgtca 1260
   tgcacgccaa attggtggtg gtgcgggctg ccctgtgctt cttaccgatg ggtggaggtt 1320
   gagtttgggg gtctccgcgg cgatggtagt gggttgacgg tttggtgtgg gttgacggca 1380
   ttgatcaatt tacttcttgc ttcaaattct ttggcagaaa acaattcatt agattagaac 1440
   tggaaaccag agtgatgaga cggattaagt cagattccaa cagagttaca tctcttaaga 1500
   aataatgtaa cccctttaga ctttatatat ttgcaattaa aaaaataatt taacttttag 1560
   actttatata tagttttaat aactaagttt aaccactcta ttatttatat cgaaactatt 1620
   tgtatgtctc ccctctaaat aaacttggta ttgtgtttac agaacctata atcaaataat 1680
   caatactcaa ctgaagtttg tgcagttaat tgaagggatt aacggccaaa atgcactagt 1740
   attatcaacc gaatagattc acactagatg gccatttcca tcaatatcat cgccgttctt 1800
   cttctgtcca catatcccct ctgaaacttg agagacacct gcacttcatt gtccttatta 1860
   cgtgttacaa aatgaaaccc atgcatccat gcaaactgaa gaatggcgca agaacccttc 1920
   ccctccattt cttatgtggc gaccatccat ttcaccatct cccgctataa aacaccccca 1980
   tcacttcacc tagaacatca tcactacttg cttatccatc caaaagatac ccaccatggc 2040
   tagatcatca agccctttgc ttctctcact ctgcattttc gccattctct tccactcttc 2100
   tctgggtagg cagcaattcc agcaggggaa cgagtgccag atcgacagga tcgacgcatc 2160
   cgagccggac aaaaccatcc aggcagaagc tggcaccatc gaggtatggg accagaaccg 2220
   ccagcaattc cagtgcgctg gtgttgccgt tgtaaggcgc accattgagc ccaaaggtct 2280
   tctcttgcct ttctacagca acacccctca gctcatctac atcgttcaag gtataaatta 2340
   aatcagttca tacaatgata accaccactt cgaatgtatt tatcaaatat caatgatcga 2400
   tgcacctgta tgtgttgtgt atattcaggt aggggagtta caggaatcat gttcccakga 2460
   tgtccagaga cattcgagga atcccagcag caaggacaac agggccaaca gggtagttcc 2520
   caagaccagc accagaagat ccgccgcttc cgtgaaggtg acgtcattgc cgtccctgcc 2580
   ggtgtagccc actggtccta caacgatggc aacgaaccag tcatggccat tgttgtccat 2640
   gacacttcca gccacctcaa ccaactggac aacaacccca gggtatataa gcattgccgt 2700
   agttgctaat aaattgcaca caattggaac tctattttca gtatctaata actttttcct 2760
   tttttggcag aacttctact tggcaggaaa cccgagagac gagttcgaac aatcgcagca 2820
   aggaggcagg ctgagccgtg gggagagtga aggtggacga ggacgcaggg aacctcttca 2880
   acctgcaaca acctcttctt gcggaatcga ctccaagctc atcgcggagg cgttcaatgt 2940
   cgacgagaac gtggcaagga ggctacagag cgagaacgac aacagaggcc agatcgtccg 3000
   agtcgaaggc gagctcgaca tcgtcagacc tccgaccagt atccaggagg agtcacagga 3060
   gcagggaggt cgtggtggtg gccgctacta ctccaatgga gtggaggaga ccttctgctc 3120
   catgagacta attgagaaca tcggcgatcc ttctcgggca gacattttca ctccagaagc 3180
   cggccgcgtt agatccctca acagccacaa cctccccgtc ctgcaatgga tccagcttag 3240
   cgccgagaga ggcgttctct acaatgtata gatctcactc acgcaccaac tctaaattga 3300
   atccctaatt atttaattca ccgatatctg accgaccggt ttgaattttg taggaagcga 3360
   tcaggctgcc gcactggaac atcaacgcac acagcatagt gtacgcgatc agaggacaag 3420
   ccagagtcca gatcgtgaac gaggaaggga attcggtgtt cgatggagtg ctgcaggaag 3480
   gacaggtggt gacggtgccg cagaacttcg cggtggtaaa gagatcccag agcgagaggt 3540
   ttgagtgggt ggcgttcaag accaacgaca acgcgatggt gaactcgcta gccgggagga 3600
   catcggcagt aagggcgatc cccgcggatg tactggctaa cgcctggagg gtgtcgccgg 3660
   aggaggcgag gagggtgaag ttcaacaggc aggagactca cttggctagc accaggggcc 3720
   agtccaggtc gcccgggagg ttgaatgtcg tcaaggaggt gatcaacttg cttatgtaaa 3780
   atgtgacggt gaaataataa cggtaaaata tatgtaataa taataataat aaagccacaa 3840
   agtgagaatg aggggaaggg gaaatgtgta atgagccagt agccggtggt gctaattttg 3900
   tatcgtattg tcaataaatc atgaattttg tggtttttat gtgttttttt aaatcatgaa 3960
   ttttaaattt tataaaataa tctccaatcg gaagaacaac attccatatc catggatgtt 4020
   tctttaccca aatctagttc ttgagaggat gaagcatcac cgaacagttc tgcaactatc 4080
   cctcaaaagc tttaaaatga acaacaagga acagagcaac gttccaaaga tcccaaacga 4140
   aacatattat ctatactaat actatattat taattactac tgcccggaat cacaatccct 4200
   gaatgattcc tattaactac aagccttgtt ggcggcggag aagtgatcgg cgcggcgaga 4260
   agcagcggac tcggagacga ggccttggat gagcagagtc tttacctgcc agggcgtgaa 4320
   ggggaagagc ggccttctgg agtaggagtt cagcaagcgg cggttccttg gcggagtaag 4380
   cggacgtaag ggtggntgtc gacgtcntcg tttcnggagg cgnattcatg aagggttaaa 4440
   gtcanatctg tagctctcga gtgctcaggg agccnaaaga cgttgggaaa ccgtcgncgt 4500
   ttggggcatc agtcngcggg gcacgcttcc ctcctgctgc tccanaancn angtanattt 4560
   aaaaganatg ggaaattaan taatggnaat nannaggagg attgnaacgg tcnganccgn 4620
   angaanagtt tttannggtt taaatactgg gggagtngna gccngccnct ggttccngtg 4680
   tagangaaac caagnnccgg gaggnttnca nnngnnaggg agaaaaagga nncatttnan 4740
   nangcngagg gacatgaanc ggtacngagc tgnggttcan nnancggcgn nnggnagtcc 4800
   cnngggaccn ggntggggtn anaagggaan ggaacattng gtngnangga naanaccntt 4860
   ttacnattgc ctttgcaggn nngtntnggc ncntncgggt nacatnccgc tgcatgggct 4920
   ttggggngcc nanaggnagc cncangggna nncngccncc ttgtncangn cgctnaagtt 4980
   cnattgtana tggncgttg                                              4999
<210> 9
   <211> 96
   <212> PRT
   <213> Linum usitatissimum
<400> 9
   Met Ala Arg Ser Ser Ser Pro Leu Leu Leu Ser Leu Cys Ile Phe Ala
   1               5                   10                  15
   Ile Leu Phe His Ser Ser Leu Gly Arg Gln Gln Phe Gln Gln Gly Asn
               20                  25                  30
   Glu Cys Gln Ile Asp Arg Ile Asp Ala Ser Glu Pro Asp Lys Thr Ile
           35                  40                  45
   Gln Ala Glu Ala Gly Glu Val Trp Asp Gln Asn Arg Gln Gln Phe Gln
       50                  55                  60
   Cys Ala Gly Val Ala Val Val Arg Arg Thr Ile Glu Pro Lys Gly Leu
   65                  70                  75                  80
   Leu Leu Pro Phe Tyr Ser Asn Thr Pro Gln Leu Ile Tyr Ile Val Gln
                   85                  90                  95
<210> 10
   <211> 85
   <212> PRT
   <213> Linum usitatissimum
<400> 10
   Gly Arg Gly Val Thr Gly Ile Met Phe Pro Xaa Cys Pro Glu Thr Phe
    1               5                  10                  15
   Glu Glu Ser Gln Gln Gln Gly Gln Gln Gly Gln Gln Gly Ser Ser Gln
               20                  25                  30
   Asp Gln His Gln Lys Ile Arg Arg Phe Arg Glu Gly Asp Val Ile Ala
           35                  40                  45
   Val Pro Ala Gly Val Ala His Trp Ser Tyr Asn Asp Gly Asn Glu Pro
       50                  55                  60
   Val Met Ala Ile Val Val His Asp Thr Ser Ser His Leu Asn Gln Leu
   65                  70                  75                      80
   Asp Asn Asn Pro Arg
                   85
<210> 11
   <211> 165
   <212> PRT
   <213> Linum usitatissimum
<400> 11.
   Asn Phe Tyr Leu Ala Gly Asn Pro Arg Asp Glu Phe Glu Gln Ser Gln
    1               5                  10                  15
   Gln Gly Gly Arg Leu Ser Arg Gly Glu Ser Glu Gly Gly Arg Gly Arg
               20                  25                  30
   Arg Glu Pro Leu Gln Pro Ala Thr Thr Ser Ser Cys Gly Ile Asp Ser
           35                  40                  45
   Lys Leu Ile Ala Glu Ala Phe Asn Val Asp Glu Asn Val Ala Arg Arg
       50                  55                  60
   Leu Gln Ser Glu Asn Asp Asn Arg Gly Gln Ile Val Arg Val Glu Gly
   65                  70                  75                      80
   Glu Leu Asp Ile Val Arg Pro Pro Thr Ser Ile Gln Glu Glu Ser Gln
                   85                  90                  95
   Glu Gln Gly Gly Arg Gly Gly Gly Arg Tyr Tyr Ser Asn Gly Val Glu
               100                 105                 110
   Glu Thr Phe Cys Ser Met Arg Leu Ile Glu Asn Ile Gly Asp Pro Ser
           115                 120                 125
   Arg Ala Asp Ile Phe Thr Pro Glu Ala Gly Arg Val Arg Ser Leu Asn
       130                 135                 140
   Ser His Asn Leu Pro Val Leu Gln Trp Ile Gln Leu Ser Ala Glu Arg
   145                 150                 155                     160
   Gly Val Leu Tyr Asn
                   165
<210> 12
   <211> 141
   <212> PRT
   <213> Linum usitatissimum
<400> 12
   Glu Ala Ile Arg Leu Pro His Trp Asn Ile Asn Ala His Ser Ile Val
    1               5                  10                  15
   Tyr Ala Ile Arg Gly Gln Ala Arg Val Gln Ile Val Asn Glu Glu Gly
               20                  25                  30
   Asn Ser Val Phe Asp Gly Val Leu Gln Glu Gly Gln Val Val Thr Val
           35                  40                  45
   Pro Gln Asn Phe Ala Val Val Lys Arg Ser Gln Ser Glu Arg Phe Glu
       50                  55                  60
   Trp Val Ala Phe Lys Thr Asn Asp Asn Ala Met Val Asn Ser Leu Ala
   65                  70                  75                  80
   Gly Arg Thr Ser Ala Val Arg Ala Ile Pro Ala Asp Val Leu Ala Asn
                   85                  90                  95
   Ala Trp Arg Val Ser Pro Glu Glu Ala Arg Arg Val Lys Phe Asn Arg
               100                 105                 110
   Gln Glu Thr His Leu Ala Ser Thr Arg Gly Gln Ser Arg Ser Pro Gly
           115                 120                 125
   Arg Leu Asn Val Val Lys Glu Val Ile Asn Leu Leu Met
       130                 135                 140
<210> 13
   <211> 11
   <212> PRT
   <213> Linum usitatissimum
<400> 13
   Gln Gln Gln Gly Gln Gln Gln Gly Gln Gln Gln
    1               5                  10
<210> 14
   <211> 18
   <212> DNA
   <213> Artificial Sequence
<220>
   <223> Description of Artificial Sequence: Primer
<400> 14
   tccactatgt aggtcata                                                    18
<210> 15
   <211> 18
   <212> DNA
   <213> Artificial Sequence
<220>
   <223> Description of Artificial Sequence: Primer
<400> 15 <210> 16
   <211> 15
   <212> DNA
   <213> Artificial Sequence
<220>
   <223> Description of Artificial Sequence: Primer
<400> 16
<210> 17
   <211> 18
   <212> DNA
   <213> Artificial Sequence
<220>
   <223> Description of Artificial Sequence: Primer
<400> 17
   gatagaaccc acacgagc 18
<210> 18
   <211> 29
   <212> DNA
   <213> Artificial Sequence
<220>
   <223> Description of Artificial Sequence: Primer
<400> 18
   tatctagact caagcatacg gacaagggt 29
<210> 19
   <211> 6
   <212> DNA
   <213> Artificial Sequence
<220>
   <223> Description of Artificial Sequence: XbaI site
<400> 19
   tctaga 6
<210> 20
   <211> 21
   <212> DNA
   <213> Artificial Sequence
<220>
   <223> Description of Artificial Sequence: Primer
<400> 20
   ggttatcatt gtatgaactg a 21
<210> 21
   <211> 6
   <212> DNA
   <213> Artificial Sequence
<220>
   <223> Description of Artificial Sequence: NcoI site
<400> 21
   ccatgg 6
<210> 22
   <211> 32
   <212> DNA
   <213> Artificial Sequence
<220>
   <223> Description of Artificial Sequence: Primer
<400> 22
   gcaagcttaa tgtgacggtg aaataataac gg 32
<210> 23
   <211> 6
   <212> DNA
   <213> Artificial Sequence
<220>
   <223> Description of Artificial Sequence: HindIII Site
<400> 23
   aagctt 6
<210> 24
   <211> 29
   <212> DNA
   <213> Artificial Sequence
<220>
   <223> Description of Artificial Sequence: Primer
<400> 24
   taggtacctg gcaggtaaag actctgctc 29
<210> 25
   <211> 6
   <212> DNA
   <213> Artificial Sequence
<220>
   <223> Description of Artificial Sequence: KpnI Site
<400> 25
   ggtacc 6